# EUROPEAN PATENT SPECIFICATION

(11) **EP 1 200 127 B1**
(45) Date of publication and mention of the grant of the patent: **25.06.2008**
(21) Application number: 00947581.5
(22) Date of filing: 21.07.2000
(51) Int. Cl.: A61K 45/06, A61P 19/02

(54) **SOLUTIONS AND METHODS FOR INHIBITION OF PAIN, INFLAMMATION AND CARTILAGE DEGRADATION**
SPÜLLÖSUNGEN UND VERFAHREN ZUR SCHMERZHEMMUNG, ENTZÜNDUNGSHEMMUNG UND HEMMUNG DES KNORPELABBAUS
SOLUTIONS ET PROCEDES PERMETTANT D'INHIBER LA DOULEUR, LES INFLAMMATIONS ET LA DEGRADATION DES CARTILAGES

(30) Priority: 21.07.1999 US 144904 P
(43) Date of publication of application: 02.05.2002
(60) Divisional application: 07005274.1
(73) Proprietor: Omeros Corporation, Seattle, WA 98101 (US)
(72) Inventor: DEMOPULOS, Gregory, A., Mercer Island, WA 98040 (US); PALMER, Pamela, P., San Francisco, CA 94127 (US); HERZ, Jeffrey, M., Mill Creek, WA 98012 (US)
(74) Representative: Bizley, Richard Edward
(86) International application number: PCT/US2000/019864
(87) International publication number: WO 2001/007067

(56) References cited:
- WO-A-00/23072
- WO-A-99/26657

## Description

### I. Field of the Invention

The present invention relates to the manufacture of therapeutic solutions and particularly for anti-inflammatory, anti-pain, and anti-cartilage degradation solutions.

### II. Background of the Invention

Arthroscopy is a surgical procedure in which a camera, attached to a remote light source and video monitor, is inserted into an anatomic joint (e.g., knee, shoulder, etc.) through a small portal incision in the overlying skin and joint capsule. Through similar portal incisions, surgical instruments may be placed in the joint, their use guided by arthroscopic visualization. As arthroscopists' skills have improved, an increasing number of operative procedures, once performed by "open" surgical technique, now can be accomplished arthroscopically. Such procedures include, for example, partial meniscectomies and ligament reconstructions in the knee, shoulder acromioplasties and rotator cuff debridements and elbow synovectomies. As a result of widening surgical indications and the development of small diameter arthroscopes, wrist and ankle arthroscopies also have become routine.

Throughout each arthroscopy, physiologic irrigation fluid (e.g., normal saline or lactated Ringer's) is flushed continuously through the joint, distending the joint capsule and removing operative debris, thereby providing clearer intra-articular visualization. U.S. Patent 4,504,493 to Marshall discloses an isomolar solution of glycerol in water for a non-conductive and optically clear irrigation solution for arthroscopy. Conventional physiologic irrigation fluids do not provide analgesic, anti-inflammatory and anti-cartilage degradation effects.

Alleviating pain and suffering in postoperative patients is an area of special focus in clinical medicine, especially with the growing number of outpatient operations performed each year. The most widely used systemic agents, cyclooxygenase inhibitors (e.g., ibuprofen) and opioids (e.g., morphine, fentanyl), have significant side effects including gastrointestinal irritation/bleeding and respiratory depression. The high incidence of nausea and vomiting related to opioids is especially problematic in the postoperative period. Therapeutic agents aimed at treating postoperative pain while avoiding detrimental side effects are not easily developed because the molecular targets for these agents are distributed widely throughout the body and mediate diverse physiological actions. Despite the significant clinical need to inhibit pain and inflammation, as well as cartilage degradation, methods for the delivery of inhibitors of pain, inflammation, and cartilage degradation at effective dosages while minimizing adverse systemic side effects have not been developed. As an example, conventional (i.e., intravenous, oral, subcutaneous or intramuscular) methods of administration of opiates in therapeutic doses frequently is associated with significant adverse side effects, including severe respiratory depression, changes in mood, mental clouding, profound nausea and vomiting.

Prior studies have demonstrated the ability of endogenous agents, such as serotonin (5-hydroxytryptamine, sometimes referred to herein as "5-HT"), bradykinin and histamine, to produce pain and inflammation. Sicuteri, F., et. al., Serotonin-Bradykinin Potentiation in the Pain Receptors in Man, Life Sci. 4, pp. 309-316 (1965); Rosenthal, S.R., Histamine as the Chemical Mediator for Cutaneous Pain, J. Invest. Dermat. 69, pp. 98-105 (1977); Richardson, B.P., et. al., Identification of Serotonin M-Receptor Subtypes and their Specific Blockade by a New Class of Drugs, Nature 316, pp. 126-131 (1985); Whalley, E.T., et. al., The Effect of Kinin Agonists and Antagonists, Naunyn-Schmiedeb Arch. Pharmacol. 36, pp. 652-57 (1987); Lang, E., et. al., Chemo-Sensitivity of Fine Afferents from Rat Skin In Vitro, J. Neurophysiol. 63, pp. 887-901 (1990).

For example, 5-HT applied to a human blister base (denuded skin) has been demonstrated to cause pain that can be inhibited by 5-HT₃ receptor antagonists. Richardson et al., (1985). Similarly, peripherally-applied bradykinin produces pain which can be blocked by bradykinin receptor antagonists. Sicuteri et al., 1965; Whalley et al., 1987; Dray, A., et. al., Bradykinin and Inflammatory Pain, Trends Neurosci. 16, pp. 99-104 (1993). Peripherally-applied histamine produces vasodilation, itching and pain which can be inhibited by histamine receptor antagonists. Rosenthal, 1977; Douglas, W.W., "Histamine and 5-Hydroxytryptamine (Serotonin) and their Antagonists", in Goodman, L.S., et. al., ed., The Pharmacological Basis of Therapeutics, MacMillan Publishing Company, New York, pp.605-638 (1985); Rumore, M.M., et. al., Analgesic Effects of Antihistaminics, Life Sci 36, pp. 403-416 (1985). Combinations of these three agonists (5-HT, bradykinin and histamine) applied together have been demonstrated to display a synergistic pain-causing effect, producing a long-lasting and intense pain signal. Sicuteri et al., 1965; Richardson et al., 1985; Kessler, W., et. al., "Excitation of Cutaneous Afferent Nerve Endings In Vitro by a Combination of Inflammatory Mediators and Conditioning Effect of Substance P," Exp. Brain Res. 91: 467-476 (1992).

In the body, 5-HT is located in platelets and in central neurons, histamine is found in mast cells, and bradykinin is produced from a larger precursor molecule during tissue trauma, pH changes and temperature changes. Because 5-HT can be released in large amounts from platelets at sites of tissue injury, producing plasma levels 20-fold greater than resting levels (Ashton, J.H., et. al., "Serotonin as a Mediator of Cyclic Flow Variations in Stenosed Canine Coronary Arteries," Circulation 73: 572-578 (1986)), it is possible that endogenous 5-HT plays a role in producing postoperative pain, hyperalgesia and inflammation. In fact, activation of platelets has been shown to result in excitation of peripheral nociceptors *in vitro.* Ringkamp, M., et. al., "Activated Human Platelets in Plasma Excite Nociceptors in Rat Skin, In Vitro," Neurosci. Lett. 170: 103-106 (1994). Similarly, histamine and bradykinin also are released into tissues during trauma. Kimura, E., et. al., "Changes in Bradykinin Level in Coronary Sinus Blood After the Experimental Occlusion of a Coronary Artery," Am Heart J. 85: 635-647 (1973); Douglas, 1985; Dray et. al. (1993).

In addition, prostaglandins also are known to cause pain and inflammation. Cyclooxygenase inhibitors, e.g., ibuprofen, are commonly used in non-surgical and post-operative settings to block the production of prostaglandins, thereby reducing prostaglandin-mediated pain and inflammation. Flower, R.J., et. al., Analgesic-Antipyretics and Anti-Inflammatory Agents; Drugs Employed in the Treatment of Gout, in Goodman, L.S., et. al., ed., The Pharmacological Basis of Therapeutics, MacMillan Publishing Company, New York, pp. 674-715 (1985). Cyclooxygenase inhibitors are associated with some adverse systemic side effects when applied conventionally. For example, indomethacin or ketorolac have well recognized gastrointestinal and renal adverse side effects.

As discussed, 5-HT, histamine, bradykinin and prostaglandins cause pain and inflammation. The various receptors through which these agents mediate their effects on peripheral tissues have been known and/or debated for the past two decades. Most studies have been performed in rats or other animal models. However, there are differences in pharmacology and receptor sequences between human and animal species.

Furthermore, antagonists of these mediators currently are not used for postoperative pain treatment. A class of drugs, termed 5-HT and norepinephrine uptake antagonists, which includes amitriptyline, has been used orally with moderate success for chronic pain conditions. However, the mechanisms of chronic versus acute pain states are thought to be considerably different. In fact, two studies in the acute pain setting using amitriptyline perioperatively have shown no pain-relieving effect of amitriptyline. Levine, J.D. et. al., "Desipramine Enhances Opiate Postoperative Analgesia, Pain 27: 45-49 (1986); Kerrick, J.M. et. al., "Low-Dose Amitriptyline as an Adjunct to Opioids for Postoperative Orthopedic Pain: a Placebo-Controlled Trial Period," Pain 52: 325-30 (1993). In both studies the drug was given orally. The second study noted that oral amitriptyline actually produced a lower overall sense of well being in postoperative patients, which may be due to the drug's affinity for multiple amine receptors in the brain.

Amitriptyline, in addition to blocking the uptake of 5-HT and norepinephrine, is a potent 5-HT receptor antagonist. Therefore, the lack of efficacy in reducing postoperative pain in the previously mentioned studies would appear to conflict with the proposal of a role for endogenous 5-HT in acute pain. There are a number of reasons for the lack of acute pain relief found with amitriptyline in these two studies. (1) The first study (Levine et al., 1986) used amitriptyline preoperatively for one week up until the night prior to surgery whereas the second study (Kerrick et al., 1993) only used amitriptyline postoperatively. Therefore, the level of amitriptyline that was present in the operative site tissues during the actual tissue injury phase, and the time at which 5-HT is purported to be released, is unknown. (2) Amitriptyline is known to be extensively metabolized by the liver. With oral administration, the concentration of amitriptyline in the operative site tissues may not have been sufficiently high for a long enough time period to inhibit the activity of postoperatively released 5-HT in the second study. (3) Since multiple inflammatory mediators exist, and studies have demonstrated synergism between the inflammatory mediators, blocking only one agent (5-HT) may not sufficiently inhibit the inflammatory response to tissue injury.

There have been a few studies demonstrating the ability of extremely high concentrations (1% - 3% solutions -- i.e., 10 - 30 mg per milliliter) of histamine₁ (H₁) receptor antagonists to act as local anesthetics for surgical procedures. This anesthetic effect is not believed to be mediated via H₁ receptors but, rather, due to a non-specific interaction with neuronal membrane sodium channels (similar to the action of lidocaine). Given the side effects (e.g., sedation) associated with these high "anesthetic" concentrations of histamine receptor antagonists, local administration of histamine receptor antagonists currently is not used in the perioperative setting.

### III. Summary of the Invention

There is described a solution constituting a mixture of multiple agents in low concentrations directed at inhibiting locally the mediators of pain, inflammation, and cartilage degradation in a physiologic electrolyte carrier fluid. There is also described a method for perioperative delivery of the irrigation solution containing these agents directly to a surgical site, where it works locally at the receptor and enzyme levels to preemptively limit pain, inflammation, and cartilage degradation at the site. Due to the local perioperative delivery method, a desired therapeutic effect can be achieved with lower doses of agents than are necessary when employing other methods of delivery (i.e., intravenous, intramuscular, subcutaneous and oral). The anti-pain and/or anti-inflammation agents and/or anti-cartilage degradation agents in the solution include agents selected from the following classes of receptor antagonists and agonists and enzyme activators and inhibitors, each class acting through a differing molecular mechanism of action for pain and/or inflammation inhibition and/or cartilage degradation. Representative agents for the inhibition of pain and/or inflammation include, for example: (1) serotonin receptor antagonists; (2) serotonin receptor agonists; (3) histamine receptor antagonists; (4) bradykinin receptor antagonists; (5) kallikrein inhibitors; (6) tachykinin receptor antagonists, including neurokinin, and neurokinin₂ receptor subtype antagonists; (7) calcitonin gene-related peptide (CGRP) receptor antagonists; (8) interleukin receptor antagonists; (9) inhibitors of enzymes active in the synthetic pathway for arachidonic acid metabolites, including (a) phospholipase inhibitors, including PLA₂ isoform inhibitors and PLC isoform inhibitors, (b) cyclooxygenase inhibitors, and (c) lipooxygenase inhibitors; (10) prostanoid receptor antagonists including eicosanoid EP-1 and EP-4 receptor subtype antagonists and thromboxane receptor subtype antagonists; (11) leukotriene receptor antagonists including leukotriene B₄ receptor subtype antagonists and leukotriene D₄ receptor subtype antagonists; (12) opioid receptor agonists, including µ-opioid, δ-opioid, and κ-opioid receptor subtype agonists; (13) purinoceptor antagonists including P_{2X} receptor antagonists and P_{2Y} receptor antagonists; and (14) calcium channel antagonists. Each of the above agents functions either as an anti-inflammatory agent and/or as an anti-nociceptive, i.e., anti-pain or analgesic, agent. The selection of agents from these classes of compounds is tailored for the particular application. Representative agents for the inhibition of cartilage degradation include, for example: (1) antagonists of receptors for the interleukin-1 family of proteins, including, for example, IL-1β, ILrI7 and IL-18; (2) antagonists of the tumor necrosis factor (TNF) receptor family, including, for example, TNF-R1; (3) agonists for interleukin 4, 10 and 13 receptors; (4) agonists for the TGF-β receptor superfamily, including, for example, BMP-2, BMP-4 and BMP-7; (5) inhibitors of COX-2; (6) inhibitors of the MAP kinase family, including, for example, p38 MAP kinase; (7) inhibitors of the matrix metalloproteinases (MMP) family of proteins, including, for example, MMP-3 and MMP-9; (8) inhibitors of the NF-κB family of proteins, including, for example, the p50/p65 dimer complex with IκB; (9) inhibitors of the nitric oxide synthase (NOS) family, including, for example, iNOS; (10) agonists and antagonists of integrin receptors, including, for example, agonists of ανβ₃ integrin; (11) inhibitors of the protein kinase C (PKC) family; (12) inhibitors of the protein tyrosine kinase family, including, for example, the src subfamily; (13) modulators of protein tyrosine phosphatases; and (14) inhibitors of protein src homology 2 (SH2) domains.

There is also described methods and solutions are provided for reducing or preventing destruction of articular cartilage in a joint, by administering directly to the joint of a patient a composition which includes one or more metabolically active chondroprotective agents together with one or more agents for the inhibition of pain, inflammation, or the like, as previously described, or alternatively a combination of two or more metabolically active chondroprotective agents in a pharmaceutically effective carrier for intra-articular delivery. Metabolically active agents include, but are not limited to, compounds that act directly or indirectly to modulate or alter the biological, biochemical or biophysical state of a cell, including agents that alter the electrical potential of the plasma membrane, the ligand binding or enzymatic activity of cellular receptors, intracellular or extracellularly located enzymes, protein-protein interactions, RNA-protein interactions, or DNA-protein interactions. In the present invention the use in the manufacture of a medicament in solution form for local delivery to a joint of a patient of metabolically active chondroprotective agents are provided that are based upon a combination of at least two agents as defined in claim 1, which act simultaneously on distinct molecular targets. In a representative embodiment, at least one agent is a cytokine or growth factor receptor agonist that directly provides anti-inflammatory activity and/or promotes cartilage anabolic processes and at least a second agent is a receptor antagonist or enzyme inhibitor that acts to inhibit pro-inflammatory and/or cartilage catabolic processes. Anti-inflammatory/anabolic cytokines, which act functionally to suppress the role of pro-inflammatory cytokines in the joint, promote cartilage matrix synthesis and inhibit matrix degradation. These receptor agonists include, for example, specific anti-inflammatory and anabolic cytokines, such as the interleukin (IL) agonists (e.g., IL-4, IL-10 and IL-13) and specific members of the transforming growth factor-β superfamily (e.g., TGFβ and BMP-7), insulin-like growth factors (e.g., IGF-1) and fibroblast growth factors (e.g., bFGF). At least a second agent is drawn from a class of receptor antagonists or enzyme inhibitors that acts to inhibit and reduce the activity or the expression of a pro-inflammatory molecular target (e.g., the IL-1 receptor antagonists, TNF-α receptor antagonists, cyclooxygenase-2 inhibitors, MAP kinase inhibitors, nitric oxide synthase (NOS) inhibitors, and nuclear factor kappaB (NFκB) inhibitors). The multiple agent combination of anabolic agents and inhibitors of catabolism can be delivered locally by intra-articular injection or via infusion, including administration periprocedurally (ie., pre-operatively and/or intra-operatively and/or post-operatively) during surgical arthroscopic procedures.

Articular cartilage is a specialized extracellular matrix that is produced and maintained by metabolically active articular chondrocytes. The maintenance of a normal, healthy extracellular matrix reflects a dynamic balance between the rate of biosynthesis and incorporation of matrix components, and the rate of their degradation and subsequent loss from the cartilage into the synovial fluid. While the regulatory mechanisms that underlie the matrix homeostasis are not well understood, they are clearly altered in inflammatory joint diseases and in response to joint trauma such that the rate of matrix breakdown exceeds the rate of new synthesis of matrix components. Matrix homeostasis is generally regarded to represent a dynamic balance between the effects of catabolic cytokines and anabolic cytokines (including growth factors). The optimal combination of therapeutic agents useful for cartilage protection shifts the dynamic matrix equilibrium through accelerating the synthetic rate and simultaneously inhibiting the rate of breakdown, thus maximizing anabolic processes and promoting repair.

Catabolic cytokines, such as IL-1β and TNF-α act at specific receptors on chondrocytes to induce production of MMPs that induce matrix degradation while the degradation is inhibited by anabolic cytokines such as TGF-β, BMP-2 and IGF-1. Hence, a therapeutic approach that is based only upon inhibiting catabolic processes (such as a combination of an MMP inhibitor and an IL-1 antagonist) is not optimal for cartilage repair since anabolic agents are needed to induce or accelerate biosynthesis and assembly of components for matrix production. Secondly, the multiplicity of catabolic cytokines (IL-1, TNF, IL-17, IL-18, LIF) that contribute to cartilage matrix destruction indicate it will not be practical to block all the catabolic cytokine activity. Conversely, an approach that relies only upon use of anabolic agents, such as IGF-1, BMP-2 or BMP-7, is not optimal since it does not address the counter-regulatory role of the catabolic cytokines. TGF-β, BMP-2 and IGF-1 also act at specific receptors to induce chondrocytes to produce matrix components, which is inhibited by IL,1β, TNF-α, IL-17 and LIF. Therefore, the optimal therapeutic combination for chondroprotection is composed of at least one anabolic agent and one inhibitor of cartilage catabolism.

There is described a method for manufacturing a medicament compounded as a dilute irrigation solution for use in continuously irrigating an operative site, typically at the site of a joint of a patient, during an arthroscopic operative procedure. The method entails dissolving in a physiologic electrolyte carrier fluid at least one anti-cartilage degradation agent and preferably one or more anti-pain/anti-inflammatory agents, and for some applications anti-cartilage degradation agents, each agent included at a concentration of preferably no more than about 100,000 nanomolar, more preferably no more than about 25,000 nanomolar, and most preferably no more than about 10,000 nanomolar.

There is described the delivery of a dilute combination of multiple receptor antagonists and agonists and enzyme inhibitors and activators directly to a wound or operative site, during therapeutic or diagnostic procedures for the inhibition of pain, inflammation and cartilage degradation. Since the active ingredients in the solution are being locally applied directly to the operative tissues in a continuous fashion, the drugs may be used efficaciously at extremely low doses relative to those doses required for therapeutic effect when the same drugs are delivered orally, intramuscularly, subcutaneously or intravenously. As used herein, the term "local" encompasses application of a drug in and around a wound or other operative site, and excludes oral, subcutaneous, intravenous and intramuscular administration. The term "continuous" as used herein encompasses uninterrupted application, repeated application at frequent intervals, and applications which are uninterrupted except for brief cessations such as to permit the introduction of other drugs or agents or procedural equipment, such that a substantially constant predetermined concentration is maintained locally at the wound or operative site.

The advantages of low dose applications of agents are three-fold. The most important is the absence of systemic side effects which often limit the usefulness of these agents. Additionally, the agents selected for particular applications in the solutions herein are highly specific with regard to the mediators and mediation targets on which they work. This specificity is maintained by the low dosages utilized. Finally, the cost of these active agents per operative procedure is low.

The advantages of local administration of the agents via irrigation or other fluid application are the following: (1) local administration guarantees a known concentration at the target site, regardless of interpatient variability in metabolism, blood flow, etc.; (2) because of the direct mode of delivery, a therapeutic concentration is obtained instantaneously and, thus, improved dosage control is provided; and (3) local administration of the active agents directly to a wound or operative site also substantially reduces degradation of the agents through systemic processes, e.g., first- and second-pass metabolism, that would otherwise occur if the agents were given orally, intravenously, subcutaneously or intramuscularly. This is particularly true for those active agents that are peptides, which are metabolized rapidly. Thus, local administration permits the use of compounds or agents which otherwise could not be employed therapeutically. For example, some agents in the following classes are peptidic: bradykinin receptor antagonists; tachykinin receptor antagonists; opioid receptor agonists; CGRP receptor antagonists; and interleukin receptor antagonists, TNF-receptor antagonists; TGF-β receptor agonists; BMP-2 and BMP-7 receptor agonists; IL4, IL10 and IL-13 receptor agonists; and integrin receptor agonists and antagonists. Local, continuous delivery to the wound or operative site minimizes drug degradation or metabolism while also providing for the continuous replacement of that portion of the agent that may be degraded, to ensure that a local therapeutic concentration, sufficient to maintain receptor occupancy or enzymatic saturation, is maintained throughout the duration of the operative procedure.

Local administration of the solution perioperatively throughout a surgical procedure as described herein produces a preemptive analgesic, anti-inflammatory and cartilage protective effect. As used herein, the term "perioperative" encompasses application intraprocedurally, pre- and intraprocedurally, intra- and postprocedurally, and pre-, intra- and postprocedurally. To maximize the preemptive anti-inflammatory, analgesic (for certain applications) and cartilage protective (for certain applications) effects, the solutions are most preferably applied pre-, intra- and postoperatively. By occupying the target receptors or inactivating or activating targeted enzymes prior to the initiation of significant operative trauma locally, the agents of the present solution modulate specific pathways to preemptively inhibit the targeted pathologic process. If inflammatory mediators and processes are preemptively inhibited before they can exert tissue damage, the benefit is more substantial than if given after the damage has been initiated.

Inhibiting more than one pain, inflammatory or cartilage degradation mediator by application of the multiple agent solution has been shown to dramatically reduce the degree of inflammation and pain, and theoretically should provide a cartilage protective effect. The irrigation solutions described herein include combinations of drugs, each solution acting on multiple receptors or enzymes. The drug agents are thus simultaneously effective against a combination of pathologic processes, including pain and inflammation, and loss of cartilage homeostasis. The action of these agents is considered to be synergistic, in that the multiple receptor antagonists and inhibitory agonists provide a disproportionately increased efficacy in combination relative to the efficacy of the individual agents. The synergistic action of several of the agents are discussed, by way of example, below in the detailed descriptions of those agents.

Used perioperatively, the solution should result in a clinically significant decrease in operative site pain and inflammation, and of cartilage degradation, relative to currently-used irrigation fluids, thereby decreasing the patient's postoperative analgesic (i.e., opiate) requirement and, where appropriate, allowing earlier patient mobilization of the operative site. No extra effort on the part of the surgeon and operating room personnel is required to use the present solution relative to conventional irrigation fluids. For optimum chondroprotection, the solutions are administered directly to a joint prior to, during and/or after a surgical procedure.

### IV. Brief Description of the Drawings

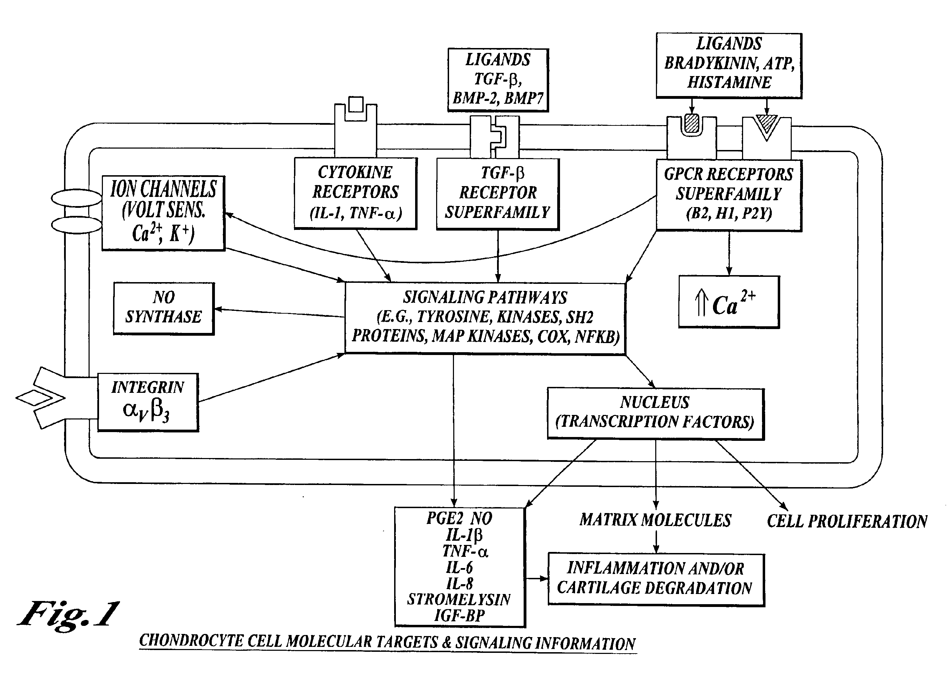

FIGURE 1 is a schematic overview of a chondrocyte cell showing molecular targets and flow of signaling information leading to the production of mediators of inflammation and shifts in cartilage metabolism. The integration of extrinsic signals through several families of cell surface receptors, including cytokine receptor such as the interleukin-1 (IL-1) receptor family and the tumor necrosis factor (TIVF) receptor family, the TGF-β receptor superfamily and integrins is shown to converge on common intracellular signaling pathways that include major groups of protein molecules that are therapeutic targets of drugs included in the solutions of the present invention (MAP kinases, PKC, tyrosine kinases, SH2 proteins, COX, PLA2 and NF-6B. Activation of these signaling pathways controls chondrocyte expression of a number of inducible gene products, including IL-1, TNF- α, IL-6, IL-8 and Stromelysin (MMP-3), and other mediators (nitric oxide (NO) and PGE2) which may lead to inflammation and/or cartilage degradation, or synthesis of matrix molecules and chondrocyte proliferation;

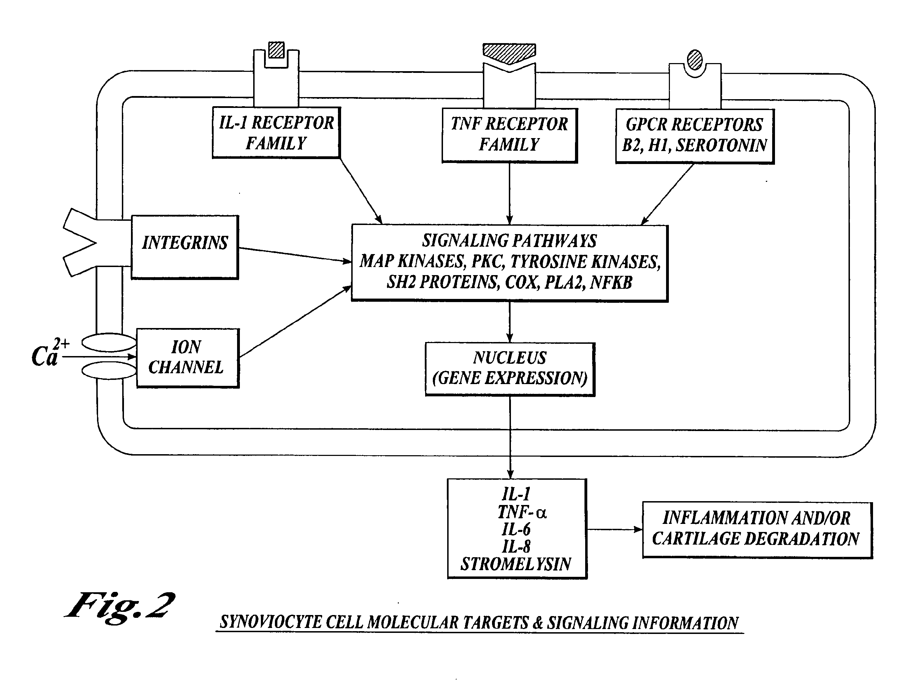

FIGURE 2 provides a a schematic overview of a synoviocyte cell showing molecular targets and flow of signaling information leading to the production of mediators of inflammation and shifts in cartilage metabolism. The integration of extrinsic signals through several families of cell surface receptors, including cytokine receptors which include the interleukin-1 (IL-1) receptor family and the tumor necrosis factor (TNF) receptor family, the G-protein coupled receptors which include bradykinin, histamine and serotonin subtypes, and integrins is shown to converge on common intracellular signaling pathways that include major groups of protein molecules that are therapeutic targets of drugs included in the solutions (MAP kinases, PKC, tyrosine kinases, SH2 proteins, COX. PLA2 and NF-6B). Activation of these signaling pathways controls synoviocyte expression of a number of inducible gene products, including IL-1, TNF-α, IL-6. IL-8 and Stromelysin (MMP-3), which may lead to inflammation and/or cartilage degradation;

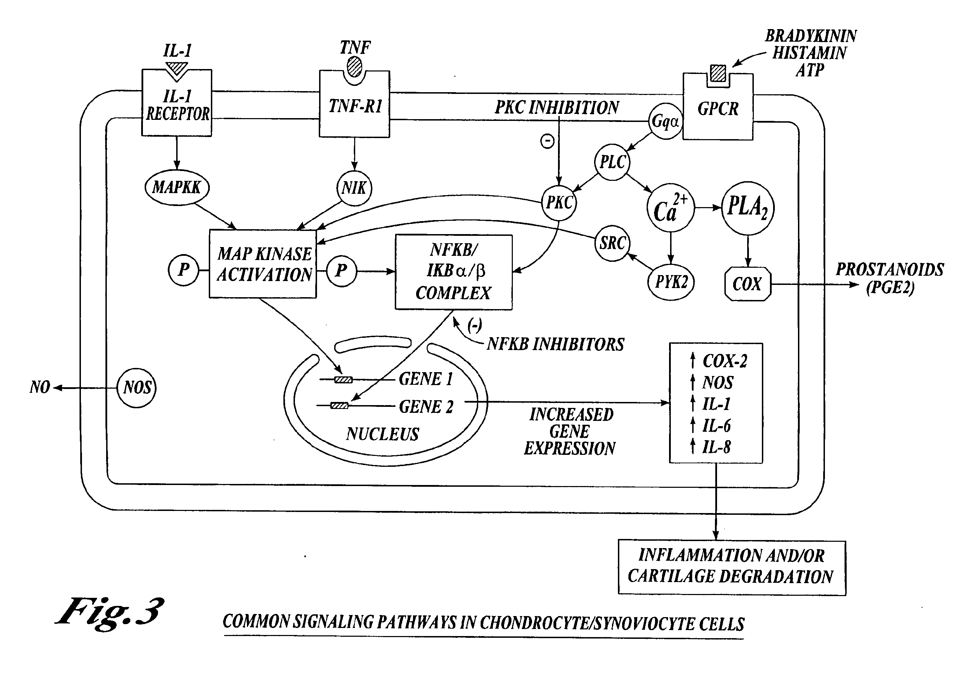

FIGURE 3 is a a diagram of common signaling pathways in both chondrocyte and synoviocyte cells, including key signaling proteins responsible for "crosstalk" between GPCR activated receptor pathways and pro-inflammatory cytokine pathways that lead to inflammation and or cartilage degradation;

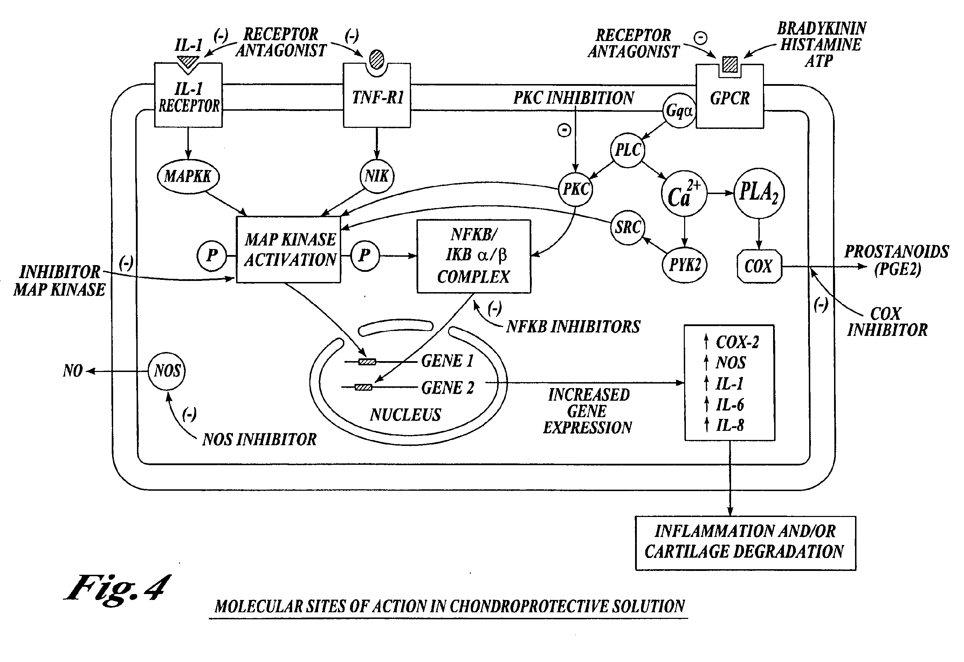

FIGURE 4 is a a diagram of of common signaling pathways in both chondrocyte and synoviocyte cells , including key signaling proteins responsible for "crosstalk" between GPCR activated receptor pathways and pro-inflammatory cytokine pathways. Specific moelcular sites of action for some drugs in a preferred chondroprotective solution are identified;

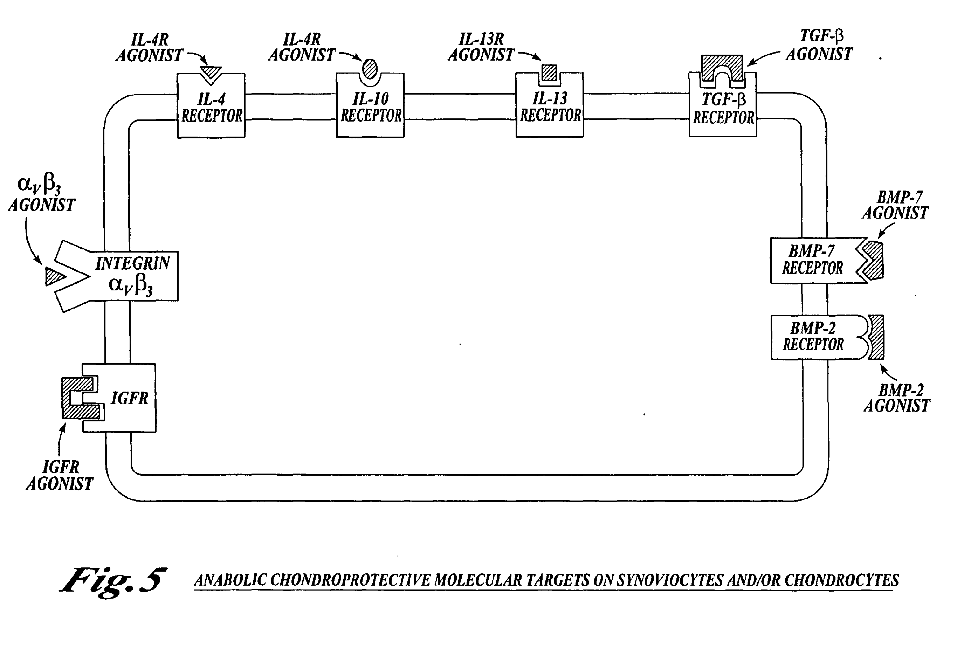

FIGURE 5 is a diagram of molecular targets present on either chondrocytes or synoviocytes that promote an anabolic response of cartilage. Specific sites of action of some drugs in the preferred chondroprotective solution are identified;

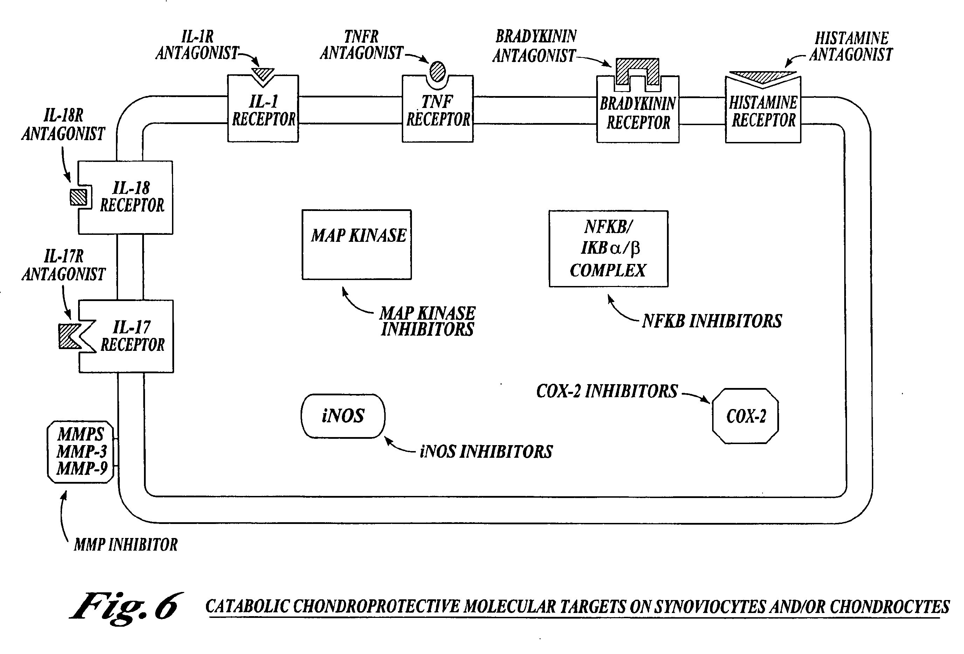

FIGURE 6 is a diagram of molecular targets present on either chondrocytes or synoviocytes that promote a catabolic response in cartilage. Specific sites of action of some drugs in the preferred chondroprotective solution are identified;

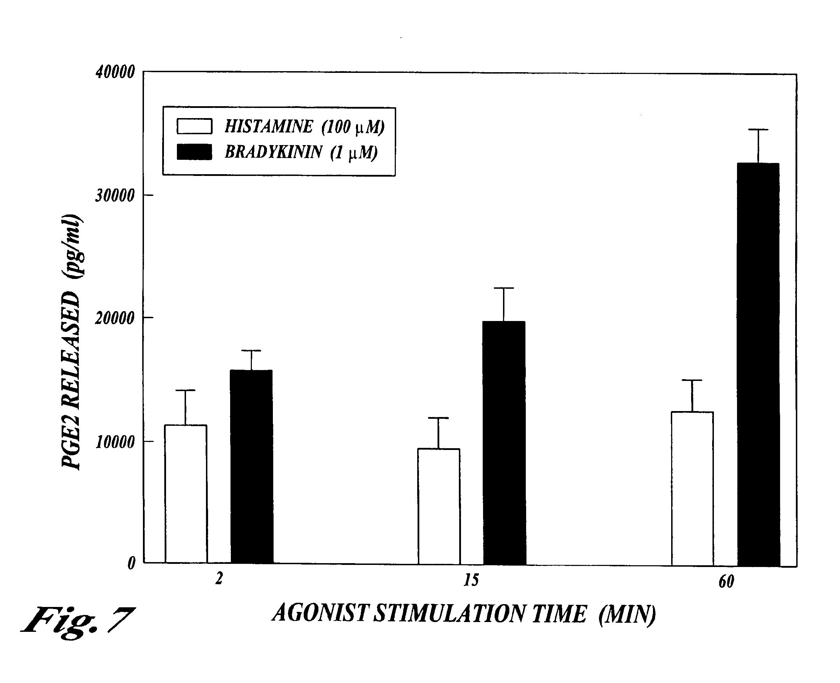

FIGURE 7 is a graphical representation of the production of prostaglandin E2 in synovial cultures by G-protein regulatory agonists following overnight priming with interleukin-1 (IL.-1, 10U/ml). The cultures were stimulated for the indicated times with histamine (100 µM, open bars), or bradykinin (1 µM, closed bars), and the prostaglandin E2 released to the culture supernatant was determined as described in Example 6. The values shown are the mean ± the standard deviation from a representative experiment, and are corrected for basal prostaglandin E2 production by unstimulated cultures;

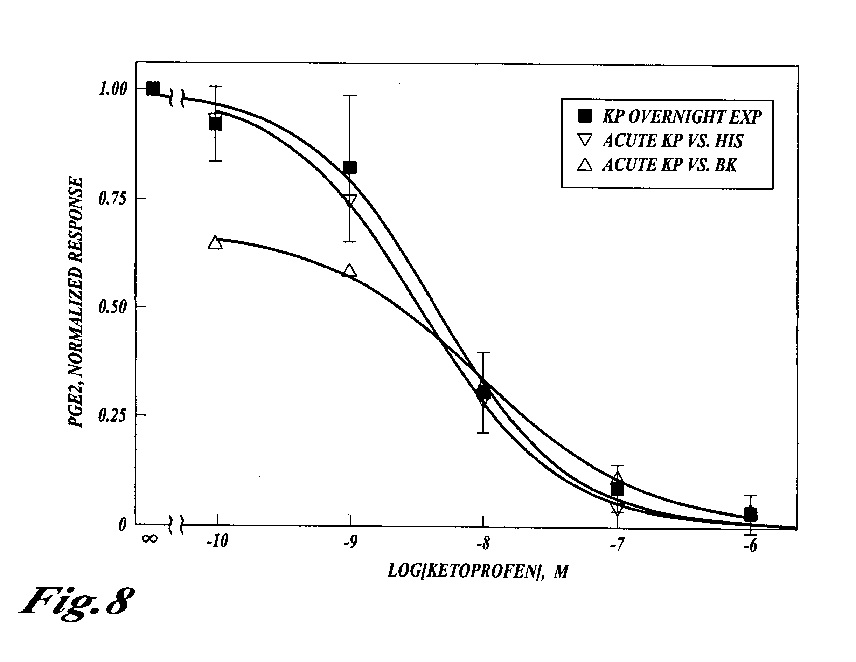

FIGURE 8 is a graphical representation of the inhibition of prostaglandin E2 production in synovial cultures by ketoprofen. The cultures were primed overnight with IL-1 (10U/ml) in the presence (shown as **"■"**) or absence (shown as "Δ" or "∇") of the indicated concentrations of ketoprofen. After one day, prostaglandin E2 was measured in the supernatants of cultures treated overnight with ketoprofen, and the remaining cultures were washed, incubated for 10 minutes with the indicated concentrations of ketoprofen, and then prostaglandin E2 production was measured in response to a subsequent 3 minute challenge with histamine (100 µM, ∇) or bradykinin (1 µM, Δ) in the continuing presence of the indicated amounts of ketoprofen. The data shown are normalized to the maximum response obtained for each agonist, respectively, and represent the mean ± the standard deviation from three experiments performed on different cell lines; and

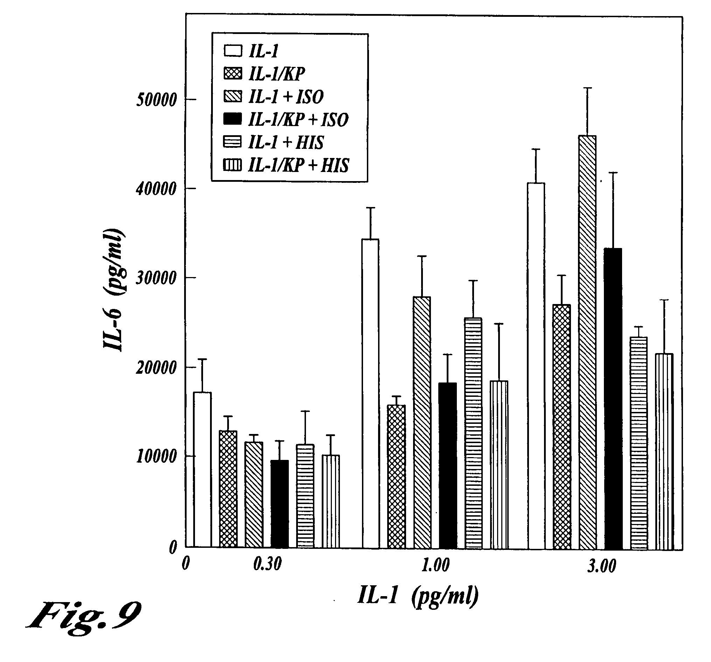

FIGURE 9 is a graphical representation of the effect of ketoprofen on IL-6 production by synovial cultures at 16 hours in the presence of the indicated concentrations of IL-1 plus the added G-protein coupled receptor ligands. Cultures were incubated for 16 hours with IL-1 at the indicated concentration (0.3, 1.0 and 3.0 pg/ml) in the absence and presence of 0.75 µM ketoprofen in experimental growth medium with one of the following additional receptor ligands: 1) isoproterenol (ISO) at 1.0 µM to activate the camp pathway, or 2) histamine (HIS) at 100 µM to activate the IP3/calcium pathway. Culture supernatants were collected and replaced with fresh media aliquots containing the same agonist additions at 8 hour intervals. Following treatment, the supernatant medium corresponding to the treatment interval from 8 to 16 hours was collected and analyzed for IL-6.

### V. Detailed Description of the Preferred Embodiment

The irrigation and injectable solutions described herein are dilute solutions of one or more chondroprotective agents and, optionally, one or more pain and/or inflammation inhibitory agents in a physiologic carrier. The carrier is a liquid solution, which as used herein is intended to encompass biocompatible solvents, suspensions, polymerizable and non-polymerizable gels, pastes and salves, as well as components of sustained release delivery systems, such as microparticles, microspheres or nanoparticles composed of proteins, liposomes, carbohydrates, synthetic organic compounds, or inorganic compounds. Preferably the carrier is an aqueous solution that may include physiologic electrolytes, such as normal saline or lactated Ringer's solution.

The anti-inflammation and/or anti-pain agents are selected from the group consisting of: (1) serotonin receptor antagonists; (2) serotonin receptor agonists; (3) histamine receptor antagonists; (4) bradykinin receptor antagonists; (5) kallikrein inhibitors; (6) tachykinin receptor antagonists, including neurokinin₁ and neurokinin₂ receptor subtype antagonists; (7) calcitonin gene-related peptide (CGRP) receptor antagonists; (8) interleukin receptor antagonists; (9) inhibitors of enzymes active in the synthetic pathway for arachidonic acid metabolites, including (a) phospholipase inhibitors, including PLA₂ isoform inhibitors and PLC isoform inhibitors (b) cyclooxygenase inhibitors, and (c) lipooxygenase inhibitors; (10) prostanoid receptor antagonists including eicosanoid EP-1 and EP-4 receptor subtype antagonists and thromboxane receptor subtype antagonists; (11) leukotriene receptor antagonists including leukotriene B₄ receptor subtype antagonists and leukotriene D₄ receptor subtype antagonists; (12) opioid receptor agonists, including µ-opioid, δ-opioid, and κ-opioid receptor subtype agonists; (13) purinoceptor agonists and antagonists including P_{2X} receptor antagonists and P_{2Y} receptor antagonists; and (14) calcium channel antagonists.

Suitable chondroprotective agents include, for example, (1) antagonists of receptors for the interleukin family of proteins, including, for example, IL-1β, IL-17 and IL-I8; (2) antagonists of the tumor necrosis factor (TNF) receptor family, including, for example, TNF-R1; (3) agonists for interleukin 4,10 and 13 receptors; (4) agonists for the TGF-β receptor superfamily, including, for example, BMP-2, BMP-4 and BMP-7; (5) inhibitors of COX-2; (6) inhibitors of the MAP kinase family, including, for example, p38 MAP kinase; (7) inhibitors of the matrix metalloproteinases (MMP) family of proteins, including, for example, MMP-3 and MMP-9; (8) inhibitors of the NF-κB family of proteins, including, for example, the p50/p65 dimer complex with IxB; (9) inhibitors of the nitric oxide synthase (NOS) family, including, for example, iNOS; (10) agonists and antagonists of integrin receptors, including, for example, agonists of (αᵥβ₃ integrin; (11) inhibitors of the protein kinase C (PKC) family, (12) inhibitors of the protein tyrosine kinase family, including, for example, the src subfamily; (13) modulators of protein tyrosine phosphatases; and (14) inhibitors of protein src homology 2 (SH2) domains.

Specific preferred embodiments of the solution described herein for use in chondroprotection and arthroscopic procedures preferably include a combination of agents that act simultaneously on distinct molecular targets to promote cartilage anabolism and inhibit unregulated or excess cartilage catabolic processes to achieve maximum inhibition of inflammatory processes and maintain cartilage homeostasis, thereby achieving a chrondroprotective effect within the joint.

In each of the surgical solutions described herein, the agents are included in low concentrations in a liquid or fluid solution and are delivered locally in low doses relative to concentrations and doses required with conventional methods of drug administration to achieve the desired therapeutic effect. As used herein, "liquid" or "fluid" is intended to encompass pharmaceutically acceptable, biocompatible solvents, suspensions, polymerizable and non-polymerizable gels, pastes and salves. Preferably the carrier is an aqueous solution that may include physiologic electrolytes, such as normal saline or lactated Ringer's solution. It is impossible or not practical to obtain an equivalent therapeutic effect by delivering similarly dosed agents via other (i.e., intravenous, subcutaneous, intramuscular or oral) routes of drug administration since drugs given systemically are subject to first-and second-pass metabolism. The concentration of each agent is determined in part based on its receptor dissociation constant, K_{d} or enzyme inhibition constant, Kᵢ. As used herein, the term dissociation constant is intended to encompass both the equilibrium dissociation constant for its respective agonist-receptor or antagonist-receptor interaction and the equilibrium inhibitory constant for its respective activator-enzyme or inhibitor-enzyme interaction. Each agent is preferably included at a low concentration of 0.1 to 10,000 times K_{d} or Kᵢ except for cyclooxygenase inhibitors, which may be required at larger concentrations depending on the particular inhibitor selected. Preferably, each agent is included at a concentration of 1.0 to 1.000 times K_{d} or Kᵢ and most preferably at approximately 100 times K_{d} or Kᵢ. These concentrations are adjusted as needed to account for dilution in the absence of metabolic transformation at the local delivery site. The exact agents selected for use in the solution, and the concentration of the agents, varies in accordance with the particular application, as described below.

A solution as described herein can include just a single or multiple pain and/or inflammation inhibitory agent(s), multiple chondroprotective agent(s) at least one of which is an anabolic chondroprotective agent and at least one of which is an inhibitor of cartilage catabolism, or a combination of both chondroprotective agent(s) and pain and/or inflammation inhibitory agents, at low concentration. However, due to the aforementioned synergistic effect of multiple agents, and the desire to broadly block pain and inflammation, and cartilage destruction, it is preferred that multiple agents be utilized.

The surgical solutions constitute a novel therapeutic approach by combining multiple pharmacologic agents acting at distinct receptor and/or enzyme molecular targets. To date, pharmacologic strategies have focused on the development of highly specific drugs that are selective for individual receptor subtypes and enzyme isoforms that mediate responses to individual signaling neurotransmitters and hormones. Furthermore, despite inactivation of a single receptor subtype or enzyme, activation of other receptor subtypes or enzymes and the resultant signal transduction often can trigger a cascade effect. This explains the significant difficulty in employing a single receptor-specific drug to block a pathophysiologic process in which multiple signaling mediators (e.g., cytokines, growth factors or eicosonoids) play a role. Therefore, targeting only a specific individual receptor subtype or isotype is likely to be ineffective.

In contrast to the standard approach to pharmacologic therapy, the therapeutic approach of the present surgical solutions is based on the rationale that a combination of drugs acting simultaneously on distinct molecular targets is highly effective in the inhibition of the full spectrum of events that underlie the development of a pathophysiologic state. Furthermore, instead of targeting a specific receptor subtype alone, the surgical solutions are composed of drugs that target common molecular mechanisms operating in different cellular physiologic processes involved in the development of pain, inflammation, and cartilage degradation (see FIGURE 1). In this way, the cascading of additional receptors and enzymes in the nociceptive, inflammatory, and cartilage degradation pathways is minimized by the surgical solutions. In these pathophysiologic pathways, the surgical solutions inhibit the cascade effect both "upstream" and "downstream".

An example of "upstream" inhibition is the cyclooxygenase antagonists in the setting of pain and inflammation. The cyclooxygenase enzymes (COX₁ and COX₂) catalyze the conversion of arachidonic acid to prostaglandin H which is an intermediate in the biosynthesis of inflammatory and nociceptive mediators including prostaglandins, leukotrienes, and thromboxanes. The cyclooxygenase inhibitors block "upstream" the formation of these inflammatory and nociceptive mediators. This strategy precludes the need to block the interactions of the seven described subtypes of prostanoid receptors with prostanoid products of the COX biochemical pathway. A similar "upstream" inhibitor included in the surgical solutions is aprotinin, a kallikrein inhibitor. The enzyme kallikrein, a serine protease, cleaves the high molecular weight kininogens in plasma to produce bradykinins, important mediators of pain and inflammation. By inhibition of kallikrein, aprotinin effectively inhibits the synthesis of bradykinins, thereby providing an effective "upstream" inhibition of these inflammatory mediators.

The surgical solutions also make use of "downstream" inhibitors to control the pathophysiologic pathways. In synoviocyte and chondrocyte preparations that have been treated with a variety of inflammatory cytokines (e.g., IL-1β and TNFα) implicated in progressive articular cartilage degeneration, MAP kinase inhibitors produce a cartilage protective effect. The p38 MAP kinase is a point of conveyance in signaling pathways for multiple catabolic cytokines, and its inhibition prevents upregulation of multiple cellular products mediating cartilage degradation. The MAP kinase inhibitors, therefore, provide a significant advantage to the surgical solutions in the settings of joint inflammation by providing "downstream" cartilage protective effects that are independent of the physiologic combination of cytokine receptor agonists initiating the shift cartilage homeostasis.

The following is a description of suitable drugs falling in the aforementioned classes of anti-inflammation/anti-pain agents and chondroprotective agents, as well as suitable concentrations for use in solutions described herein. While not wishing to be limited by theory, the justification behind the selection of the various classes of agents which is believed to render the agents operative is also set forth.

### 1. Inhibitors of Pain and/or Inflammation

### 1. Serotonin Receptor Antagonists

Serotonin (5-HT) is thought to produce pain by stimulating serotonin₂ (5-HT₂) and/or serotonin₃ (5-HT₃) receptors on nociceptive neurons in the periphery. Most researchers agree that 5-HT₃ receptors on peripheral nociceptors mediate the immediate pain sensation produced by 5-HT (Richardson et al., 1985). In addition to inhibiting 5-HT-induced pain, 5-HT₃ receptor antagonists, by inhibiting nociceptor activation, also may inhibit neurogenic inflammation. Barnes PJ., et. al., Modulation of Neurogenic Inflammation: Novel Approaches to Inflammatory Disease, Trends in Pharmacological Sciences 11, pp. 185-189 (1990). A study in rat ankle joints, however, claims the 5-HT₂ receptor is responsible for nociceptor activation by 5-HT. Grubb, B.D., et al., A Study of 5-HT-Receptors Associated with Afferent Nerves Located in Normal and Inflamed Rat Ankle Joints, Agents Actions 25, pp. 216-18 (1988). Therefore, activation of 5-HT₂ receptors also may play a role in peripheral pain and neurogenic inflammation.

One goal of the solutions described herein is to block pain and a multitude of inflammatory processes. Thus, 5-HT₂ and 5-HT₃ receptor antagonists are both suitably used, either individually or together, in the solution, as shall be described subsequently. Amitriptyline (Elavil™) is a suitable 5-HT₂ receptor antagonist. Amitriptyline has been used clinically for numerous years as an anti-depressant, and is found to have beneficial effects in certain chronic pain patients. Metoclopramide (Reglan™) is used clinically as an anti-emetic drug, but displays moderate affinity for the 5-HT₃ receptor and can inhibit the actions of 5-HT at this receptor, possibly inhibiting the pain due to 5-HT release from platelets. Thus, it also is suitable for use herein.

Other suitable 5-HT₂ receptor antagonists include imipramine, trazodone, desipramine and ketanserin. Ketanserin has been used clinically for its antihypertensive effects. Hedner, T., et. al., Effects of a New Serotonin Antagonist, Ketanserin, in Experimental and Clinical Hypertension, Am J of Hypertension, pp. 317s-23s (Jul. 1988). Other suitable 5-HT₃ receptor antagonists include cisapride and ondansetron. Suitable serotonin 1B receptor antagonists include yohimbine, N-[-methoxy-3-(4-methyl-l-piperanzinyl)phenyll-2'-methyl-4'-(5-methyl-1, 2, 4-oxadiazol-3-yl)[1, 1-biphenyl]-4-carboxamide ("GR127935") and methiothepin. Therapeutic and preferred concentrations for use of these drugs in the solution are set forth in Table 1

**Table 1**

| Therapeutic and Preferred Concentrations of Pain and/or inflammation Inhibitory Agents | | |
|---|---|---|
| | Therapeutic Concentrations | Preferred Concentrations |
| Class of Agent | (Nanomolar) | (Nanomolar) |
| Serotonin₂ Receptor Antagonists: | | |
| amitriptyline | 0.1-1,000 | 50 - 500 |
| MDL-11,939 | 0.1- 1,000 | 50 - 500 |
| AMI-193 | 0.1- 2,000 | 50 - 500 |
| desipramine | 0.1- -1,000 | 50 - 500 |
| ketanserin | 0.1- 1,000 | 50-500 |

| Serotonin₃ Receptor Antagonists: | | |
|---|---|---|
| tropisetron | 0.01 - 100 | 0.05 - 50 |
| metoclopramide | 10-10,000 | 200 - 2,000 |
| cisapride | 0.1- 1,000 | 20 - 200 |
| ondansetron | 0.1-1,000 | 20 - 200 |

| Serotonin_{1B} (Human 1D_{β}) Antagonists: | | |
|---|---|---|
| Isamoltare | 0.1-1,000 | 50 - 500 |
| GR127935 | 0.1-1,000 | 10 - 500 |
| methiothepin | 0.1-500 | 1 - 100 |
| SB216641 | 0.2 - 2,000 | 2 - 200 |

### 2. Serotonin Receptor Agonists

5-HT_{1A}, 5-HT_{1B} and 5-HT_{1D} receptors are known to inhibit adenylate cyclase activity. Thus including a low dose of these serotonin_{1A}, serotonin_{1B} and serotonin 1_{D} receptor agonists in the solution should inhibit neurons mediating pain and inflammation. The same action is expected from serotonin_{1E} and serotonin _{1F} receptor agonists because these receptors also inhibit adenylate cyclase.

Buspirone is a suitable 1A receptor agonist for use herein. Sumatriptan is a suitable 1A, 1B, 1D and 1F receptor agonist. A suitable 1B and 1D receptor agonist is dihydroergotamine. A suitable 1E receptor agonist is ergonovine. Therapeutic and preferred concentrations for these receptor agonists are provided in Table 2.

**Table 2**

| Therapeutic and Preferred Concentrations of Pain and/or inflammation Inhibitory Agents | | |
|---|---|---|
| Class of Agent | Therapeutic Concentrations (Nanomolar) | Preferred Concentrations (Nanomolar) |
| Serotonin_{1A} Agonists: | | |
| buspirone | 1-1,000 | 10 - 200 |
| sumatriptan | 1 -1,000 | 10 - 200 |

| Serotonin_{1B} Agonists: | | |
|---|---|---|
| dihydroergotamine | 0.1 - 1,000 | 10 -100 |
| sumatriptan | 1 -1,000 | 10 - 200 |
| naratriptan | 1 -1,000 | 10 - 200 |
| rizatriptan | 1 -1,000 | 10 - 200 |
| zolmitriptan | 1 - 1,000 | 10 - 200 |
| L-694.247 | 1 - 1,000 | 10 - 200 |

| Serotonin_{1D} Agonists: | | |
|---|---|---|
| dihydroergotamine | 0.1- 1,000 | 10 -100 |
| sumatriptan | 1 - 1,000 | 10 - 200 |
| naratriptan | 1 - 1,000 | 10 - 200 |
| rizatriptan | 1-1.000 | 10-200 |
| zolmitriptan | 1-1,000 | 10-200 |
| L-694.247 | 1-1,000 | 10 - 200 |

| Serotonin_{1E} Agonists: | | |
|---|---|---|
| ergonovine | 10 - 2,000 | 100-1,000 |

| Serotonin_{1F} Agonists: | | |
|---|---|---|
| sumatriptan | 1 - 1,000 | 10 - 200 |

### 3. Histamine Receptor Antagonists

Histamine receptors generally are divided into histamine₁ (H₁) and histamine₂ (H₂) subtypes. The classic inflammatory response to the peripheral administration of histamine is mediated via the H₁ receptor. Douglas, 1985. Therefore, the solution preferably includes a histamine H₁ receptor antagonist. Promethazine (Phenergan™) is a commonly used anti-emetic drug which potently blocks H₁ receptors, and is suitable for use herein. Interestingly, this drug also has been shown to possess local anesthetic effects but the concentrations necessary for this effect are several orders higher than that necessary to block H₁ receptors, thus, the effects are believed to occur by different mechanisms. The histamine receptor antagonist concentration in the solution is sufficient to inhibit H₁ receptors involved in nociceptor activation, but not to achieve a "local anesthetic" effect, thereby eliminating the concern regarding systemic side effects.

Other suitable H₁ receptor antagonists include terfenadine, diphenhydramine, amitriptyline, mepyramine and tripolidine. Because amitriptyline is also effective as a serotonin₂ receptor antagonist, it has a dual function as used herein. Suitable therapeutic and preferred concentrations for each of these H₁ receptor antagonists are set forth in Table 3.

**Table 3**

| Therapeutic and Preferred Concentrations of Pain and/or inflammation Inhibitory Agents | | |
|---|---|---|
| Class of Agent | Therapeutic Concentrations (Nanomolar) | Preferred Concentrations (Nanomolar) |
| Histamine₁ Receptor Antagonists: | | |
| promethazine | 0.1 - 1,000 | 50 - 200 |
| diphenhydramine | 0.1 -1,000 | 50 - 200 |
| amitriptyline | 0.1 -1,000 | 50 - 500 |
| terfenadine | 0.1 - 1,000 | 50 - 500 |
| mepyramine (pyrilamine) | 0.1 -1,000 | 5 - 200 |
| tripolidine | 0.01 - 100 | 5 - 20 |

### 4. Bradykinin Receptor Antagonists

Bradykinin receptors generally are divided into bradykinin₁ (B₁) and bradykinin₂ (B₂) subtypes. Studies have shown that acute peripheral pain and inflammation produced by bradykinin are mediated by the B₂ subtype whereas bradykinin-induced pain in the setting of chronic inflammation is mediated via the B₁ subtype. Perkins, M.N., et. al., Antinociceptive Activity of the Bradykinin B1 and B2 Receptor Antagonists, des Arg9, [Leu8]-BK and HOE 140, in Two Models of Persistent Hyperalgesia in the Rat, Pain 53, pp. 191-97 (1993); Dray, A., et. al., Bradykinin and Inflammatory Pain, Trends Neurosci 16, pp. 99-104 (1993), each of which references is hereby expressly incorporated by reference.

At present, bradykinin receptor antagonists are not used clinically. Some of these drugs are peptides, and thus they cannot be taken orally, because they would be digested Antagonists to B₂ receptors block bradykinin-induced acute pain and inflammation. Dray et. al., 1993. B₁ receptor antagonists inhibit pain in chronic inflammatory conditions. Perkins et al., 1993; Dray et. al., 1993. Therefore, depending on the application, the solution preferably includes either or both bradykinin B₁ and B₂ receptor antagonists. For example, arthroscopy is performed for both acute and chronic conditions, and thus an irrigation solution for arthroscopy could include both B₁ and B₂ receptor antagonists.

Suitable bradykinin receptor antagonists for use herein include the following bradykinin₁ receptor antagonists: the [des-Arg]¹⁰ derivative of D-Arg-(Hyp³-Thi⁵-D-Tic⁷-Oic⁸)-BK ("the [des-Arg¹⁰] derivative of HOE 140", available from Hoechst Pharmaceuticals); and [Leu⁸] des-Arg⁹-BK. Suitable bradykinin₂ receptor antagonists include: [D-Phe⁷]-BK; D-Arg-(Hyp³-Thi^{5,8}-D-Phe⁷)-BK ("NPC 349"); D-Arg-(Hyp³--D-Phe⁷)-BK ("NPC 567"); and D-Arg-(Hyp³-Thi⁵-D-Tic⁷-Oic⁸)-BK ("HOE 140"). These compounds are more fully described in the previously incorporated Perkins et. al. 1993 and Dray et al. 1993 references. Suitable therapeutic and preferred concentrations are provided in Table 4.

**Table 4**

| Therapeutic and Preferred Concentrations of Pain and/or inflammation Inhibitory Agents | | |
|---|---|---|
| Class of Agent | Therapeutic Concentrations (Nanomolar) | Preferred Concentrations (Nanomolar) |
| Bradykinin, Receptor Antagonists: | | |
| [Leu⁸] des-Arg⁹-BK | 1-1,000 | 50-500 |
| [des-Arg 10] derivative of HOE 140 | 1-1,000 | 50-500 |
| [leu⁹] [des-Arg¹⁰] kalliden | 0.1-500 | 10-200 |

| Bradykinin₂ Receptor Antagonists: | | |
|---|---|---|
| [D-Phe⁷]-BK | 100 - 10,000 | 200 - 5,000 |
| NPC 349 | 1-1,000 | 50 - 500 |
| NPC 567 | 1-1,000 | 50-500 |
| HOE 140 | 1- 1,000 | 50-500 |

### 5. Kallikrein Inhibitors

The peptide bradykinin is an important mediator of pain and inflammation, as noted previously. Bradykinin is produced as a cleavage product by the action of kallikrein on high molecular weight kininogens in plasma. Therefore kallikrein inhibitors are believed to be therapeutic in inhibiting bradykinin production and resultant pain and inflammation. A suitable kallikrein inhibitor for use herein is aprotinin. Suitable concentrations for use in the solutions described herein are set forth below in Table 5.

**Table 5**

| Therapeutic and Preferred Concentrations of Pain and/or inflammation Inhibitory Agents | | |
|---|---|---|
| Class of Agent | Therapeutic Concentrations (Nanomolar) | Preferred Concentrations (Nanomolar) |
| Kallikrein Inhibitor: | | |
| Aprotinin | 0.1 - 1,000 | 50 - 500 |

### 6. Tachykinin Receptor Antagonists

Tachykinins (TKs) are a family of structurally related peptides that include substance P, neurokinin A (NKA) and neurokinin B (NKB). Neurons are the major source of TKs in the periphery. An important general effect of TKs is neuronal stimulation, but other effects include endothelium-dependent vasodilation, plasma protein extravasation, mast cell recruitment and degranulation and stimulation of inflammatory cells. Maggi, C.A., Gen. Pharmacol., Vol. 22, pp. 1-24 (1991). Due to the above combination of physiological actions mediated by activation of TK receptors, targeting of TK receptors is a reasonable approach for the promotion of analgesia and the treatment of neurogenic inflammation.

### 6a. Neurokinin₁ Receptor Subtype Antagonists

Substance P activates the neurokinin receptor subtype referred to as NK₁. Substance P is an undecapeptide that is present in sensory nerve terminals. Substance P is known to have multiple actions which produce inflammation and pain in the periphery after C-fiber activation, including vasodilation, plasma extravasation and degranulation of mast cells. Levine, J.D., et. al., Peptides and the Primary Afferent Nociceptor, J. Neurosci. 13, p. 2273 (1993). A suitable Substance P antagonist is ([D-Pro⁹[spiro-gamma-lactam]Leu¹⁰,Trp¹¹]physalaemin-(1-11)) ("GR 82334"). Other suitable antagonists which act on the NK₁ receptor are: 1-imino-2-(2-methoxy-phenyl)-ethyl)-7,7-diphenyl-4-perhydroisoindolone(3aR,7aR) ("RP 67580"); and 2S,3S-cis-3-(2-methoxybenzylamino)-2-benzhydrylquinuclidine ("CP 96,345"). Suitable concentrations for these agents are set forth in Table 6.

**Table 6**

| Therapeutic and Preferred Concentrations of Pain and/or inflammation Inhibitory Agents | | |
|---|---|---|
| Class of Agent | Therapeutic Concentrations (Nanomolar) | Preferred Concentrations (Nanomolar) |
| Neurokinin₁ Receptor Subtype Antagonists | | |
| GR 82334 | 1-1,000 | 10 - 500 |
| CP 96,345 | 1-10,000 | 100-1,000 |
| RP 67580 | 0.1-1,000 | 100-1,000 |

### 6b. Neurokinin₂ Receptor Subtype Antagonists

Neurokinin A is a peptide which is colocalized in sensory neurons with substance P and which also promotes inflammation and pain. Neurokinin A activates the specific neurokinin receptor referred to as NK₂. Edmonds-Alt, S., et. al., A *Potent and Selective Non-Peptide Antagonist of the Neurokinin A (NK₂) Receptor,* Life Sci. SO:PL101 (1992). Examples of suitable NK₂ antagonists include: ((S)-N-methyl-N-[4-(4-acetylanfino-4-phenylpiperidino)-2-(3,4-dichlorophenyl)butyl]-benzamide ("(±)-SR 48968"); Met-Asp-Trp-Phe-Dap-Leu ("MEN 10,627"); and cyc(Gln-Trp-Phe-Gly-Leu-Met) ("L 659,877"). Suitable concentrations of these agents are provided in Table 7.

**Table 7**

| Therapeutic and Preferred Concentrations of Pain and/or inflammation Inhibitory Agents | | |
|---|---|---|
| Class of Agent | Therapeutic Concentrations (Nanomolar) | Preferred Concentrations (Nanomolar) |
| Neurokinin₂ Receptor Subtype Antagonists: | | |
| MEN 10,627 | 1-1,000 | 10-1,000 |
| L 659,877 | 10-10,000 | 100-10,000 |
| (±)-SR 48968 | 10-10,000 | 100-10,000 |

### 7. CGRP Receptor Antagonists

Calcitonin gene-related peptide (CGRP) is a peptide which is also colocalized in sensory neurons with substance P, and which acts as a vasodilator and potentiates the actions of substance P. Brain, S.D., et. al., Inflammatory Oedema Induced by Synergism Between Calcitonin Gene-Related Peptide (CGRP) and Mediators of Increased Vascular Permeability, Br. J. Pharmacol. 99, p. 202 (1985). An example of a suitable CGRP receptor antagonist is I-CGRP-(8-37), a truncated version of CGRP. This polypeptide inhibits the activation of CGRP receptors. Suitable concentrations for this agent are provided in Table 8.

**Table 8**

| Therapeutic and Preferred Concentrations of Pain and/or inflammation Inhibitory Agents | | |
|---|---|---|
| Class of Agent | Therapeutic Concentrations (Nanomolar) | Preferred Concentrations (Nanomolar) |
| CGRP Receptor Antagonist: | | |
| I-CGRP-(8-37) | 1-1,000 | 10-500 |

### 8. Interleukin Receptor Antagonist

Interleukins are a family of peptides, classified as cytokines, produced by leukocytes and other cells in response to inflammatory mediators. Interleukins (IL) may be potent hyperalgesic agents peripherally. Ferriera, S.H., et. al., Interleukin-1β as a Potent Hyperalgesic Agent Antagonized by a Tripeptide Analogue, Nature 334, p. 698 (1988). An example of a suitable IL-1β receptor antagonist is Lys-D-Pro-Thr, which is a truncated version of IL-1β. This tripeptide inhibits the activation of IL-1β receptors. Suitable concentrations for this agent are provided in Table 9.

**Table 9**

| Therapeutic and Preferred Concentrations of Pain and/or inflammation Inhibitory Agents | | |
|---|---|---|
| Class of Agent | Therapeutic Concentrations (Nanomolar) | Preferred Concentrations (Nanomolar) |
| Interleukin Receptor Antagonist: Lys-D-Pro-Thr | 1-1,000 | 10-500 |

### 9. Inhibitors of Enzymes Active in the Synthetic Pathway for Arachidonic Acid Metabolites

### 9a. Phospholipase Inhibitors

The production of arachidonic acid by phospholipase A₂ (PLA₂) enzymes (cPLA₂, iPLA₂, sPLA₂) and phospholipase C (PLC) results in a cascade of reactions that produces numerous mediators of inflammation, know as eicosanoids. There are a number of stages throughout this pathway that can be inhibited, thereby decreasing the production of these inflammatory mediators. Examples of inhibition at these various stages are given below.

Inhibition of the enzyme PLA₂ isoform inhibits the release of arachidonic acid from cell membranes, and therefore inhibits the production of prostaglandins and leukotrienes resulting in decreased inflammation and pain. Glaser, K.B., Regulation of Phospholipase A2 Enzymes: Selective Inhibitors and Their Pharmacological Potential, Adv. Pharmacol. 32, p. 31 (1995). An example of a suitable PLA₂ isoform inhibitor is manoalide. Suitable concentrations for this agent are included in Table 10. Inhibition of the phospholipase C_{γ} (PLC_{γ}) isoform also will result in decreased production of prostanoids and leukotrienes, and, therefore, will result in decreased pain and inflammation. An example of a PLCγ isoform inhibitor is 1-[6-((17β-3-methoxyestra-1,3,5(10)-trien-17-yl)amino)hexyl]-1H-pyrrole-2,5-dione.

**Table 10**

| Therapeutic and Preferred Concentrations of Pain and/or inflammation Inhibitory Agents | | |
|---|---|---|
| Class of Agent | Therapeutic Concentrations (Nanomolar) | Preferred Concentrations (Nanomolar) |
| Phospholipase Inhibitor: | | |
| manoalide | 100-100,000 | 500-10,000 |
| aristolochic acid | 40-400,000 | 400-40,000 |
| ACA | 10-100,000 | 100-10,000 |
| HELSS | 6-6,000 | 60-6,000 |

### 9b. Cyclooxygenase Inhibitors

Nonsteroidal anti-inflammatory drugs (NSAIDs) are widely used as anti-inflammatory, anti-pyretic, anti-thrombotic and analgesic agents. Lewis, R.A., Prostaglandins and Leukotrienes, In: Textbook of Rheumatology, 3d ed. (Kelley W.N., et. al., eds.), p. 258 (1989). The molecular targets for these drugs are type I and type II cyclooxygenases (COX-1 and COX-2). These enzymes are also known as Prostaglandin H Synthase (PGHS)-1 (constitutive) and -2 (inducible), and catalyze the conversion of arachidonic acid to Prostaglandin H which is an intermediate in the biosynthesis of prostaglandins and thromboxanes. The COX-2 enzyme has been identified in endothelial cells, macrophages, and fibroblasts. This enzyme is induced by IL-1 and TNF-α, and its expression is upregulated at sites of inflammation. Constitutive activity of COX-1 and induced activity of COX-2 both lead to synthesis of prostaglandins which contribute to pain and inflammation.

Many NSAIDs currently on the market (diclofenac, naproxen, indomethacin, ibuprofen, etc.) are generally nonselective inhibitors of both isoforms of COX, but may show greater selectively for COX-1 over COX-2, although this ratio varies for the different compounds. Use of COX-1 and 2 inhibitors to block formation of prostaglandins represents a better therapeutic strategy than attempting to block interactions of the natural ligands with the seven described subtypes of prostanoid receptors. Reported antagonists of the eicosanoid receptors (EP-1, EP-2, EP-3) are quite rare and only specific, high affinity antagonists of the thromboxane A2 receptor have been reported. Wallace, J. and Cirino, G. Trends in Pharm. Sci., Vol. 15 pp. 405-406 (1994).

Representative therapeutic and preferred concentrations of of cyclooxygenase inhibitors for use in the solution are provided in Table 11.

**Table 11**

| Therapeutic and Preferred Concentrations of Pain and/or inflammation Inhibitory Agents | | |
|---|---|---|
| Class of Agent | Therapeutic Concentrations (Nanomolar) | Preferred Concentrations (Nanomolar) |
| Cyclooxygenase Inhibitors: | | |
| ketorolac | 100 - 10,000 | 500 - 5,000 |
| indomethacin | 1,000 - 500,000 | 10,000 - 200,000 |

### 9c. Lipooxygenase Inhibitors

Inhibition of the enzyme lipooxygenase inhibits the production of leukotrienes, such as leukotriene B₄, which is known to be an important mediator of inflammation and pain. Lewis, R.A., Prostaglandins and Leukotrienes, In: Textbook of Rheumatology, 3d ed. (Kelley W.N., et. al., eds.), p. 258 (1989). An example of a 5-lipooxygenase antagonist is 2,3,5-trimethyl-6-(12-hydroxy-5,10-dodecadiynyl)-1,4-benzoquinone ("AA 861"), suitable concentrations for which are listed in Table 12.

**Table 12**

| Therapeutic and Preferred Concentrations of Pain and/or inflammation Inhibitory Agents | | |
|---|---|---|
| Class of Agent | Therapeutic Concentrations (Nanomolar) | Preferred Concentrations (Nanomolar) |
| Lipooxygenase Inhibitor: AA 861 | 100-10,000 | 500-5,000 |
| Caffeic acid | 500-50,000 | 2,000-20,000 |

### 10. Prostanoid Receptor Antagonists

Specific prostanoids produced as metabolites of arachidonic acid mediate their inflammatory effects through activation of prostanoid receptors. Examples of classes of specific prostanoid antagonists are the eicosanoid EP-1 and EP-4 receptor subtype antagonists and the thromboxane receptor subtype antagonists. A suitable prostaglandin E₂ receptor antagonist is 8-chlorodibenz[b,f][1 ,4]oxazepine-10(11H)-carboxylic acid, 2-acetylhydrazide ("SC 19220"). A suitable thromboxane receptor subtype antagonist is [15-[1α, 2β(5Z), 3β, 4a]-7-[3-[2-(phenylamino)-carbonyl] hydrazino] methyl]-7-oxobicyclo-[2,2,1]-hept-2-yl]-5-heptanoic acid ("SQ 29548"). Suitable concentrations for these agents are set forth in Table 13.

**Table 13**

| Therapeutic and Preferred Concentrations of Pain and/or inflammation Inhibitory Agents | | |
|---|---|---|
| Class of Agent | Therapeutic Concentrations (Nanomolar) | Preferred Concentrations (Nanomolar) |
| Eicosanoid EP-1 Antagonist: | | |
| SC 19220 | 100-10,000 | 500-5,000 |

### 11. Leukotriene Receptor Antagonists

The leukotrienes (LTB₄, LTC₄, and LTD₄) are products of the 5-lipooxygenase pathway of arachidonic acid metabolism that are generated enzymatically and have important biological properties. Leukotrienes are implicated in a number of pathological conditions including inflammation. Specific antagonists are currently being sought by many pharmaceutical companies for potential therapeutic intervention in these pathologies. Halushka, P.V., et al., Annu. Rev. Pharmacol. Toxicol. 29: 213-239 (1989); Ford-Hutchinson, A. Crit. Rev. Immunol. 10: 1-12 (1990). The LTB₄ receptor is found in certain immune cells including eosinophils and neutrophils. LTB₄ binding to these receptors results in chemotaxis and lysosomal enzyme release thereby contributing to the process of inflammation. The signal transduction process associated with activation of the LTB₄ receptor involves G-protein-mediated stimulation of phosphotidylinositol (PI) metabolism and elevation of intracellular calcium (see FIGURE 2).

An example of a suitable leukotriene B₄ receptor antagonist is SC (+)-(S)-7-(3-(2-(cyclopropylmethyl)-3-methoxy-4-[(methylamino)-carbonyl]phenoxy(propoxy)-3,4-dihydro-8-propyl-2H-1-benzopyran-2-propanoic acid ("SC 53228"). Concentrations for this agent are provided in Table 14. Other suitable leukotriene B₄ receptor antagonists include [3-[-2(7-chloro-2-quinolinyl)ethenyl]phenyl] [[3-(dimethylamino-3-oxopropyl)thio] methyl]thiopropanoic acid ("MK 0571") and the drugs LY 66,071 and ICI 20,3219. MK 0571 also acts as a LTD₄ receptor subtype antagonist.

**Table 14**

| Therapeutic and Preferred Concentrations of Pain and/or inflammation Inhibitory Agents | | |
|---|---|---|
| Class of Agent | Therapeutic Concentrations (Nanomolar) | Preferred Concentrations (Nanomolar) |
| Leukotriene B₄ Antagonist: | | |
| SC 53228 | 100-10,000 | 500-5,000 |

### 12. Opioid Receptor Agonists

Activation of opioid receptors results in anti-nociceptive effects and, therefore, agonists to these receptors are desirable. Opioid receptors include the µ-, δ- and κ-opioid receptor subtypes. The µ-receptors are located on sensory neuron terminals in the periphery and activation of these receptors inhibits sensory neuron activity. Basbaum, A.I., et. al., Opiate analgesia: How Central is a Peripheral Target?, N. Engl. J. Med., 325:1168 (1991). δ- and κ-receptors are located on sympathetic efferent terminals and inhibit the release of prostaglandins, thereby inhibiting pain and inflammation. Taiwo, Y.O., et. al., Kappa- and Delta-Opioids Block Sympathetically Dependent Hyperalgesia, J. Neurosci., Vol. 11, page 928 (1991). The opioid receptor subtypes are members of the G-protein coupled receptor superfamily. Therefore, all opioid receptor agonists interact and initiate signaling through their cognate G-protein coupled receptor. Examples of suitable µ-opioid receptor agonists are fentanyl and Try-D-Ala-Gly-[N-MePhe]-NH(CH₂)-OH ("DAMGO"). An example of a suitable δ-opiold receptor agonist is [D-Pen²,D-Pen⁵]enkephalin ("DPDPE"). An example of a suitable κ-opioid receptor agonist is (trans)-3,4-dichloro-N-methyl-N-[2-(1-pyrrolidnyl)cyclohexyl]-benzene acetamide ("U50,488"). Suitable concentrations for each of these agents are set forth in Table 15.

**Table 15**

| Therapeutic and Preferred Concentrations of Pain and/or inflammation Inhibitory Agents | | |
|---|---|---|
| Class of Agent | Therapeutic Concentrations (Nanomolar) | Preferred Concentrations (Nanomolar) |
| µ-Opioid Agonist: | | |
| DAMGO | 0.1-100 | 0.5-20 |
| sufentanyl | 0.01-50 | 1-20 |
| fentanyl | 0.1-500 | 10-200 |
| PL 017 | 0.05-50 | 0.25-10 |

| δ-Opioid Agonist: | | |
|---|---|---|
| DPDPE | 0.1-500 | 1.0-100 |

| κ-Opioid Agonist: | | |
|---|---|---|
| U50,488 | 0.1-500 | 1.0-100 |

### 13. Purinoceptor Antagonists

Extracellular ATP acts as a signaling molecule through interactions with P₂ purinoceptors. One major class of purinoceptors are the P₂ₓ purinoceptors which are ligand-gated ion channels possessing intrinsic ion channels permeable to Na⁺, K⁺, and Ca²⁺. P₂ₓ receptors described in sensory neurons are important for primary afferent neurotransmission and nociception. ATP is known to depolarize sensory neurons and plays a role in nociceptor activation since ATP released from damaged cells stimulates P_{2X} receptors leading to depolarization of nociceptive nerve-fiber terminals. The P2X₃ receptor has a highly restricted distribution (Chen, C.C., et. al., Nature, Vol. 377, pp. 428-431 (1995)) since it is selectively expressed in sensory C-fiber nerves that run into the spinal cord and many of these C-fibers are known to carry the receptors for painful stimuli. Thus, the highly restricted localization of expression for the P2X₃ receptor subunits make these subtypes excellent targets for analgesic action (see FIGURES 3 and 7).

Calcium-mobilizing purine receptors, which belong to the G-protein receptor superfamily, have been described on the surface of mammalian articular chondrocytes. ATP was found to stimulate a dose-dependent, transient rise in the concentration of calcium ions in differentiated, primary chondrocytes. Heterologous desensitization experiments demonstrated that chondrocytes showed no subsequent response to UTP after initial stimulation with ATP. These results are consistent with the presence of a P2Y receptors of the cell surface of chondrocytes. Purine-induced calcium mobilization in passaged chondrocytes showed the same pharmacological profile with respect to agonist sensitivity. ATP and UTP did not alter cartilage matrix synthesis as measured by rate of incorporation of [35S]sulfate into glycosaminoglycan by cartilage explants or primary chondrocytes. Matrix degradation, measured by release of glycosaminoglycan from cartilage explants, was also unaltered by either agonist. The presence of a functional P2Y purine receptor on the surface of primary articular chondrocytes enable concentrations of extracellular purines, such as ATP, to activate chondrocyte metabolism.

Other studies have defined the expression of both P1 and P2 purine receptor genes by human articular chondrocytes and profiled ligand-mediated prostaglandin E2 release. The P2Y2 receptor agonists ATP and UTP stimulated a small release of PGE2 that was synergistically enhanced after pretreatment with human IL-1α. PGE2 release in response to coaddition of ATP and UTP after IL-1 pretreatment was mimicked by phorbol myristate acetate. The function of the P2Y2 receptor is to increase IL-1-mediated PGE2 release, thereby promoting pain and inflammation within the joint. Thus, the use of P2Y antagonists should prevent activation of inflammatory mediator production by both synoviocytes and chondrocytes.

Suitable antagonists of P_{2X}/ATP purinoceptors include, by way of example, suramin and pyridoxylphosphate-6-azophenyl-2,4-disulfonic acid ("PPADS"). Suitable concentrations for these agents are provided in Table 16.

**Table 16**

| Therapeutic and Preferred Concentrations of Pain and/or inflammation Inhibitory Agents | | |
|---|---|---|
| Class of Agent | Therapeutic Concentrations (Nanomolar) | Preferred Concentrations (Nanomolar) |
| P2X and/or P2Y Antagonists: | | |
| suramin | 100-100,000 | 10,000-100,000 |
| PPADS | 100-100,000 | 10,000-100,000 |

### 14. Ca²⁺ Channel Antagonists

Calcium channel antagonists are a distinct group of drugs that interfere with the transmembrane flux of calcium ions required for activation of cellular responses mediating neuroinflammation. Calcium entry into synoviocytes and chondrocytes is a key event mediating activation of responses in these cells. Furthermore, the role of bradykinin, histamine, serotonin (SHT₂) and neurokinin receptors (NK₁ and NK₂) in mediating the neuroinflammation signal transduction pathway includes increases in intracellular calcium, thus leading to activation of calcium channels on the plasma membrane. In many tissues, calcium channel antagonists, such as nifedipine, can reduce the release of arachidonic acid, prostaglandins, and leukotrienes that are evoked by various stimuli. Moncada, S., Flower, R. and Vane, J. in Goodman's and Gilman's Pharmacological Basis of Therapeutics, (7th ed.), MacMillan Publ. Inc., pp. 660-5 (1995).

Finally, calcium channel antagonists and either tachykinin, histamine or bradykinin antagonists exhibit synergistic effects in inhibiting neuroinflammation. The role of neurokinin receptors in mediating neuroinflammation has been established. The neurokinin₁ (NK₁) and neurokinin₂ (NK₂) receptor (members of the G-protein coupled superfamily) signal transduction pathway includes increases in intracellular calcium, thus leading to activation of calcium channels on the plasma membrane. Similarly, activation of bradykinin, (BK₂) receptors is coupled to increases in intracellular calcium in synoviocytes and chondrocytes. Thus, calcium channel antagonists interfere with a common mechanism involving elevation of intracellular calcium, part of which enters through L-type channels. This is the basis for synergistic interaction between calcium channel antagonists and antagonists to neurokinin, histamine, P₂Y and bradykinin₂ receptors.

Suitable calcium channel antagonists for the practice of the present invention include nisoldipine, nifedipine, nimodipine, lacidipine, isradipine and amlodipine. Suitable concentrations for these agents are set forth in Table 17.

**Table 17**

| Therapeutic and Preferred Concentrations of Spasm Inhibitory Agents | | |
|---|---|---|
| Class of Agent | Therapeutic Concentrations (Nanomolar) | Preferred Concentrations (Nanomolar) |
| Calcium Channel Antagonists: | | |
| nisoldipine | 1-10,000 | 100-1,000 |
| nifedipine | 1-10,000 | 100-5,000 |
| nimodipine | 1-10,000 | 100-5,000 |
| lacidipine | 1-10,000 | 100-5,000 |
| isradipine | 1-10,000 | 100-5,000 |
| amlodipine | 1-10,000 | 100-5,000 |

### II. Agents For The Inhibition Of Cartilage Degradation

Recent advances in the understanding of the biochemistry and molecular biology of inflammation and cartilage destruction have implicated a role for numerous endogenous cytokines. Multiple pro-inflammatory mediators that have been implicated in producing loss of cartilage in the inflamed joint are the cytokines, TNF-α, IL-1, IL-6 and IL-8. Elevated levels of a number of these pro-inflammatory cytokines appear rapidly in the synovial fluid of acutely injured knee joints and remain elevated in patients for at least 4 weeks (Cameron, ML et al., "Synovial fluid cytokine concentrations as possible prognostic indicators in the ACL-deficient knee," Knee Surg. Sports Traumatol. Arthroscopy 2:38-4-4 (1994)). These cytokines are produced locally in the joint from several activated cell types, including synovial fibroblasts, synovial macrophages, and chondrocytes. The locally produced cytokines mediate pathophysiological events in acute and chronic inflammatory conditions and are important autocrine and paracrine mediators of cartilage catabolism. The actions of these cytokines are characterized by their ability to cause multiple effects on distinct cellular targets and by their ability to interact in either a positive or negative synergistic manner with other cytokines. IL-1 and TNF-α are particularly important since they also initiate chondrodestructive effects by disrupting the balance between the normal turnover and destruction of cartilage matrix components by modulating the activity of endogenous proteins, e.g., matrix metalloproteinases (MMPs) and tissue inhibitor of metalloproteinase (TIMP). Cytokine control of cartilage homeostasis represents a highly regulated balance between active mediators acting on chondrocytes which determines whether matrix degradation or repair occurs.

Injury to the joint frequently produces an inflammatory response within the joint space which involves the synovial tissue and may lead to degradation of articular cartilage. Dramatic shifts in synovial and cartilage metabolism of the human knee have been described following joint injury and arthroscopic surgery (Cameron, M.L. et al. (1994), *supra;* Cameron, M.L. et al., "The natural history of the anterior cruciate ligament-deficient knee: Changes in synovial fluid cytokine and keratan sulfate concentrations," Am. J. Sports Med. 25:751-754 (1997)). Specific pro-inflammatory cytokine levels increase dramatically (up to 2-4 orders of magnitude) in knee joint synovial fluids during the acute inflammatory phase seen after anterior cruciate ligament (ACL) rupture. Significant changes also occur in concentrations of cartilage matrix molecules due to overproduction of matrix metalloproteinases (MMPs), such as collagenase and stromelysin-1, which are elevated in the synovial fluid of patients after acute trauma (Lohmander, L.S. et al., "Temporal patterns of stromelysin-1 tissue inhibitor, and proteoglycan fragments in human knee joint fluid after injury to the cruciate ligament or meniscus," J. Orthopaedic Res. 12:21-28 (1994)). Temporally, the changes in cytokines and cartilage matrix markers (e.g., proteoglycans) in synovial fluid, which are correlated with cartilage degeneration, are maximal in the acute injury period but persist for extended periods (3 months to one year), declining slowly and remaining greater than pre-injury baseline levels.

Trauma due to arthroscopic surgery itself causes significant post-surgical inflammation which reflects additional inflammatory activation of cells in the joint, including upregulation of cyclooxgenase-2 and other pro-inflammatory cytokines. A significant proportion (60-90%) of patients with rupture of the ACL show radiographic changes of the knee indicative of osteoarthritis (OA) 10-15 years after injury (Cameron, M.L. et al. (1994), *supra*)*.* Thus, the combined effects of initial joint injury and surgical trauma may induce a sustained inflammatory state and associated changes in cartilage matrix metabolism which appear to be causative factors resulting in the subsequent development of degenerative changes in articular cartilage and early development of osteoarthritis. The magnitude of this health problem is substantial since the total estimated number of arthroscopic procedures performed in the United States alone in 1996 was 1.8 million with an estimated growth rate of approximately 10% per annum. Thus, it is desirable to provide a pharmaceutical method to prevent degradation of articular cartilage within the joint.

While post-surgical pain and inflammation are recognized as significant clinical problems, current pharmacological treatment regimens for arthroscopic surgery are only directed at acute postoperative analgesia. Existing surgical treatment modalities do not address the chronic inflammatory state that is induced post-operatively and the need to inhibit cartilage destruction of the operatively treated joint. There is a clear need, therefore, to develop an effective, integrated drug therapy that will address both the acute and chronic aspects of pain and inflammation, as well as pathological changes in cartilage metabolism in the injured and operatively treated joint.

There is described a method is provided for reducing or preventing destruction of articular cartilage in a joint, by administering directly to the joint of a patient a composition which includes one or more metabolically active chondroprotective agents together with one or more agents for the inhibition of pain and/or inflammation, as previously described, or alternatively a combination of two or more metabolically active chondroprotective agents, at least one of which promotes cartilage anabolic processes and at least one of which is an inhibitor of cartilage catabolic processes, in a pharmaceutically effective carrier for intra-articular delivery. Metabolically active agents include, but are not limited to, all compounds that act directly or indirectly to modulate or alter the biological, biochemical or biophysical state of a cell, including agents that alter the electrical potential of the plasma membrane, the ligand binding or enzymatic activity of cellular receptors, intracellular or extracellularly located enzymes, protein-protein interactions, RNA-proteins interactions, or DNA-protein interactions. For example, such agents may include receptor agonists that initiate signal transduction cascades, antagonists of receptors that inhibit signalling pathways, activators and inhibitors of intracellular or extracellular enzymes and agents that modulate the binding of transcription factors to DNA.

Specifically, there is described a pharmacological method of treating the injured or operatively treated joint using a combination of cartilage protective agents delivered locally to achieve maximal therapeutic benefit. The use of a combination of chondroprotective agents overcomes the limitations of existing therapeutic approaches that rely upon on the use of a single agent to block a multifactorial cartilage destructive process in which a shift between synthesis and degradation, in favor of catabolic processes has occurred. This uniquely utilizes the approach of combining of agents that act simultaneously on distinct molecular targets to promote cartilage anabolism and inhibit unregulated or excess cartilage catabolic processes to achieve maximum inhibition of inflammatory processes and maintain cartilage homeostasis, thereby achieving a chrondroprotective effect within the joint Inhibition of a single molecular target or biochemical mechanism known to induce cartilage destruction (catabolism), such as inhibiting interieukin-1(H,-1) binding to the IL-1 receptor, will likely not be optimal, since, for example, the actions of TNF-α mediated through its unique receptor shares many overlapping pro-inflammatory and cartilage catabolic functions with IL-1 and is also recognized as a major mediator of cartilage destruction in the joint. Similarly, utilizing pharmaceutical agents that only enhance cartilage anabolic processes in the absence of inhibiting catabolic processes will not optimally counteract catabolic factors present within the injured joint.

Specifically, there is described pharmaceutical compositions of metabolically active chondroprotective agents that are based upon a combination of at least two agents that act simultaneously on distinct molecular targets. In a representative embodiment, at least one agent is a cytokine or growth factor receptor agonist which directly provides anti-inflammatory activity and/or promotes cartilage anabolic processes and at least a second agent is a receptor antagonist or enzyme inhibitor that acts to inhibit pro-inflammatory and/or cartilage catabolic processes. A representative drug combination includes at least one agent drawn from a class of anti-inflammatory/anabolic cytokines which act functionally to suppress the role of pro-inflammatory cytokines in the joint, promote cartilage matrix synthesis and inhibit matrix degradation. These receptor agonists include, but are not limited to, specific anti-inflammatory and anabolic cytokines, such as the interleukin (IL) agonists (e.g., IL-4, IL-10 and IL-13) and specific members of the transforming growth factor-β superfamily (e.g., TGFβ and BMP-7), insulin-like growth factors (e.g., IGF-1) and fibroblast growth factors (e.g., bFGF). At least a second agent is drawn from a class of receptor antagonists or enzyme inhibitors that acts to inhibit and reduce the activity or the expression of a pro-inflammatory molecular target (e.g., the IL-1 receptor antagonists, TNF-α receptor antagonists, cyclooxygenase-2 inhibitors, MAP kinase inhibitors, nitric oxide synthase (NOS) inhibitors, and nuclear factor kappaB (NFκB) inhibitors). The metabolically active agents include both functional agonists and antagonists of receptors located on the surfaces of cells, as well as inhibitors of membrane bound or extracellularly secreted enzymes (e.g., stromelysin and collagenase). In addition, many of the agents are directed at novel targets which are the intracellularly localized enzymes and transcription factors that transduce and integrate the signals of the surface receptors, including inhibitors of the enzymes NOS, COX-2, and mitogen-activated protein kinases (MAPK) and inhibitors of protein-DNA interactions such as the transcription factor NFκB. This method allows the integrity of cartilage to be maintained by simultaneously promoting cytokine-driven anabolic processes and inhibiting catabolic processes.

The multiple drug combination can be delivered locally by intra-articular injection or via infusion, including administration periprocedurally (ie., pre-operatively and/or intra-operatively and/or post-operatively) during surgical arthroscopic procedures, alone or coupled with post-operative sustained delivery, such as by a regulated pump delivery system or other sustained release delivery system. Sustained release delivery systems may include, but are not limited to, microparticles, microspheres or nanoparticles composed of proteins, liposomes, carbohydrates, synthetic organic compounds, or inorganic compounds. Thus, in some embodiments, a combination of agents is to be delivered via injection or infusion, alone or together with analgesic and/or anti-inflammatory agents. The rapid onset of action achieved by direct, local delivery of the chondroprotective agents at or closely following the time of injury (e.g., perioperatively) has the potential to inhibit initial processes before they can trigger subsequent responses and thereby preemptively limit local tissue damage and the subsequent loss of cartilage.

Advantages of this include 1) a combination drug therapy directed to the multifactorial causes of cartilage destruction during acute and chronic conditions; 2) the combination of chondroprotective agents may be combined with anti-inflammatory and analgesic agents; 3) local delivery of the drug combination achieves an instantaneous therapeutic concentration of chondroprotective agents within the joint; 4) using an irrigation solution periprocedurally provides continuous maintenance of drug levels within the joint in a therapeutically desirable range during an arthroscopic surgical procedure; 5) local delivery permits a reduction in total drug dose and dosing frequency compared to systemic delivery; 6) local site-directed delivery to the joint avoids systemic toxicity and reduces adverse effects; and 7) direct, local delivery to the joint enables use of novel, pharmaceutically active peptides and proteins, including cytokines and growth factors, which may not be therapeutically useful if limited to systemic routes of administration.

### 1. Interleukin-1 (IL-1) Receptor Antagonists

The interleukin IL-1 exists in two forms, IL-1α and IL,1β, which are polypeptides derived from separate gene products which share a similar spectrum of immunoregulatory and pro-inflammatory functions. IL-1 is a 17 kD polypeptide that can both act upon and be produced by a number of cell types in the joint, including synovial fibroblasts and macrophages, chondrocytes, endothelial cells and monocytes and macrophages. There is substantial evidence that IL-1 plays a pivotal role in joint inflammation and in the pathophysiological loss of articular cartilage that occurs in the injured joint.

The actions of both forms of this cartilage destructive cytokine are mediated by one of two IL receptors (IL,-IR), type I IL-1 or type II IL-1 receptors. IL-1 receptors are structurally distinct and belong to a separate superfamily characterized by the presence of immunoglobulin binding domains. These receptors bear close amino acid homology with other receptors containing immunoglobulin domains. Expression of the larger type I IL-1 receptor is present on T cells and fibroblasts while the smaller type II IL-1 receptor is present on B cells, monocytes, neutrophils, and bone marrow cells.

Type II IL-1 receptors bind IL-1β with high affinity, but IL-1β binding does not initiate intracellular signal transduction as it does upon binding to the type I IL-1 receptor. In contrast, the type II receptor serves as a precursor for a soluble IL-1 binding factor that has been shown to be shed from cells and this soluble receptor acts as a physiological IL-1β antagonist. A naturally occurring IL-1 binding protein has been described which corresponds to the soluble external portion of the type II receptor.

A naturally occurring secreted, soluble ligand that binds to IL-1 receptors, alternatively referred to as the IL-1 receptor antagonist (sIL-IRA, IL-1Ra, IL-1ra), has been cloned, sequenced and found to encode a 22 kD protein. IL-1Ra competitively inhibits the binding of IL-1α and IL-1β to both type I and II IL-1 receptors. IL-IRa is a pure receptor antagonist since its binding to the receptor does not activate the cellular signal transduction machinery of membrane associated IL-1 receptors. Despite high affinity binding of this protein to the IL-1Rs, a 10-100 fold molar excess is required to inhibit IL-1 biological responses of cells that express the type I IL-1R. Cells known to produce IL-1Ra include monocytes, neutrophils, macrophages, synoviocytes and chondrocytes. IL-1Ra has been shown to inhibit PGE₂ synthesis, induction of pro-inflammatory cytokines and MMPs, and nitric oxide production. Secreted IL-1Ra is released *in vivo* during experimentally induced inflammation. Importantly, IL-1Ra is expressed in synovial tissue and is present in normal human synovial fluid. In patients with knee injuries, levels of IL-1Ra in the synovial fluid dramatically increase in the acute phase after injury, and subsequently decrease to below normal levels in sub-acute and chronic states. Thus, the IL-1Ra has been shown to play a physiological role in responses of the joint to injury.

IL-1 is considered the dominant cartilage destructive cytokine that plays a pivotal role in joint destruction due to its ability to stimulate the production of degradative enzymes and pro-inflammatory cytokines by both chondrocytes and synoviocytes. Moreover, IL-1β is a potent inhibitor of proteoglycan and collagen synthesis by chondrocytes. At the cellular level, IL-1β-induced responses of synovial fibroblasts include increased production of PGE2, collagenase and other neutral proteases and the upregulation of pro-inflammatory cytokines, IL-6 and IL-8.

IL-1, which is present in the joint fluid of patients with arthritic diseases, stimulates chondrocytes to: 1) synthesize elevated amounts of enzymes such as stromelysin, fibroblast and neutrophil collagenase and plasminogen activator, and 2) inhibit synthesis of plasminogen activator inhibitor-1 and TIMP. In addition, IL-1β is a potent inhibitor of the synthesis of matrix constituents such as type II collagen, the predominant form of collagen in articular cartilage, and proteoglycans. The imbalance between the levels of inhibitors and proteases leads to an increase in the amount of active proteases. This increase, combined with a suppression of matrix biosynthesis, results in degradation of cartilage. In animal studies, injection of IL-1 into rabbit knee joints causes depletion of proteoglycan from the articular cartilage.

Since IL-1 is one of the key cytokines involved in the pathogenesis of chronic synovitis and cartilage degradation, reducing its production or blocking its action represents an appropriate strategy for new treatments to reduce synovial inflammation and to provide a chondroprotective effect. A variety of therapeutic approaches for antagonizing the interaction of the agonist, IL-1, with its natural membrane bound receptor can be utilized which include: 1) naturally occurring specific inhibitors of IL-1 activity that have been characterized to date, including IL-1Ra and soluble IL-1 receptors; 2) anti-IL-1 Abs; and 3) small molecule antagonists which may be either peptidic or nonpetidic.

The ability to block the actions of this key cytokine will have effects on many cell types in the joint (e.g., synovial fibroblasts and chondrocytes), thus inhibiting subsequent pathological effects such as infiltration of inflammatory cells into the joint, synovial hyperplasia, synovial cell activation, as well as cartilage breakdown and inhibition of cartilage matrix synthesis. An IL-1 receptor antagonist should block the propagation of the inflammatory response by iL-1 and thereby interrupt the disease process. The therapeutic potential of a number of IL-1 receptor antagonists have been established in animal models of inflammation and arthritis (RA and OA). Patients suffering from RA have improved clinically following a subcutaneous injection of IL-1Ra or an intra-articular injection of soluble Type I IL-1R.

The effects of IL-1β and IL-1Ra depend on their respective local concentrations. In the supernatants of RA synovium pieces, IL-1β levels were threefold higher than those of IL-1Ra. Thus, the spontaneous local production of IL-IRa is not sufficient to inhibit IL-1β effects because a larger (10 to 100-fold) molar excess of IL-1Ra is required to inhibit IL-1-induced biological responses in cells that express type I IL-1R. Thus, high doses of IL-1Ra have been used *in vivo* to block IL-1 in human volunteers in patients with RA. IL-1Ra present locally in the synovium provides a negative signal, down-regulating at least part of the IL-1-mediated processes in synovitis, such as leukocyte accumulation in the inflamed tissue, PGE₂ production and collagenase production by synovial cells. A chondroprotective effect of IL-1Ra has been demonstrated using direct injection of IL-IRa into the joint in a canine ACL model and by employing a gene therapy approach based upon transfection of the IL-1Ra gene into human synovial fibroblasts.

There is described the local delivery of an IL-1 soluble receptor protein, which is comprised of an extracellular domain of a IL-1R, and which is capable of binding an IL-1 cytokine molecule in solution. In particular, and by way of example, a soluble human IL-1 receptor (shuIL-1R) polypeptide comprising essentially the amino acid sequence 1-312, as disclosed within US Patent No. 5,319,071 and US Patent No. 5,726,148, is disclosed herein for use in combination with one or more drugs chosen from either an anti-inflammatory class, anti-pain class, or chondroprotective class. Alternatively, the local delivery of a fusion protein consisting of the sIL-1R binding domain polypeptide is proposed for use to promote chondroprotection, as disclosed in US Patent 5,319,071. In addition, the local delivery of an IL-1 receptor antagonist is as disclosed within US Patent 5,817,306. The shuIL.-IR soluble receptor has been shown to bind IL-1 with nanomolar affinity. Local delivery of a therapeutically effective concentration of an IL-1R soluble receptor, such as shuIL-IR, may occur by direct injection of the joint or in an irrigation solution (e.g., during an arthroscopic surgical procedure) in combination with one or more chondroprotective drugs, anti-inflammatory drugs, or anti-pain drugs and is disclosed herein as a cartilage protective agent when applied locally to tissues of the joint in a variety of inflammatory or pathophysiological conditions. Such treatment will preemptively inhibit IL-1 stimulation of production of collagenase-1 and stromelysin-1. Employing a wholly different method based on gene delivery for local production of type 1 soluble receptors for IL-1 and/or TNF-α, it has been found that the presence of soluble receptors for these cytokines are able to confer protection to the rabbit knee joint during the acute inflammatory phase of antigen induced-arthritis.

IL₋1 receptor antagonist peptides (11-15 amino acids) that bind specifically with high affinity to the human type I IL-1 receptor are suitable for use. These small peptides provide a number of advantages over larger recombinant IL-1 soluble receptors or recombinant IL-Ira, including ease and cost of synthesis and the ability to penetrate biological barriers. Two of the most potent peptides, based on *in vitro* efficacy are; Ac-FEWTPGWYQJYALPL-NH2 (AF12198, IC₅₀=2nM) and Ac-FEWTPGWYQJY-NH₂ (AF11567). AF11567 is a truncated version of AF12198, lacking the 4 C-terminal residues and exhibiting slightly lower affinity for the type I 1L-1 receptor but possessing a similar plasma half-life of 2.3-2.6 hrs. Poor solubility and rapid metabolism appeared to limit the *in vivo* efficacy of AF12198 when administered systemically via intravenous infusion. These limitations are in part overcome through direct, local delivery methods such as injection into the intra-articular joint space or by inclusion in the surgical irrigation fluid or other infusion. Examples of IL-1 receptor antagonist agents are listed below. For all modes of local delivery (i.e., injection, infusion and irrigation) the optimal dose and/or concentration of each suitable agent is that which is therapeutically effective. As an example, for each of the listed agents, the preferred and most preferred concentrations of an irrigation solution containing the listed agent are provided, such concentrations expected to be therapeutically effective.

**Table 18**

| Therapeutic and Preferred Concentrations of Interleukin-1 Receptor Antagonists | | |
|---|---|---|
| Compound | Therapeutic Concentrations (nM) | Most Preferred Concentrations (nM) |
| rshuIL-1R | 0.2-2000 | 200 |
| rhIL-1ra | 0.2-2000 | 200 |
| anti-IL1-antibody | 0.2-2000 | 200 |
| AF12198 | 0.2-2000 | 200 |
| AF11567 | 0.2-2000 | 200 |

### 2. Tumor Necrosis Factor (TNF) Receptor Antagonists

TNF-α, a cytokine mainly produced by activated macrophages, has many biological actions including transcriptional regulation of several genes that are mediated by specific TNF receptors, as well as immunoregulatory activities. Originally, two different receptors termed TNF-R1 and TNF-R2 were cloned and characterized and also found to be produced as soluble receptors.

Receptors in this family are single transmembrane proteins with considerable homology in their extracellular domains whereas their relatively short intracellular domains bear very little sequence homology. The actions of TNF are the result of the factor binding to cell surface receptors that are present on virtually all cell types that have been studied. Two receptors have been identified and cloned. One receptor type, termed TNFR-II (or Type A or 75kDa) encodes a transmembrane protein of 439 amino acids and has an apparent molecular weight of 75kDa. The second receptor type, terned TNFR-1 (or Type B or 55 kDa) shows an apparent molecular weight of 55 kDa and encodes a transmembrane protein of 426 amino acids. TNFR1 contains an intracellular domain which can initiate signalling through the NF-xB pathway.

Both of the TNF receptors exhibit high affinity for binding TNFα. Soluble TNF receptors (sTNFR) have been isolated and proved to arise as a result of shedding of the extracellular domains of the membrane bound receptors. Two types of sTNFR have been identified and designated as sTNFR1 (TNF BPI) and sTNFRII (TNF BPII). Both of these soluble receptor forms have been shown to represent the truncated forms of the two types of TNFR described above.

TNF-α plays a central role in the sequence of cellular and molecular events underlying the inflammatory response and cartilage destruction. Many of the effects of TNF-α overlap with the pro-inflammatory effects of IL-1. Among the pro-inflammatory actions of TNF-α is its stimulation of the release of other pro-inflammatory cytokines including IL-1, IL-6 and IL-8. TNF-α also induces the release of matrix metalloproteinases from neutrophils, fibroblasts and chondrocytes that degrade cartilage, in part through the stimulation of collagenase. Furthermore, TNF-α upregulates COX-2 in normal human articular chondrocytes and synovial fibroblasts, resulting in increased PGE2 production.

This cytokine, along with IL-1, is considered to initiate and produce pathological effects on cartilage in the joint, including leukocyte infiltration, synovial hyperplasia, synovial cell activation, cartilage breakdown and inhibition of cartilage matrix synthesis. In particular, during synovial inflammation, increased levels of TNF-α are found in synovial fluid of joints and increased production of TNF-α by synovial cells occurs. Thus, local delivery of a soluble TNF-α receptor in an irrigation solution, infusion, or injection will bind free TNF-α and function as an antagonist of TNF receptors in the surrounding tissue, thus providing a cartilage protective effect.

There is described the use of functional antagonists of TNF-α that act extracellularly to block interaction of the ligand with their cognate membrane receptors either by scavaging of available free ligand or by direct competitive interaction with the receptor itself, alone or in combiantion with other agents to provide a chondroprotective effect. A variety of therapeutic approaches for antagonizing the interaction of the agonist, TNF-α, with its natural membrane bound receptor can be utilized which include: 1) the use of naturally occurring specific inhibitors of TNF-α activity that have been characterized to date, including soluble TNF-α receptors; 2) the use of anti- TNF-α antibodies and 3) the use of small molecule antagonists which may be either peptidic or nonpeptidic.

There is described the use of a chimeric soluble receptor (CSR) protein, in which the extracellular domain of a TNF receptor, which possesses binding activity for a TNF molecule, is covalently linked to a domain of an IgG molecule. In particular, and by way of first example, a chimeric polypeptide (recombinant chimera) comprising the extracellular domain of the TNF receptor extracellular polypeptide coupled to the CH2 and CH3 regions of a mouse IgG1 heavy chain polypeptide could be used, as disclosed in US Patent No. 5,447,851. The chimeric TNF soluble receptor (also termed the "chimeric TNF inhibitor" in US Patent No. 5,447,851) has been shown to bind TNF-α with high affinity and has been demonstrated to be highly active as an inhibitor of TNF-α biological activity. In addition, a second example is a chimeric fusion construct comprised of the ligand binding domain of the TNF receptor with portions of the Fc antibody (termed Fc fusion soluble receptors) that have been created for TNF-α receptors. There is described the use of a soluble TNF receptor. Fc fusion protein, or any modified forms, as disclosed in US Patent No. 5,605,690. The molecular form of the active soluble receptor fusion protein can be either monomeric or dimeric. Existing studies establish that such a soluble TNF receptor:Fc fusion protein retains high binding affinity for TNF-α

Within the context of defining soluble receptors as pharmacological antagonists, the term soluble receptors includes, but is not limited to: (1) soluble receptors which correspond to naturally (endogenous) produced amino acid sequences or soluble fragments thereof consisting of an extracellular domain of full-length membrane receptor, (2) recombinant soluble receptors which are truncated or partial sequences of the full length, naturally occurring receptor amino acid sequences which retain the ability to bind cognate ligand and retain biological activity and analogs thereof, and (3) chimeric soluble receptors which are recombinant soluble receptors comprised of truncated or partial sequences corresponding to a portion of the extracellular binding domain of the full length receptor amino acid sequences attached through oligomers (e.g., amino acids) to a sequence corresponding to a portion of an IgG polypeptide (e.g., IgG hinge and Fc domain) which retain biological activity and the ability to bind cognate ligand.

Soluble, extracellular ligand-binding domains of cytokine receptors occur naturally in body fluids and are thought to be involved in the regulation of the biologic activities of cytokines. The naturally occurring existence of soluble, truncated forms of a number of hematopoietic cytokine receptors has been reported (IL-1R, IL-4R, IL-6R, TNFR). For example, soluble TNFR is found at concentrations of about 1-2 ng/ml in the serum and urine of healthy subjects. Lacking signal transduction functions, these cytokine binding proteins arise as a result of alternative splicing of the mRNA for the complete receptor sequence (membrane-bound form) or as a result of proteolytic cleavage and release of the membrane-bound form of the receptor. Although the *in vivo* functions of these soluble truncated receptors are not fully established, they appear to act as physiological antagonists of their complementary endogenous cytokines. This antagonism occurs because (1) scavenging of the free ligand through binding to its cognate soluble receptor reduces the effective free concentration available to the membrane-bound receptors and (2) actions of the cytokines are only produced subsequent to binding to cell surface receptors.

The TNF-α soluble receptor will function as a natural antagonist of the TNF-R1 and TNF-R2 by competing with these cell surface receptors for common pool of free ligand. Pharmacologically, the TNF soluble receptor will function as an antagonist through its ability to decrease free ligand bioavailability rather than by a mechanism of competitive inhibition (ie., competing with an endogenous ligand for a common binding site on a membrane receptor). Addition of a therapeutically effective amount of the TNF soluble receptor to the joint should effectively neutralizing the biological activity of the ligand. Experiments in which recombinant soluble receptors have been administered *in vivo* have demonstrated the capacity to inhibit inflammatory responses and act as antagonists.

Agents suitable as chondroprotective agents for use in combination with other chondroprotective, anti-pain and/or anti-inflammatory agents to inhibit cartilage destruction include soluble TNFR, the human chimeric polypeptide (recombinant chimera) comprising the extracellular domain of the TNF-α receptor (p80) linked to the Fc portion of human IgGl, and the anti-TNF-α antibody. For all modes of local delivery (i.e., injection, infusion and irrigation) the optimal dose and/or concentration of each suitable agent is that which is therapeutically effective. As an example, for each of the listed agents, the preferred and most preferred concentrations of an irrigation solution containing the listed agent are provided, such concentrations expected to be therapeutically effective.

**Table 19**

| Therapeutic and Preferred | ConcentrationsTNF-Receptor Antagonists | |
|---|---|---|
| Compound | Therapeutic Concentrations (nM) | Most Preferred Concentrations (nM) |
| sTNFR | 0.1-2000 | 200 |
| chimeric rhTNFR:Fc | 0.1-2000 | 200 |
| anti-TNF-α antibody | 0.2-2000 | 200 |

### 3. Interleukin Receptor Agonists

Some cytokines are signaling glycoproteins that are important mediators of synovial inflammation and cartilage destruction. Recent analysis of the mechanism of cartilage destruction suggests that not only is the absolute level of pro-inflammatory master cytokine, IL-1, important in determining loss of cartilage, but that cytokine control of cartilage homeostasis is governed by the balance of catabolic and anabolic regulatory cytokines, and anabolic growth factors. If the balance between IL-1β and IL-1Ra production is altered in the inflammatory state in favor of IL-1β, then it will contribute to the pathogenesis of chronic inflammatory conditions and cartilage destruction, such as is known to occur after knee joint surgery. Potential therapeutic agents that would inhibit production of the pro-inflammatory cytokines at the sites of inflammation within the joint include the anti-inflammatory cytokines, IL-4, IL-10, and IL-13. These cytokines have been observed to greatly reduce articular cartilage destruction *in vitro* and *in vivo* via their effect on a range of pathways that reduce the impact of IL-1. Thus, anti-inflammatory cytokines such as IL-4, IL-10, and IL-13, may be useful in reducing inflammation by: 1) reducing the production of pro-inflammatory cytokines, and 2) inducing the production of natural anti-inflammatory cytokines such as IL-1Ra, as recently demonstrated *in vivo* for IL-4.

IL.-4 appears to attenuate the inflammatory process in the synovium of rheumatoid arthritis (RA) patients. In rheumatoid synovium, IL-4 has been shown to inhibit the production of pro-inflammatory cytokines by pieces of synovium, to inhibit proliferation of synoviocytes and decrease bone resorption. IL-4 may promote a direct chondroprotective effect through suppression of matrix metalloproteinase-3 (MMP-3) synthesis in human articular chondrocytes. A cell culture system employing human articular chondrocytes was used to evaluate the effect of IL-4 on IL-1-induced production of MMP-3 and tissue inhibitor of metalloproteinase-1 (TIMP-1). It was found that IL-4 suppressed IL-1-stimulated MMP-3 protein and enzyme activity. In addition, IL-4 suppressed IL-1-induced MMP-3 mRNA. Induction of iNOS can be inhibited by IL-4, IL-10 and IL-13. Thus, IL-4 may be characterized as a protective mediator of joint destruction seen in inflammatory joint diseases.

Furthermore, the effects of IL-4 on the balance of IL-1 regulatory cytokine levels have also been found to support a cartilage protective role. IL-4 and IL-10 were found to suppress the production of inflammatory cytokines by freshly prepared rheumatoid synovial cells. While each interleukin was effective alone, the combination of IL-4 and IL-10 synergistically inhibited the IL-1 and TNF-α stimulated production of IL-6 and IL-8, without effects on cell viability. The addition of IL-4 to RA synovium cultures increased the production of IL-1Ra and decreased that of IIL-1β. *In vivo* treatment with IL-4 has recently been reported to promote a reduction in rat experimental arthritis by acting differentially on the IL-1β/IL-IRa balance. IL-13, another cytokine that shares many properties with IL-4, also induced IL-1Ra in RA synovium. Therefore, the local delivery of an IL-4 and IL-13 combination may provide a synergistic therapeutic value.

IL-10 has a number of properties that indicate that it is a good candidate to inhibit cartilage destruction. It inhibits both IL-1 and TNF-α release and stimulates TEMP-1 production while inhibiting MMP-2. The production of IL-10 inside the RA synovium has recently been reported and anti-inflammatory effects of IL-10 have been characterized. IL.-10 suppressed IL-1β production in an *ex vivo* RA model using pieces of synovium, but to a lesser extent than IL-4.

A protective effect of IL-4 and IL-10 treatment on cartilage destruction has been found in animal models of arthritis employing non-local methods of delivery for the cytokines. In a murine collagen-induced arthritis model, combination treatment of IL-4 and IL-10 produced substantial improvement. In addition to suppression of macroscopic signs of inflammation, combined treatment with IL-4 and IL- 10 also reduced cellular infiltrates in the synovial tissue and caused pronounced protection against cartilage destruction. Moreover, levels of mRNA for TNF-α and IL-1 were highly suppressed both in the synovial tissue and in the articular cartilage. In contrast, levels of IL-1 receptor antagonist (IL-1Ra) mRNA remained elevated, which suggests that the mechanism of protection may be related to suppressed production of TNF-α and IL-1, with concomitant up-regulation of the IL-1Ra/IL,-1 balance. These data are consistent with a dominant role of IL-10 in the endogenous suppression of the inflammatory response and destruction of articular cartilage, and a combined treatment with IL-4 and IL-10 appears of potential therapeutic value.

The role of endogenous IL-4 and IL-10 and the therapeutic effect of addition of these cytokines on joint inflammation and cartilage destruction in the early stages of the macrophage dependent murine streptococcal cell wall (SCW) arthritis model have also been investigated. It was demonstrated that endogenous IL-10 plays a role in the regulation of SCW arthritis. Addition of exogenous IL-10 further enlarged the suppressive effect of endogenous IL-10. An even more pronounced effect was found with the combination of IL-4 and IL-10. The combination resulted in a reduced swelling and an increase in chondrocyte proteoglycan synthesis. Treatment with the combination of IL-4 and IL-10 substantially diminished levels of TNF-α, as occurs for IL-10 treatment alone, but also resulted in strongly reduced IL-1β levels in the synovium, an added effect of potential clinical benefit. Overall, the data is consistent with a role for both IL-4 and IL-10 as chondroprotective agents delivered locally to joints to prevent cartilage destruction, and indicates a combination containing IL-4 and IL-10 may provide a greater potential therapeutic value than either agent alone.

Severe combined immunodeficient (SCID) mice were used as a model to assess the effect of IL-4 or IL-10 injection on cartilage degradation and mononuclear cell (MNC) recruitment to human rheumatoid synovium *in vivo.* Human rheumatoid synovium and cartilage from five rheumatoid arthritis patients were injected with recombinant human IL-4 (rhIL-4, 100 ng; rhIL-10, 100 ng), a combination of IL-4 and IL-10, or TNF-alpha (1000 U), or phosphate-buffered saline twice a week for 4 weeks. It was found that a combination of human IL-4 and IL-10 inhibited cartilage degradation and invasion by human synovial tissue, establishing the chondroprotective properties of these interleukin agonists.

Human IL-13 has been cloned and sequenced and has been found to share many of the properties of IL-4. IL-13 is about 25% homologous to IL-4. Like IL-4, IL-13 decreases the production of pro-inflammatory cytokines, including IL-1 and TNF-α, by synovial fluid mononuclear cells. IL-13 exhibits anti-inflammatory effects *in vivo* and thus has therapeutic potential in the treatment of cartilage destruction in the joint.

Compounds useful as IL-4, IL-10 and IL-13 agonists include naturally occurring human IL-4, IL-10 and IL-13, human recombinant IL-4 (rhIL-4), rhE-10, and rhIL-13 as well as partial sequences thereof, or peptide sequences which have been constructed using recombinant DNA techniques to recognize the IL-4, EL-10 and IL-13 receptors and are capable of activating these receptors on a cell surface. This specifically includes multispecific molecules comprised of an anti-Fc receptor portion and an anti-IL-4, anti-%-10, and anti-IL-13 receptor portion, wherein at least one portion is constructed using recombinant DNA techniques. Within the context of defining interleukin agonists as pharmacological agonists, the term interluekin agonist includes, but is not limited to: (1) peptide sequences which correspond to naturally (endogenous) produced amino acid sequences or fragments thereof, (2) recombinant interleukins which are truncated or partial sequences of the full length naturally occurring interleukin amino acid sequences which retain the ability to bind cognate receptor and retain biological activity and analogs thereof, and (3) chimeric interleukins which are recombinant polypeptides comprised of truncated or partial sequences corresponding to a portion of the of the full length amino acid sequences attached through oligomers (e.g., amino acids) to a sequence corresponding to a portion of an IgG polypeptide (e.g., IgG hinge and Fc domain) which retain the ability to bind the cognate receptor and retain biological activity.

Examples of interleukin agonists suitable for the present invention are listed below. For all modes of local delivery (i.e., injection, infusion and irrigation) the optimal dose and/or concentration of each suitable agent is that which is therapeutically effective. As an example, for each of the listed agents, the preferred and most preferred concentrations of an irrigation solution containing the listed agent are provided, such concentrations expected to be therapeutically effective.

**Table 20**

| Therapeutic and Preferred Concentrations Interleukin Agonists | | |
|---|---|---|
| Compounds | Therapeutic Concentrations (nanomolar) | Preferred Concentrations (nanomolar) |
| rhuman IL-4 | 0.5-5,000 | 5-500 |
| rhuman IL-10 | 0.5-5,000 | 5-500 |
| rhuman IL-13 | 0.5-5,000 | 5-500 |

### 4. Transforming Growth Factor-β Superfamily Agonists

Transforming growth factor-β (TGF-β) subfamily members are 25 kD pleiotropic, multifunctional proteins capable of influencing a variety of cellular functions and are known to be involved in tissue repair and remodeling. In many cases, it enhances the cell interaction with the extracellular matrix (ECM) and increases accumulation of ECM by stimulating production and secretion of ECM proteins and protease inhibitors. TGF-β also has been shown to have synergistic interactions with other cytokines, generally showing anti-inflammatory activities. Multiple isoforms of TGF-β have been identified which share close amino acid sequence homologies. TGF-β1, TGF-β2, and TGF- β3 have been found in human tissue and are active in mammalian cells, although differing in binding affinity.

Members of the TGF- β subfamily are potent modulators of chondrocyte proliferation, differentiation and extracellular matrix accumulation. In cartilage organ cultures, TGF-β1 regulates metabolism of proteoglycans and stimulates collagen and glycosaminoglycan synthesis by rabbit articular chondrocytes. In addition, TGF-β1 increases TIMP expression in human articular chondrocytes and down-regulates expression of IL-1 receptors in articular cartilage.

Bone morphogenetic proteins (BMPs) are multifunctional regulators of cell growth, differentiation and apoptosis that belong to the transforming growth factor (TGF)-β superfamily. More than a dozen members of the BMP protein family have been identified in mammals, which can be subclassified into several groups depending on their structures. BMP-2 and BMP-4 are highly similar to each other. BMP-5, BMP-6, osteogenic protein (OP)-1 (also called BMP-7), and OP-2BMP-8 are structurally similar to each other. Growth-differentiation factor (GDF)-5 (also termed cartilage-derived morphogenetic protein-1), GDF-6 (also cartilage-derived morphogenetic protein-2), and GDF-7 form another related group. In contrast to BMP-2, BMP-4, BMP-6, and OP-1BMP-7, which induce bone and cartilage formation *in vivo,* GDF-5, GDF-6, and GDF-7 more efficiently induce cartilage and tendon-like structures *in vivo* (Wolfman et al., 1997).

Members of the TGF-β superfamily exert their effects via binding to two types of serine/threonine kinase receptors, both of which are essential for signal transduction (Massague, 1998). The type II receptors are constitutively active kinases, which transphosphorylate type I receptors upon ligand binding. The type I receptors activate intracellular substrates such as Smad proteins and it is through this mechanism that specificity of intracellular signal transduction occurs. Seven different type I receptors have been isolated in mammals, which were originally termed activin receptor-like kinase (ALK)-1-ALK7. BMP type IA receptor (BMPR-IA or ALK-3) and BMP type IB receptor (BMPR-IB or ALK-6) are structurally similar to each other and specifically bind BMPs together with type II receptors. ALK-2 has been shown to bind activin, but recent data revealed that it is a type I receptor for certain BMPs, e.g., OP-1/BMP-7 (Macias-Silva et al., 1998). ALK-1 is structurally highly similar to ALK-2, but its physiological ligand is still unknown. ALK-5 and ALK-4 are type I receptors for TGF-β (TβR-I) and activin (ActR-IB), respectively. ALK-7 is structurally similar to ALK-4 and ALK-5, but its ligand has not been determined yet.

Naturally occuring TGF-P and BMP agonists as well as synthetic or human recombinant (rh) agonists suitable for use in the cartilage-protective solution may interact with any of the BMP receptors described above. As used herein, the term "TGF-β and BMP agonists" includes fragments, deletions, additions, amino acid substitutions, mutations, and modifications thereof which retain the biological characteristics of the naturally occurring human TGF-β and BMP agonist ligands. The TGF-β or BMP agonists may be used alone or in synergistic combination with other members of the TGF-β superfamily as anabolic cartilage agents (chondrogenic or promoting cartilage matrix repair) or in combination with inhibitory agents that block cartilage catabolism.

Type I receptors function as downstream components of type II receptors. The specificity of the intracellular signals by type I receptors is determined by a specific region in the serine/threonine kinase domain, termed the L45 loop. Thus, the structures of the L45 loop of BMPR-IA/ALK-3 and BMPR-IB/ALK-6 (BMPR-I group) are identical to each other, and they may transduce similar signals in cells. Similarly, the L45 loops of TβR-I/ALK-5, ActR-IB/ALK-4, and ALK-7 (TβR-I groups) are identical to each other, and they activate similar substrates (Chen et al., 1998). The L45 loops of ALK-1 and ALK-2 (ALK-1 group) are most divergent from the other type I receptors, but they activate substrates similar to that of the type I receptors of the BMPR-I group (Armes et al., 1999).

Various proteins may transduce signals from the TGF-β and BMP serine/threonine kinase receptors. Among them, the best-studied molecules are proteins of the Smad family. Eight different Smad proteins have been identified in mammals, and these proteins are classified into three subgroups, i.e., receptor-regulated Smads (R-Smads), common partner Smads (Co-Smads)> and inhibitory Smads. R-Smads are directly activated by type I receptors, from complexes with Co-Smads, and translocate into the nucleus. The Smad heteromers bind to DNA directly and indirectly via other DNA-binding proteins and thus regulate the transcription of target genes. Smad1, Smad5, and Smad8 are activated by BMPs, whereas Smad2 and Smad3 are activated by TGF-β. For example, Smad2, in combination with Smad4 that functions as a Co-Smad, is translocated to the nucleus where it activates the transcription of genes that mediate the biological effects of TGF. Smad6 and Smad7 are structurally distantly related to the other Smads and act as inhibitory Smads. It has been shown that BMPs induce new cartilage and bone formation *in vitro* and *in vivo* and regulate chondrocyte growth and differentiation. Furthermore, these proteins are also implicated in the cartilage repair process. Various studies have shown that BMPs also promote and maintain the chondrogenic phenotype, which is indicated by their ability to stimulate proteoglycan synthesis in chick limb bud cells culture and fetal rat chondroblasts, as well as in rabbit and bovine articular chondrocytes. The importance of BMPs for cartilage and bone formation has been proven by transgenic approach in which specific BMP gene knockouts were studied.

One member of the BMP family, osteogenic protein (OP-1 or BMP-7), appears particularly important for cartilage homeostasis under normal and pathological conditions, such as during repair of cartilage. OP-1 appears to be the only member of the BMP family, along with cartilage-derived morphogenetic proteins, which is expressed by adult articular chondrocytes (Chubinskaya,S., J. Histochemistry and Cytochemistry 48: 239-50 (2000)). OP- was originally purified from bone matrix and shown to induce cartilage and bone formation. The human OP-1 gene has been cloned and biologically active recombinant OP-1 homodimers have been produced. Human recombinant OP-1 can stimulate synthesis of aggrecan and collagen Type II by human articular chondrocytes *in vitro.* It can also counteract the deleterious effects of IL-1 on the metabolism of these chondrocytes and block bovine cartilage damage mediated by fibronectin fragments. This effect was demonstrated by studying the effects of recombinant human OP-1 on the production of proteoglycan, prostaglandin E2, and IL-1 receptor antagonist by human articular chondrocytes cultured in the presence of interleukin-lbeta. Treatment of human articular chondrocytes with OP-1 was effective in overcoming the down-regulation of proteoglycan synthesis induced by low doses of IL-1β. Furthermore, a study found that OP-1 stimulates the synthesis of hyaluronan and CD44, other molecules required for matrix assembly by human chondrocytes. These studies of the expression and regulation to OP-1 in human adult cartilage suggest a role for OP-1 in cartilage protection and repair and indicate that OP-1 can be used as a therapeutic agent that promotes cartilage anabolism and repair of human articular cartilage.

OP-1 (BMP-7) induces cartilage and bone formation when implanted at intra- and extraskeletal sites *in vivo.* The influence of OP-1 on healing of full-thickness articular cartilage defects was investigated by drilling two adjacent holes through articular cartilage of rabbit knee joint. OP-1 induced articular cartilage healing and regeneration of the joint surface that contained cells resembling mature joint chondrocytes.

These data suggest that one preferred form of the solution useful for the prevention of cartilage degradation and maintaining biological homeostasis of articular cartilage in humans after surgical trauma could include local application of a member of the TGF-β superfamily, preferably either TGFβ2, BMP-7 (OP-1) or BMP-2, or an equivalent agonist which acts through the same receptors employed by these ligands. The local delivery may occur in combination with a drug or drugs that are inhibitors of cartilage catabolic processes (eg. such as MAP kinase inhibitors, MMP inhibitors or nitric oxide synthase inhibitors) and/or other agents for the inhibition of pain and inflammation.

Within the context of defining TGF-β and BMP agonists as pharmacological agonists, the term TGF-β and BMP agonist includes, but is not limited to: (1) peptide sequences which correspond to naturally (endogenous) produced amino acid sequences or fragments thereof, (2) recombinant TGF-βs and BMPs which are truncated or partial sequences of the full length naturally occurring TGF-β and BMP amino acid sequences which retain the ability to bind cognate their respective receptor and retain biological activity and analogs thereof, and (3) chimeric TGF-βs and BMPs which are recombinant polypeptides comprised of truncated or partial sequences corresponding to a portion of the of the full length amino acid sequences attached through oligomers (e.g., amino acids) to a sequence corresponding to a portion of an IgG polypeptide (e.g., IgG hinge and Fc domain) which retain the ability to bind the cognate receptor and retain biological activity.

Examples of TGF-β and BMP agonists suitable for use herein are listed below. For all modes of local delivery (i.e., injection, infusion and irrigation) the optimal dose and/or concentration of each suitable agent is that which is therapeutically effective. As an example, for each of the listed agents, the preferred and most preferred concentrations of an irrigation solution containing the listed agent are provided, such concentrations expected to be therapeutically effective.

A range of therapeutic concentrations for delivery in the surgical solution to the joint may be estimated from values of the dissociation constants (Kd) of each ligand for its cognate receptor. While these values will vary for particular cell types and tissues, the following example is given for BMP-4. Binding experiments with ¹²⁵I-BMP-4, revealed the presence of specific, high-affinity binding sites with an apparent dissociation constant of 110 pM and about 6000 receptors per cell. Therefore, at 11 nM BMP-4, binding of the ligand will be maximal and the available receptors will be fully occupied (saturated). The presence of functional receptors for BMP-4 on primary articular chondrocytes has been demonstrated.

**Table 21**

| Therapeutic and Preferred Concentrations TGF-β and BMP-Receptor Agonists | | |
|---|---|---|
| Compound | Therapeutic Concentrations (nanomolar) | Most Preferred Concentrations (nanomolar) |
| TGF-β1 | 0.05-500 | 0.5-100 |
| TGF-β2 | 0.05-500 | 0.5-100 |
| BMP-2 | 0.1-2000 | 1-200 |
| BMP-4 | 0.1-2000 | 1-200 |
| BMP-7 (OP-1) | 0.1-2000 | 1-200 |

### 5. Cyclooxygenase-2 (Cox-2) Inhibitors

Nonsteroidal anti-inflammatory drugs (NSAIDs) are widely used as anti-inflammatory agents, but have not been specifically developed or therapeutically employed as chondroprotective agents. The direct molecular target for an NSAID drug is the first enzyme in the prostaglandin synthetic pathway, referred to either as prostaglandin endoperoxide synthase or fatty acid cyclooxygenase. Two related forms of cyclooxygenase, termed cyclooxygenase-1 or type 1 (COX-1) and cyclooxygenase-2 (COX-2) have been characterized. These isozymes are also known as Prostaglandin G/ H Synthase (PGHS)-1 and PGHS-2. Both enzymes catalyze the rate-limiting step in the formation of prostanoids that is the conversion of arachidonic acid to prostaglandin H2. COX-1 is present in platelets and endothelial cells and exhibits constitutive activity. In contrast, COX-2 has been identified in endothelial cells, macrophages, fibroblasts and other cells in the joint and its expression is induced by pro-inflammatory cytokines, such as IL-1 and TNF-α.

Within the inflamed joint, COX-2 expression is upregulated and it has been shown that large increases in activity of COX-2 occur concomitant with its upregulation, leading to increased synthesis of prostaglandins which are present in the synovial fluid of patients suffering from inflammatory arthropathies. Cellular sources of prostaglandins (PGs) in the joint include activated chondrocytes, type A and B synoviocytes and infiltrating macrophages. Cellular functions important in cartilage metabolism modulated by PGs include gene expression, extracellular matrix synthesis and proliferation. Because COX-2 is expressed in inflamed joint tissue or after exposure to mediators of inflammation (e.g., as a result of injury or surgical trauma), the use of a COX-2 inhibitor is expected to provide both anti-inflammatory and cartilage protective activity.

Cartilage destruction in inflammatory arthropathies can be triggered as a consequence of joint injury and as a result of arthroscopic surgical procedures. Chondrocytes are the only cell type in articular cartilage and are known to participate in the breakdown of their own matrix through release of endogenous inflammatory mediators, including PGs. Studies have shown that COX-2 gene expression, protein synthesis, and PG release in normal human articular chondrocytes is rapidly induced by cytokines, including IL-1, TNF-α and IL-6. Levels of mRNA are detected as early as 2 hours after cytokine induction, reach high levels at 6 hours and show a remarkably long duration of expression for at least 72 hours. Similarly, cell culture studies of IL-1α and TNF-α activation of human synoviocytes have shown large increases in expression of COX-2 and production of prostaglandin E2 (PGE2). Treatment with a variety of NSAIDS, such as ketoprofen, abolishes the induced PGE2 response. In a chondrocyte cell culture system, the specific COX-2 inhibitor compound NS-398 prevented the increase in PGE2 production induced by the cytokines while COX-1 levels remained stable (Morisset, S., 1998, J. Rheumatol. 25:1146-53). Thus, it can be deduced that blocking PG production by activated chondrocytes which is associated with expression of COX-2 can provide a chondroprotective effect.

NSAIDs are commonly used in the treatment of patients with osteoarthritis or rheumatoid arthritis, but their effects on articular cartilage metabolism in the context of these arthritic diseases remains a subject of debate. For instance, the clinical treatment of osteoarthritis and rheumatoid arthritis with NSAIDs is successful in reducing inflammation. However, it is thought that some NSAIDs which are not selective for COX-2, primarily salicylates and indomethacin, accelerate osteoarthritic cartilage destruction by impairing proteoglycan synthesis by chondrocytes, whereas other NSAIDS are thought to have a somewhat chondroprotective effect by stimulating cartilage repair. Most studies have shown that NSAIDs have little or no effect on cartilage. Due to the current lack of use of this class of drugs in the treatment of synovitis and cartilage destruction following traumatic joint injury and surgical trauma, the unique properties of each NSAID on the pathophysiological mechanisms that contribute to cartilage destruction will need to be established.

Since the two COX isozymes are pharmacologically distinct, isozyme-specific (selective) cyclooxygenase inhibitors that are useful for anti-inflammatory therapy have been developed and some of these same COX-2 inhibitors have been tested in models of joint inflammation. However, the effects *in vitro* of the COX-2 inhibitors on the synthesis and degradation of cartilage proteoglycans, as well as synovial production of IL-1, IL, 6, IL-8, and prostanoids, indicate that certain NSAIDs may vary considerably in their effects *in vivo* on cartilage and synovial production of interleukins and eicosonoids, such that the integrated effects of these parameters may influence the outcome of COX-2 inhibitors on cartilage integrity. For example, some NSAIDS can accelerate joint damage in osteoarthritis by enhancing the production of pro-inflammatory cytokines or inhibiting cartilage proteoglycan synthesis. However, despite the possible variance in clinical effect among COX- 2 specific inhibitors, inhibition of COX-2 typically results in a reduction of synovitis and an expected decrease in the risk of cartilage damage.

A variety of biochemical and cellular and animal assays have been developed to assess the relative selectivity of inhibitors for the COX-1 and COX-2 isoforms. In general, a criteria for defining selectivity is the ratio of the COX-1/COX-2 inhibitory constants (or COX-2/COX-1) obtained for a given biochemical or cellular assay system. The selectivity ratio accounts for different absolute IC₅₀ values for inhibition of enzymatic activity that are obtained between microsomal and cellular assay systems (e.g., platelet and macrophage cell lines stably expressing recombinant human COX isozymes). Furthermore, inhibition of COX-2 mimics the inhibitory effects triggered by chondroprotective (inhibitory) cytokines, such as IL-4, which down-regulate intracellular COX-2 synthesis. Comparison of the selectivity of more than 45 NSAIDs and selective COX-2 inhibitors (1997, Can. J. Physiol. Pharmacol. 75:1088-95) showed the following rank-ordered relative selectivity for COX-2 vs. COX-1: DuP 697 > SC-58451= celecoxib > nimesulide = meloxicam = piroxicam = NS-398= RS-57067 >SC-57666 > SC-58125 > flosulide > etodolac > L-745,337 > DFU-T-614, with IC₅₀ values ranging from 7 nM to 17 µM.

From the molecular and cellular mechanism of action defined for selective COX-2 inhibitors, such as celecoxib and rotecoxib, as well as from animal studies, these compounds are expected to exhibit chondroprotective action when applied perioperatively in an irrigation solution or in an injection directly to a joint. In particular, COX-2 inhibitors are expected to be effective drugs delivered in an irrigation solution during an arthroscopic surgical procedure or by direct injection into a joint prior to, during or after a surgical procedure or other injury to the joint.

Examples of COX-2 inhibitors suitable for use herein are listed below. For all modes of local delivery (i.e., injection, infusion and irrigation) the optimal dose and/or concentration of each suitable agent is that which is therapeutically effective. As an example, for each of the listed agents, the preferred and most preferred concentrations of an irrigation solution containing the listed agent are provided, such concentrations expected to be therapeutically effective.

**Table 22**

| Therapeutic and Preferred Concentrations of Cyclooxygenase-2 Inhibitors | | |
|---|---|---|
| Compounds | Therapeutic Preferred Concentrations (nM) | Most Preferred Concentrations (nM) |
| rofecoxib (MK 966) | 0.3-30,000 | 30-3,000 |
| SC-58451 | 0.3-30,000 | 30-3,000 |
| celecoxib (SC-58125) | 0.3-30,000 | 30-3,000 |
| meloxicam | 0.5-50,000 | 50-5,000 |
| nimesulide | 0.5-50,000 | 50-5,000 |
| diclofenac | 0.3-30,000 | 30-3,000 |
| NS-398 | 0.3-30,000 | 30-3,000 |
| L-745,337 | 0.2-100,000 | 20-10,000 |
| RS57067 | 0.2-100,000 | 20-10,000 |
| SC-57666 | 0.2-100,000 | 20-10,000 |
| flosulide | 0.2-100,000 | 20-10,000 |

### 6. MAP Kinase Inhibitors

The mitogen-activated protein (MAP) kinases are a group of protein serine/threonine kinases that are activated in response to a variety of extracellular stimuli and function in transducing signals from the cell surface to the nucleus. The MAP kinase cascade is one of the major intracellular signalling pathways that transmit signals from growth factors, hormones and inflammatory cytokines to intermediate early genes. In combination with other signalling pathways, these activated mitogen-activated protein-kinases (MAPKs) differentially alter the phosphorylation state and activity of transcription factors, and ultimately regulate cell proliferation, differentiation and cellular response to environmental stress. For example, a member of the MAPK family (p38) mediates the major biochemical signal transduction pathways from the potent pro-inflammatory cytokines, IL-1 and TNF-α, leading to induction of cyclooxygenase-2 (COX-2) in stimulated macrophages, through cis-acting factors involved in the transcriptional regulation of the COX-2 gene.

The members of the MAP kinase class of agents are composed of at least three families that are known to differ in sequence, size of the activation loop, activation by extracellular stimuli and participation in distinct signal transduction pathways. Prominent members among this family of MAP kinases include the extracellular signal-regulated kinases (ERKs), ERK1 and ERK2 (p44MAPK and p42MAPK, respectively); stress-activated protein kinase 1 (SAPK1) family which is also referred to as the JNK or jun N-terminal kinase family; and the p38 MAP kinase family which is also known as stress-activated kinase 2/3 (SAPK-2/3). The p38 kinases are activated by stresses, most notably pro-inflammatory cytokines. Within the p38 family, there are at least four distinct homologs (isotypes or isoenzymes) which standard nomenclature refers to either as SAPK2a, SAPK2b, SAPK2d, SAPK3, or p38 α, β, δ (SAPK4) and γ, respectively. The inhibitors of MAP kinases may interact with any one or combination of the above MAP kinases. For specific MAP kinase inhibitors, the inhibitory constants characterized through assays of purified *in vitro* enzymes and in cellular assays may vary over a wide range of concentrations and demonstrate utility in this application. Activation of p38 MAP kinase is mediated by dual phosphorylation of threonine and tyrosine residues. Both TNF-α and IL-1 treatment of cells has been shown to rapidly (within 5 min) increase phosphorylation and activate p38 MAP kinase.

Previous work has shown that small-molecule inhibitors can specifically inhibit p38 MAP kinase (Lee, J. et al., Nature 372: 739-746 (1994)) and produce anti-inflammatory effects at the biochemical level and in various animal models. Cuenda and coworkers (Cuenda, A. et al., FEBS Lett. 364: 229 (1995)) showed that the compound, SB203580 [4-(4-fluorophenyl)-2-(4-methylsulfinylphenyl)-5-(4-pyridyl)imidazole] inhibited p38 *in vitro* (IC₅₀ = 0.6 µM), suppressed the activation of MAPK activating protein kinase-2 and prevented the phosphorylation of heat shock protein (hsp) 27 in response to IL-1 and cellular stresses *in vivo.* The kinase selectivity of SB203580 inhibitory action for p38 was demonstrated by its failure or at best weak inhibition of at least 15 other protein kinases *in vitro,* including members of the PKC, PKA, src and receptor tyrosine kinase families (Lee, J, Pharmacol. Ther. 82: 389-397 (1999)). In cellular studies, pre-incubation with SB 203580 has been shown to block the IL-1 and TNF-α induced phosphorylation of the enyzme and subsequent IL-8 production. This supports the preemptive effect of delivering the inhibitors during the surgical procedure.

The role of p38 mitogen-activated protein kinase (MAPK) in biochemical inflammatory responses resulting in destruction of cartilage has been studied using SB203580, which specifically inhibits the enzyme. Actions of IL-1 that are selectively controlled by p38 MAPK are the regulation of prostaglandin H synthase-2 (COX-2), metalloproteinases, and IL-6 (Ridley, S et al., 1997, J. Immunol. 158:3165-73). In human fibroblasts and vascular endothelial cells, SB203580 inhibited (IC₅₀= 0.5 µM) IL-1-induced phosphorylation of hsp 27 (an indicator of p38 MAPK activity) in fibroblasts without affecting the other known IL-1-activated protein kinase pathways (p42/p44 MAPK, p54 MAPK/c-Jun N-terminal kinase). In addition, SB203580 significantly inhibited IL-1-stimulated IL-6 (30 to 50% at 1 µM) but not IL-8 production from human fibroblasts and endothelial cells.

Importantly, SB203580 strongly inhibited IL-1-stimulated prostaglandin production by fibroblasts and human endothelial cells. This was associated with the inhibition of the induction of COX-2 protein and mRNA. PGE₂ contributes to increased expression of matrix metalloproteinases that are important mediators of cartilage degradation. Both synovial fibroblasts and chondrocytes express the COX-2 gene at high levels upon activation by cytokines and extracelluar stimuli. The MAPK inhibitor provides chondroprotective activity due to its inhibitory activity on MAP kinases expressed in these and other cell types.

MAPK inhibitors are expected to be effective as cartilage protective agents when applied locally to tissues of the joint in a variety of inflammatory or pathophysiological conditions. SB 203580 has been characterized in several pharmacological models *in vivo* and demonstrated to have activity under long term, oral dosing. SB203580 was found to inhibit the stimulation of collagenase-1 and stromelysin-1 production by IL-1 without affecting synthesis of TIMP-1. Furthermore, SB203580 prevented an increase in IL-1-stimulated collagenase-1 and stromelysin-1 mRNA. In a model of cartilage breakdown, short-term IL-1-stimulated proteoglycan resorption and inhibition of proteoglycan synthesis were unaffected by SB 203580, while longer term collagen breakdown was prevented. In addition, SB203580 was found to be effective in inhibiting IL-1-induced nitric oxide production in bovine articular cartilage explants and chondrocytes (Badger 1998). These *in vitro* observations provide a basis for cartilage protective activity of the MAP kinase inhibitor administered directly and locally to these tissues in the joint.

p38 MAP kinase is involved in TNF-induced cytokine expression, and drugs which function as inhibitors of p38 MAP kinase activity block the production of pro-inflammatory cytokines (Beyaert, R. et al., EMBO J. 15:1914-23 (1996)). TNF-α treatment of cells activated the p38 MAPK pathway as shown by increased phosphorylation of p38 MAPK itself and activation of its substrate proteins. Pretreatment of cells with SB203580 completely blocked TNF-α induced activation of MAPK activating protein kinase-2 and hsp27 phosphorylation. Under the same conditions, SB203580 also completely inhibited TNF-α induced synthesis of IL-6 and expression of a reporter gene that was driven by a minimal promoter containing two NF-6B elements. Thus, these studies and related studies on other p38 inhibitors show that the action of inhibitors, such as SB203580 and FR133605, on p38 MAPK interfere selectively with TNF-α- and IL-1-induced activation of various proteins linked to the cartilage degradation. Thus, the selective inhibition of the MAP kinase signalling pathways of these key pro-inflammatory cytokines by inhibition of a kinase downstream of the receptor indicate that MAP kinase inhibitors may provide a chondroprotective effect.

SB 203580 has been evaluated in several animal models of cytokine inhibition and inflammatory disease. It was demonstrated to be a potent inhibitor of inflammatory cytokine production *in vivo* in both mice and rats with IC₅₀ values of 15 to 25 mg/kg. SB 203580 possessed therapeutic activity in collagen-induced arthritis in DBA/LACJ mice with a dose of 50 mg/kg resulting in significant inhibition of paw inflammation. Antiarthritic activity was also observed in adjuvant-induced arthritis in the Lewis rat when SB203580 was administered p.o. at 30 and 60 mg/kgJday. Additional evidence was obtained for beneficial effects on bone resorption with an IC₅₀ of 0.6 µM.

In summary, a variety of biochemical, cellular and animal studies show that p38 MAPK plays an important role in the regulation of responses to IL-1 and TNF-α and that it is involved in the regulation of mRNA levels of some inflammatory-responsive genes, such as COX-2. Inhibitors of p38 block the production of pro-inflammatory cytokines and inhibit the production of MMPs, and have been demonstrated to inhibit collagen breakdown in cartilage explants.

The use of MAPK inhibitor to block the actions of key pro-inflammatory cytokines, such as IL-1 and TNF-α, will have beneficial effects on many cell types in the joint, including synovial fibroblasts, macrophages and chondrocytes, thus inhibiting subsequent pathological effects such as infiltration of inflammatory cells into the joint, synovial hyperplasia, synovial cell activation, and cartilage breakdown. Thus, a MAPK inhibitor should block the propagation of the inflammatory response by the aforementioned cytokines, and thereby interrupt the disease process.

Examples of MAPK inhibitors are listed below. For all modes of local delivery (i.e., injection, infusion and irrigation) the optimal dose and/or concentration of each suitable agent is that which is therapeutically effective. As an example, for each of the listed agents, the preferred and most preferred concentrations of an irrigation solution containing the listed agent are provided, such concentrations expected to be therapeutically effective.

**Table 23**

| **Therapeutic and Preferred Concentrations of MAP Kinase Inhibitors** | | |
|---|---|---|
| **Compounds** | **Therapeutic Concentrations (nanomolar)** | **Preferred Concentrations (nanomolar)** |
| SB 203580 | 0.5-50,000 | 50-5,000 |
| SB 203580 iodo | 0.5-50,000 | 50-5,000 |
| SB 202190 | 0.2-20,000 | 20-2,000 |
| SB 242235 | 0.2-10,000 | 20-1,000 |
| SB 220025 | 0.2-10,000 | 20-1,000 |
| RWJ 67657 | 0.3-30,000 | 30-3,000 |
| RWJ 68354 | 0.9-90,000 | 90-9,000 |
| FR 133605 | 1-100,000 | 10-10,000 |
| L-167307 | 0.5-50,000 | 50-5,000 |
| PD 98059 | 0.1-10,000 | 10-1000 |
| PD 169316 | 1-100,000 | 10-10,000 |

### 7. Inhibitors Of Matrix Metalloproteinases

Destruction of articular cartilage is a common feature in joint diseases such as osteoarthritis and rheumatoid arthritis, but also occurs following injury to the joint. Pathophysiologically, a structural breakdown of proteoglycans and collagen is observed, which impairs the biomechanical properties of cartilage. The maintenance of a normal, healthy extracellular matrix reflects a balance between the rate of biosynthesis and incorporation of matrix components, and the rate of their degradation and subsequent loss from the cartilage into the synovial fluid. A variety of proteases have the potential to cleave cartilage and are involved in the degradation process, most notably the matrix metalloproteinases.

Matrix metalloproteinases (MMPs), or matrixins, are a family of at least 15 zinc endopeptidases that function extracellularly and play a key role in pathological degradation of tissue. Current nomenclature and alternative names for members of the MMP are provided in Table 23. Most MMPs are highly regulated and most are not constitutively expressed in normal tissues. However, pro-inflammatory cytokines, such as IL-1 and TNF-α, initiate transcription and expression. An imbalance created by upregulation and activation of tissue-degrading MMPs is a primary causative factor in the cartilage breakdown process during chronic inflammatory diseases and sustained synovial inflammatory responses subsequent to joint injury. Cartilage matrix metabolism has been studied in patients with either a meniscal injury or anterior cruciate ligament rupture in the knee. It was shown that concentrations of stromelysin-1 (MMP-3), collagenase, tissue inhibitor of metalloproteinases (TIMP-1), and proteoglycan fragments increased in human knee synovial fluid after traumatic knee injury. Temporally, these values increased immediately over reference levels and remained significantly elevated (10-fold increase) over a period of one year. These changes likely drive the increase in the concentration of proteoglycan fragments that are observed in synovial fluid after knee ligament injury.

**Table 24**

| Matrix Metalloproteinases | | | |
|---|---|---|---|
| MMP | Alternative Names | EC Number | Substrates |
| MMP-1 | Collagenase Fibroblast Collagenase Interstitial Collagenase | EC3.4.24.7 | Collagens (I, II, II, VII, and X);Gelatin; aggrecan; hyaluronidase-treated versican; proteoglycan link protein; large tenascin-C; α₁ - antitrypsinla/α₁ -proteinase inhibitor (α₁) - AT); α₁) antichymotrypsin (α₁ - ACHYM); α₂, M; rat α₁ M; pregnancy zone protein; rat α₁I₃ (α₁ - inhibitor 3); ovostatin; entactin; MBP; GST-TNFFINF peptide; L-selection; IL-1β; serum amyloid A; IGF-BP5; IGF-BP3; MMP-2; MMP-13 |
| MMP-2 | 72-kDa Gelatinase Gelatinase A Type IV Collagenase Neutrophil Gelatinase | EC3.4.24.24 | Collagens (1, IV, V, VI, X, XI, and XIV); Gelatin; elastin; fibronectin; laminin-1, laminin-5; gelactin-3; aggrecan; decorin; hyaluronidase-treated versican; proteoglycan link protein; osteonectin; MBP; GST-TNF/TNF peptide; IL-1β; Aβ₁₋₄₀; Aβ₁₀₋₂₀, APP₆₉₅ ; α₁ - AT; prolysyl oxidase fusion protein; IGF-BP5; IGF-BP3; FGF R1; MMP-1; MMP-9; MMP-13 |
| MMP-3 | Stromelysin-1 Transin | EC3.4.24.17 | Collagens (III, IV, V, IX); Gelatin; aggrecan; versican and hyaluronidase-treated veriscan; perlecan; decorin; proteoglycan link protein; large tenascin-C; fibronectin; laminin; entactin; osteonection; elastin; casein; α₁-ACHYM; antithrombin-III; α₂ M; ovostain; Substance P; MBP; GST-TNF/TNF peptide; IL-1β; serum amyloid A; IGF-BP3; fibrinogen and cross-linked fibrin; plasminogen; MMP-1"superactivation", MMP-2/TI1VIP-2 complex; MMP-7; MMP-8; MMP-9; MMP-13 |
| MMP-7 | Matrilysin PUMP | EC3.4.24.23 | Collagen IV and X; Gelatin; aggrecan; decorin; proteoglycan link protein; fribronectin and laminin; insoluble fibronectin fibrils; enactin; large and small tenascin-C; osteonectin; β4 integrin; elastin; casein; transferrin; MBP; α₁ -AT; GST-TNF/TNF peptide; plasminogen; MMP-1; MMP-2; MMP-9; MMP-9/TIMP-1 |
| MMP-8 | Neutrophil Collagenase Collagenase I | EC3.4.24.34 | Collagens (I, II, III, V, VII and X); Gelatin; aggrecan; α₁ -AT; α₁-ACHYM; α₂-antiplasmin; fibronectin |
| MMP-9 | 92 kDa Gelatinase Gelatinase B | EC3.4.24.35 | Collagens (IV, V, VII, X and XIV); Gelatin; elastin; galectin-3; aggrecan; hyalurondise-treated versican; proteoglycan link protein; fibronectin; entactin; osteonectin; α₁ -AT; MBP; GST-TNF/TNF peptide; IL-1β; Aβ₁₋₄₀: plasminogen |
| MMP-10 | Stromelysin-2 | EC3.4.24.22 | Collagens (III, IV and V); Gelatin; casein; aggrecan; elastin; proteoglycan link protein; MMP-1; MMP-8 |
| MMP-11 | Stromelysin-3 | | Human enzyme: α₁-AT; α₂ M; casein, laminin, fibronectin, gelatin, collagen IV and carboxymethylated transferrin |
| MMP-12 | Macrophage Metalloelastase | | Collagen IV; Gelatin; elastin and κ -elastin; casein; α₁ -AT; fibronectin; vitronectin; laminin; enactin; proteoglycan monomer; GST-TNF; MBP; fibrinogen; fibrin; plasminogen |
| MMP-13 | Collagenase-3 | | Collagens (I, II and III, IV, IX, X and XIV); Gelatin, α1-ACHYM and plasminogen activator inhibitor 2; aggrecan; perlecan; large tenascin-C, fribronectin; osteonectin;MMP-9 |
| MMP-14 | MT-MMP-1 | | Collagen (I, II and III); Gelatin, casean, κ-elastin, fribronectin, laminin, vitronectin and |
| | | | proteoglycans; large tenascin-C, enactin; α₁ -AT, α₂ M; GST-TNF; MMP-2; MMP-13 |
| MMP-15 | MT-MMP-2 | | Fibronectin, large tenascin-C, entactin, laminin, aggrecan, perlecan; GST-TNF; MMP-2 |

The MMP family of enzymes has been shown to be secreted from human chondrocytes and by cells of the synovium, such as synovial fibroblasts. Furthermore, using *in situ* hybridization, it was shown that human synovium synthesizes both stromelysin-1 and collagenase. Stromelysin-1 (MMP-3) is capable of degrading all of the components of the cartilage matrix. There is evidence that chondrocytes contribute to cartilage degradation by the release of the matrix-degrading enzyme, collagenase-3. Upon activation by pro-inflammatory cytokines, MMPs are secreted from cells in a latent form, are activated extracellularly, and are inhibited by tissue inhibitors of metalloproteinases (TIMPs). The balance between the activities of MMPs and TIMPs is thought to be important for the maintenance of an intact cartilage matrix. Under pathological conditions such as osteoarthritis and rheumatoid arthritis, several studies have shown elevated amounts of MMPs, resulting in an imbalance between MMPs and TIMPs that is considered to account for the observed cartilage destruction.

The MMPs are regulated by cytokines, such as interleukin-l (IL-1), and growth factors that act on chondrocytes and synoviocytes to enhance their protease production. Other pro-inflammatory cytokines, such as IL-6, IL-8 and TNF-α, also upregulate the production of matrix-degrading enzymes. This leads to cartilage destruction, which is usually assessed as the loss of sulfated glycosaminoglycans (GAGs) and the cleavage of collagen. IL-1, which is present in the joint fluid of patients with arthritic diseases, stimulates chondrocytes to synthesize elevated amounts of enzymes such as stromelysin, fibroblast and neutrophil collagenase, and plasminogen activator. In addition, IL-1 inhibits synthesis of plasminogen activator inhibitor-1 and TIMP, and also inhibits synthesis of matrix constituents such as collagen. The imbalance between the levels of inhibitors and enzymes leads to an increase in the amount of active proteases and, combined with a suppression of matrix biosynthesis, results in cartilage degradation.

Using cartilage slices as an *in vitro* model, it has been shown that collagenase inhibitors can inhibit either the IL-1 or IL-8 stimulated invasion of articular cartilage by rheumatoid synovial fibroblasts (RSF). The collagenase inhibitors, 1,10-o-phenanthroline and phosphoramidon, substantially inhibited the concentration-dependent penetration of cartilage by RSF cells at concentrations of 10-150 µM. The selective effect of cytokines on the secretion of proteinases demonstrates that synovial fibroblast-like cell-mediated articular degradation is a highly regulated process. Thus, the ability to inhibit protease activity and associated matrix degradation locally within the joint is expected to inhibit the cartilage destruction process. The action of the inhibitors in the limited *in vitro* system suggests that therapeutic intervention using local delivery of synthetic MMP inhibitors with appropriate pharmokinetics will be effective as chondroprotective agents.

Examples of MMP inhibitors suitable for use herein include U-24522 ((R,S)-N-[2-[2-(hydoxylamino)-2-oxoethyl]-4-methyl-1-oxopentyl]-L-leucyl-L-phenylaianiamide), peptides such as MMP Inhibitor I and MMP-3 Inhibitor, and larger proteins such as TIMP-1 or fragments thereof, and are listed in the Table below: For all modes of local delivery (i.e., injection, infusion and irrigation) the optimal dose and/or concentration of each suitable agent is that which is therapeutically effective. As an example, for each of the listed agents, the preferred and most preferred concentrations of an irrigation solution containing the listed agent are provided, such concentrations expected to be therapeutically effective.

**Table 25**

| Therapeutic and Preferred Concentrations of Matrix Metalloproteinases (MMPs) Inhibitors | | |
|---|---|---|
| Compounds | Therapeutic Concentrations (nanomolar) | Most Preferred Concentrations (nanomolar) |
| U-24522 | 0.2-2,000 | 20-200 |
| minocycline | 30-500,000 | 300-3,000 |
| MMP Inhibitor I | 0.3-3,000 | 3-600 |
| 4-Abz-Gly-Pro-D-Leu-D-Ala-NHOH | | |
| MMP-3 Inhibitor | 0.5-5,000 | 5-500 |
| Ac-Arg-Cys-Gly-Val-Pro-Asp-NH₂ | | |
| rhuman TIMP1 | 0.5-5,000 | 5-500 |
| rhuman TIMP2 | 0.3-3,000 | 3-600 |
| phosphoramidon | 1,000-500,000 | 5,000-100,000 |

### 8. Inhibitors Of Nuclear Factor Kappa B (NFκB)

Pro-inflammatory and cartilage-destructive cellular pathways are regulated by extracellular and intracellular signalling mechanisms that are targets for novel therapeutic local drug delivery. The complete molecular signaling mechanisms utilized by the pro-inflammatory cytokine interleukin-1 (IL-1) to activate the transcription factor, nuclear factor kappaB (NFκB), are poorly defined. Nevertheless, a key molecule that is involved in intracellular signalling at the level of gene transcription is the pro-inflammatory transcription factor, (NFκB). NFκB activity is mediated by a family of transcription factor subunits that bind to DNA either in the form of homodimers or heterodimers. These subunits are typically present within the cytoplasm of cells in an inactive form due to the binding of the inhibitory subunit called IκB. Activation of EL-1 receptors, and other extracellular signals, induce degradation of IκB and concomitant dissociation of NFκB from the inhibitors, followed by translocation to the nucleus. NFκB, was found to be involved in IL-1 induced expression and was capable of increasing pro-inflammatory COX-2 protein expression in RA synovial fibroblasts.

The identification of NFκB as a key molecular target is based upon its role as a common downstream signaling element regulating gene expression of several critical inflammatory mediators linked to joint inflammation and cartilage-destructive pathways. The response of many genes (COX-2, collagenase, IL-6, IL-8) are governed by promoters which contain both NFκB promoter elements. Activation of NFκB mediates the induction of many proteins central to the inflammatory process, such as cytokines, cell-adhesion molecules, metalloproteinases and other proteins that participate in the production of prostaglandins and leukotrienes (COX-2) in synoviocytes. Thus, this transcription factor represents a physiologically significant target in therapies directed to the injury responses of human synovial fibroblasts, human articular chondrocytes, as well as other cells in the joint.

Specifically, it has been shown that exposure of human rheumatoid synovial fibroblasts (RSF) to interleukin 1beta (IL-1beta) results in the coordinate up-regulation of 85-kD phospholipase A2 (PLA2) and inducible cyclooxygenase (COX-2). Together, these two enzymes promote the subsequent biosynthesis of PGE₂, a primary inflammatory mediator in the joint. Oligonucleotide decoys and antisense were used to demonstrate the participation of the (NFκB), in the regulation of the prostanoid-metabolizing enzymes. Antagonizing NFκB mRNA using anti-sense oligonucleotide resulted in decreased binding to the COX gene promoter.

Hymenialdisine, a marine natural product, has recently been characterized as an inhibitor of NFκB activation and exposure of IL-1-stimulated RSF-inhibited PGE2 production in a concentration-dependent manner (IC₅₀= 1 µM). The specificity of the molecular target was shown through use of an analog, aldisine, and the protein kinase C inhibitor, RO 32-0432, which were inactive. Direct action of hymenialdisine on IL-1-induced NFκB activation was demonstrated by a significant reduction (approximately 80%) in NFκB binding to the classical κB consensus motif and inhibition of stimulated p65 migration from the cytosol of treated cells. As expected for an inhibitor of NFκB transcriptional regulation, hymenialdisine-treated RSF did not transcribe the mRNAs for either COX-2 or PLA2 in response to IL-1. Consequently, reduced protein levels for these enzymes and reductions in the ability to produce PGE2 were observed. Furthermore, IL-1-stimulated interleukin-8 (IL,-8) production, which is known to be an NFκB-regulated event, was also inhibited by hymenialdisine, whereas IL-1-induced production of vascular endothelial growth factor, a non-NFκB-regulated gene, was not affected by exposure to hymenialdisine. Thus, hymenialdisine inhibits IL-1-stimulated synovial fibroblast formation of PGE2 through its inhibitory effect on NFκB activation. This provides a basis to define its use as a novel inhibitor to block the role of NFκB in joint inflammation and cartilage destruction.

Examples of NFκB inhibitors are listed below. For all modes of local delivery (i.e., injection, infusion and irrigation), the optimal dose and/or concentration of each suitable agent is that which is therapeutically effective. As an example, for each of the listed agents, the preferred and most preferred concentrations of an irrigation solution containing the listed agent are provided, such concentrations expected to be therapeutically effective.

**Table 26**

| Therapeutic and Preferred Concentrations of Inhibitors of NFkB | | |
|---|---|---|
| Compounds | Therapeutic Concentrations (nanomolar) | Most Preferred Concentrations (nanomolar) |
| Caffeic acid phenylethyl | 1-100,000 | 50-20,000 |
| ester (CAPE) | | |
| DM-CAPE | 0.5-50,000 | 50-5,000 |
| SN-50 peptide | 0.1-100,000 | 100-20,000 |
| hymenialdisine | 1-100,000 | 100-10,000 |
| pyrolidone dithiocarbamate | 1-50,000 | 50-10,000 |

### 9. Nitric Oxide Synthase Inhibitors

Nitric oxide (NO) is a widespread intracellular and intercellular mediator involved in the pathophysiological mechanisms of some connective tissue diseases. NO is formed from L-arginine by a family of enzymes, the NO synthases, which are localized intracellularly. Three isoforms of NO synthase have been cloned and sequenced. Endothelial cell NO synthase (ecNOS) and brain NO synthase (bNOS) are constitutively active. A distinct isoform of NO synthase, inducible NOS (iNOS), is found in many cell types, including chondrocytes. It is absent under basal conditions, but is upregulated in response to pro-inflammatory mediators such as IL-1β and TNF-α. Recent findings show that IL-1 is a very potent stimulator of chondrocyte NO synthesis and that IL-1 acts through its ability to upregulate the level of the iNOS. Within the joint, chondrocytes are the major source of NO and chondrocytic iNOS induced by pro-inflammatory cytokines is considered to mediate many effects of IL-1 in inflammatory arthropathies.

Drugs that specifically inhibit chondrocyte inducible NO synthase (iNOS) may have a therapeutic role in the prevention of chondrodestruction that occurs due to joint injury (e.g., surgical procedures involving the joint). Evidence supporting such a beneficial therapeutic effect is based upon a substantial number of studies which have evaluated a variety of iNOS inhibitors for their ability to inhibit inducible NO synthase activity in cultured chondrocytes and explants of cartilage from patients with osteoarthritis. A class of compounds, termed S-substituted isothioureas, have been characterized as potent inhibitors of NO biosynthesis in cartilage. S-methyl isothiourea and S-(aminoethyl) isothiourea were 2-4 times more potent than N^{G}-monomethyl-L-arginine , 5-10 times more potent than aminoguanidine and over 300 times more potent than N^{ω}-nitro-L-arginine and N^{ω}-nitro-L-arginine methyl ester. These isothiourea compounds provide a potent and relatively specific class of inhibitors of iNOS in cartilage and thus are suitable for local delivery (Jang, D., 1996, Eur. J. Pharmacol. 312: 341-347).

The cartilage protective therapeutic potential of NO synthase inhibitors has also been assessed using in *vitro* systems such as isolated chondrocytes to define effects on the cartilage matrix. Inhibition of endogenous NO production by N^{G}-monomethyl-L-arginine (L-NMMA), an established NO synthase inhibitor, leads to the suppression of gelatinase, collagenase, and stromelysin production by IL-1β-stimulated chondrocytes. Inhibition of NO production also partially reduces the increase in the lactate production that occurs from the exposure of chondrocytes to IL-1β. Treatment of cartilage fragments with L-NMMA partially reverses the IL-1β inhibitory effect of glycosaminoglycan synthesis, inhibits IL-β-stimulated MMP activities, and increases IL-1 receptor antagonist (IL-1ra) production. NO can also modulate proteoglycan synthesis indirectly by decreasing the production of TGF-β1 by chondrocytes exposed to IL-1β. It prevents autocrine-stimulated increases in TGF-β1, thus diminishing the anabolic effects of this cytokine in chondrocytes.

A study has compared the potency of new aminoguanidine, S-methylisothiourea (SMT), S-aminoethylisothiourea (AETU), L-NMMA and N-nitro-L-arginine methyl ester (L-NAME) NOS inhibitors on the inhibitory effect of recombinant human IL-1 responses on proteoglycan synthesis and NO production. Different culture systems have been shown to respond in a concentration dependent manner to IL-1β challenge with a large increase in NO production and a marked suppression of proteoglycan synthesis. The above NOS inhibitors (at 1 to 1000 µM) inhibited NO production by cartilage cells treated with IL-1β and had marked effects on restoring proteoglycan synthesis in chondrocytes. Therefore, if NO production can be blocked using a therapeutically effective concentration and dose, then IL-1β inhibition of proteoglycan synthesis will be prevented.

NO synthase is expressed in cartilage obtained from the joint of patients with arthritic disease. In patients presenting either rheumatoid arthritis or osteoarthritis, increased levels of nitrite have been observed in the synovial fluid and it has been shown that a significant source of NO production in these patients is derived from articular cartilage. Furthermore, it has been found that sustained systemic delivery of L-NIL, a potent inhibitor of iNOS, reduces the progression of experimental OA in dogs (induced by sectioning of the ACL) and causes a substantial decrease in IL-1β, PGE₂, NO and MMP production. These findings suggest that NO is a potent regulator of the effects of IL-1β and contributes to the pathophysiology of joint diseases.

Thus, these *in vitro* and *in vivo* results indicate that specific inhibitors of NO synthases are potential novel drugs for the clinical treatment of synovial inflammation and can provide chondroprotective effects when delivered locally in combination with one or more drugs chosen from the anti-inflammatory, cartilage-protective, and anti-pain classes to treat a surgically treated joint or other injured joint.

Examples of NO synthase inhibitors suitable for use herein are listed below. For all modes of local delivery (i.e., injection, infusion and irrigation) the optimal dose and/or concentration of each suitable agent is that which is therapeutically effective. As an example, for each of the listed agents, the preferred and most preferred concentrations of an irrigation solution containing the listed agent are provided, such concentrations expected to be therapeutically effective. In one from the preferred NO synthase inhibitors for inclusion in the solutions described herein is 1400 W ((N-3-(aminomethyl)benzyl)acetamidine), a selective, slow, tight binding inhibitor of iNOS, diphenyleneiodinium and 1,3-PBYr.

**Table 27**

| Therapeutic and Preferred Concentrations of Nitric Oxide Synthase Inhibitors | | |
|---|---|---|
| Compounds | Therapeutic Concentrations (µM) | Most Preferred Concentrations (µM) |
| N^{G} -monomethyl-L-arginine | 50-50,000 | 3,000 |
| 1400 W | 0.1-1,000 | 1-20 |
| diphenyleneiodium | 0.1-1,000 | 1-100 |
| S-methyl isothiourea | 1-1,000 | 10-100 |
| S-(aminoethyl) isothiourea | 1-1,000 | 10-100 |
| L-N⁶-(1-iminoethyl)lysine | 1-1,000 | 10-100 |
| 1,3-PBITU | 0.5-500 | 5-100 |
| 2-thy1-2-thiopseudourea | 2-20,000 | 20-2,000 |

### 10. Cell Adhesion Molecules

### 10a Integin Agonists and Antagonists

Integrins are heterodimer receptors located on the plasma membrane that contain α and B subunits that bind ligands which are extracellular matrix (ECM) components or may be other large proteins, such as collagen, laminin, vitronectin, osteopontin (OPN) and fibronectin (FN). Degradation of the cartilage matrix is regulated by chondrocytes through mechanisms which depend upon the interaction of these cells with the ECM. Chondrocyte gene expression is, in part, controlled through cellular contacts involving the interaction of integrins with components of ECM in the environment surrounding the chondrocyte. Hence, integrins on chondrocytes are involved in control of cartilage homeostasis, and this family of receptors represents a class of therapeutic targets for preventing cartilage degradation.

Human chondrocytes express an array of integrin receptors composed of distinct α and β subunits, including α, β₁, α₅β₁, αVβ₁ and lesser quantities of others. Of particular importance is the αVβ₃ integrin, which is known to bind OPN. The αVβ₃ complex-specific function blocking monoclonal antibody (mAb) LM609 acts as an agonist in a manner that is similar to the ligand, OPN. It attenuates the production of a number of proinflammatory and cartilage destructive mediators, such as IL-1, NO and PGE2. Thus, the agonistic mAb LM609 is though to be suitable for use.

In addition, two peptidomimetics, MK-383 (Merck) and RO 4483 (Hoffmann-LaRoche), have been studied in Phase II clinicals. Since these are both small molecules, they have a short half-life and high potency. However, these seem to also have less specificity, interacting with other closely related integrins. These peptidomimetics are also be suitable for use.

**Table 28**

| Therapeutic and Preferred Concentrations of Integrins | | |
|---|---|---|
| Class of Agent | Therapeutic Concentrations (µg/ml) | Preferred Concentrations (µg/ml) |
| Integrins: | | |
| αVβ3 mAb LM 609 | 0.05-5,000 | 5-500 |
| echistatin | 0.1-10,000 | 100-1,000 |

### 11. Anti-chemotactic agents

Anti-chemotactic agents prevent the chemotaxis of inflammatory cells. Representative examples of anti-chemotactic targets at which these agents would act include, but are not limited to, F-Met-Leu-Phe receptors, IL-8 receptors, MCP-1 receptors, and MIP-1-I/RANTES receptors. Drugs within this class of agents are early in the development stage, but it is theorized that they may be suitable for use.

### 12. Intracellular Signaling Inhibitors

### 12a. Protein Kinase Inhibitors

### i. Protein Kinase C (PKC) Inhibitors

Protein kinase C (PKC) plays a crucial role in cell-surface signal transduction for a number of physiological processes. PKC isozymes can be activated as downstream targets resulting from initial activation of either G-protein coupled receptors (e.g., serotonin, bradykinin, etc.) or pro-inflammatory cytokine receptors. Both of these receptor classes play important roles in mediating cartilage destruction.

Molecular cloning analysis has revealed that PKC exists as a large family consisting of at least 8 subspecies (isozymes). These isozymes differ substantially in structure and mechanism for linking receptor activation to changes in the proliferative response of specific cells. Expression of specific isozymes is found in a wide variety of cell types, including: synoviocytes, chondrocytes, neutrophils, myeloid cells, and smooth muscle cells. Inhibitors of PKC are therefore likely to effect signaling pathways in several cell types unless the inhibitor shows isozyme specificity. Thus, inhibitors of PKC can be predicted to be effective in blocking the synoviocyte and chondrocyte activation and may also have an anti-inflammatory effect in blocking neutrophil activation and subsequent attachment. Several inhibitors have been described and initial reports indicate an IC₅₀ of 50 µM for calphostin C inhibitory activity. G-6203 (also known as Go 6976) is a new, potent PKC inhibitor with high selectivity for certain PKC isotypes with IC₅₀ values in the 2-10 µM range. Concentrations of these and another drug, GF 109203X, also known as Go 6850 or bisindoylmaleimide I (available from Warner-Lambert), that are believed to be suitable for use are set forth below.

**Table 29**

| Therapeutic and Preferred Concentrations of Cartilage Destruction Inhibitory Agents | | |
|---|---|---|
| Class of Agent | Therapeutic Concentrations (Nanomolar) | Preferred Concentrations (Nanomolar) |
| Protein Kinase C Inhibitors: | | |
| calphostin C | 0.5-50,000 | 100-5,000 |
| GF 109203X | 0.1-10,000 | 1-1,000 |
| G-6203 (Go 6976) | 0.1-10,000 | 1-1,000 |

### ii. Protein Tyrosine Kinase Inhibitors

Although there is a tremendous diversity among the numerous members of the receptors tyrosine-kinase (RTK) family, the signaling mechanisms used by these receptors share many common features. Biochemical and molecular genetic studies have shown that binding of the ligand to the extracellular domain of the RTK rapidly activates the intrinsic tyrosine kinase catalytic activity of the intracellular domain (see FIGURE 5). The increased activity results in tyrosine-specific phosphorylation of a number of intracellular substrates which contain a common sequence motif. Consequently, this causes activation of numerous "downstream" signaling molecules and a cascade of intracellular pathways that regulate phospholipid metabolism, arachidonate metabolism, protein phosphorylation (involving mechanisms other than protein kinases), calcium mobilization and transcriptional activation (see FIGURE 2). Growth-factor-dependent tyrosine kinase activity of the RTK cytoplasmic domain is the primary mechanism for generation of intracellular signals that lead to cellular proliferation. Thus, inhibitors have the potential to block this signaling and thereby prevent synoviocyte and chondrocyte activation.

Any of several related tyrphostin compounds have potential as specific inhibitors of tyrosine kinase activity (IC₅₀s *in vitro* in the 0.5-1.0 µM range), since they have little effect on other protein kinases and other signal transduction systems. To date, only a few of the many tyrphostin compounds are commercially available, and suitable concentrations for these agents are set forth below. In addition, staurosporine has been reported to demonstrate potent inhibitory effects against several protein tyrosine kinases of the src subfamily and a suitable concentration for this agent is set forth below.

**Table 30**

| Therapeutic and Preferred Concentrations of Inhibitory Agents | | |
|---|---|---|
| Class of Agent | Therapeutic Concentrations (Nanomolar) | Preferred Concentrations (Nanomolar) |
| Protein Kinase Inhibitors | | |
| lavendustin A | 10-100,000 | 100-10,000 |
| tyrphostin | 10-100,000 | 100-10,000 |
| AG 1296 | | |
| tyrphostin | 10-100,000 | 100-10,000 |
| AG 1295 | | |
| staurosporine | 1-100,000 | 10-1,000 |
| PD 158780 | 0.1-10,000 | 10-500 |
| PD 174265 | 0.1-10,000 | 10-500 |

### 12b. Modulators of Intracellular Protein Tyrosine Phosphatases.

Non-transmembrane protein tyrosine phosphatases (PTPases) containing src-homology₂ SH2 domains are known and nomenclature refers to them as SH-PTP1 and SH-PTP2. In addition, SH-PTP1 is also known as PTP1C, HCP or SHP. SH-PTP2 is also known as PTP1D or PTP2C. Similarly, SH-PTP1 is expressed at high levels in hematopoietic cells of all lineages and all stages of differentiation, and the SH-PTP1 gene has been identified as responsible for the motheaten (me) mouse phenotype and this provides a basis for predicting the effects of inhibitors that would block its interaction with its cellular substrates. Stimulation of neutrophils with chemotactic peptides is known to result in the activation of tyrosine kinases that mediate neutrophil responses (Cui, et al., 1994 J. Immunol.) and PTPase activity modulates agonist induced activity by reversing the effects of tyrosine kinases activated in the initial phases of cell stimulation. Agents that could stimulate PTPase activity could have potential therapeutic applications as anti-inflammatory mediators.

These same PTPases have also been shown to modulate the activity of certain RTKs. They appear to counter-balance the effect of activated receptor kinases and thus may represent important drug targets. *In vitro* experiments show that injection of PTPase blocks insulin stimulated phosphorylation of tyrosyl residues on endogenous proteins. Thus, activators of PTPase activity could serve to reverse activation of RTK-receptor action in restenosis, and are believed to be useful in the solutions. In addition, receptor-linked PTPases also function as extracellular ligands, similar to those of cell adhesion molecules. The functional consequences of the binding of a ligand to the extracellular domain have not yet been defined but it is reasonable to assume that binding would serve to modulate phosphatase activity within cells (Fashena and Zinn, 1995, Current Biology, 5, 1367-1369) . Such actions could block adhesion mediated by other cell surface adhesion molecules (NCAM) and provide an anti-inflammatory effect. No drugs have been developed yet for these applications.

### 12c. Inhibitors of SH2 Domains (src Homology₂ Domains)

SH2 domains, originally identified in the src subfamily of protein tyrosine kinases (PTKs), are noncatalytic protein sequences and consist of about 100 amino acids conserved among a variety of signal transducing proteins (Cohen, et al., 1995). SH2 domains function as phosphotyrosine-binding modules and thereby mediate critical protein-protein associations in signal transduction pathways within cells (Pawson, Nature, 573-580, 1995). In particular, the role of SH2 domains has been clearly defined as critical for receptor tyrosine kinase (RTK) mediated signaling such as in the case of the platelet-derived growth factor (PDGF) receptor. Phosphotyrosine-containing sites on autophosphorylated RTKs serve as binding sites for SH2-proteins and thereby mediate the activation of biochemical signaling pathways (see FIGURE 2) (Carpenter, G., FASEB J. 6:3283-3289, 1992; Sierke, S. and Koland, J. Biochem. 32:10102-10108, 1993). The SH2 domains are responsible for coupling the activated growth-factor receptors to cellular responses which include alterations in gene expression, and ultimately cellular proliferation. Thus, inhibitors that will selectively block the effects of activation of specific RTKs (excluding IGFR and FGFR) expressed on the surface of synoviocytes are predicted to be effective in blocking cartilage degradation after arthroscopy procedures.

At least 20 cytosolic proteins have been identified that contain SH2 domains and function in intracellular signaling. The distribution of SH2 domains is not restricted to a particular protein family, but found in several classes of proteins, protein kinases, lipid kinases, protein phosphatases, phospholipases, Ras-controlling proteins and some transcription factors. Many of the SH2-containing proteins have known enzymatic activities while others (Grb2 and Crk) function as "linkers" and "adapters" between cell surface receptors and "downstream" effector molecules (Marengere, L., et al., Nature 369:502-505, 1994). Examples of proteins containing SH2 domains with enzymatic activities that are activated in signal transduction include, but are not limited to, the src subfamily of protein tyrosine kinases (src (pp60^{c-src}), abl, lck, fyn, fgr and others), phospholipaseCγ (PLCγ), phosphatidylinositol 3-kinase (PI-3-kinase), p21-ras GTPase activating protein (GAP) and SH2 containing protein tyrosine phosphatases (SH-PTPases) (Songyang, et al., Cell 72, 767-778, 1993). Due to the central role these various SH2-proteins occupy in transmitting signals from activated cell surface receptors into a cascade of additional molecular interactions that ultimately define cellular responses, inhibitors which block specific SH2 protein binding, e.g., c-src) are desirable as agents with potential therapeutic applications in cartilage protection.

In addition, the regulation of many immune/inflammatory responses is mediated through receptors that transmit signals through non-receptor tyrosine kinases containing SH2 domains. T-cell activation via the antigen specific T-cell receptor (TCR) initiates a signal transduction cascade leading to lymphokine secretion and T-cell proliferation. One of the earliest biochemical responses following TCR activation is an increase in tyrosine kinase activity. In particular, neutrophil activation is in part controlled through responses of the cell surface immunoglobulin G receptors. Activation of these receptors mediates activation of unidentified tyrosine kinases which are known to possess SH2 domains. Additional evidence indicates that several src-family kinases (lck, blk, fyn) participate in signal transduction pathways leading from cytokine and integrin receptors and hence may serve to integrate stimuli received from several independent receptor structures. Thus, inhibitors of specific SH2 domains have the potential to block many neutrophil functions and serve as anti-inflammatory mediators.

Efforts to develop drugs targeted to SH2 domains currently are being conducted at the biochemical *in vitro* and cellular level. Should such efforts be successful, it is theorized that the resulting drugs would be useful.

### III. Synergistic Interactions Derived From Therapeutic Combinations Of Anti-pain and/or Anti-inflammation Agents And Other Agents Used In Chondroprotective Solutions

Given the complexity of the disease process associated with inflammation and loss of cartilage homeostasis after arthroscopic therapeutic procedures and the multiplicity of molecular targets involved, blockade or inhibition of a single molecular target is unlikely to provide adequate efficacy in preventing cartilage degradation and the development of osteoarthritis. Indeed, a number of animal studies targeting different individual molecular receptors and or enzymes have not proven effective in animal models or have not yielded efficacy in clinical trials to date. Therefore, a therapeutic combination of drugs acting on distinct molecular targets and delivered locally appears desirable for clinical effectiveness in the therapeutic approach to cartilage protection. As described below, the rationale for this synergistic molecular targeted therapy is derived from recent advances in understanding fundamental biochemical mechanisms by which synoviocyte and chondrocyte cells in the synovium and cartilage transmit and integrate stimuli to which they are exposed during arthroscopic procedures.

### "Crosstalk" and Convergence in Major Signaling Pathways

The molecular switches responsible for cell signaling have been traditionally divided into major discrete signaling pathways, each comprising a distinct set of protein families that act as transducers for a particular set of extracellular stimuli and mediating distinct cell responses. One such pathway transduces signals from neurotransmitters and hormones through G-protein coupled receptors (GPCRs) to produce contractile responses which include the production of inflammatory mediators, such as PGE2. The GPCRs couple to intracellular targets through activation of trimeric G proteins (see FIGURE 2). Examples of signaling molecules involved in activation of synoviocytes and chondrocytes through the GPCR pathway are histamine, bradykinin, serotonin and ATP. A second major pathway transduces signals from pro-inflammatory cytokines, such as IL-1, through a kinase cascade and NF-6B protein into regulation of gene expression and the production of catabolic cytokines and other catabolic factors, including NO.

Signals transmitted from neurotransmitters and hormones stimulate either of two classes of receptors: GPCRs, composed of seven-helix transmembrane regions, or ligand-gated ion channels. "Downstream" signals from both kinds of receptors converge on controlling the concentration of cytoplasmic Ca²⁺ (see FIGURE 3). Each GPCR transmembrane receptor activates a specific class of trimeric G proteins, including G_{q}, Gᵢ or many others. G_{q} subunits activate phospholipase Cγ, resulting in activation of protein kinase C (PKC) and an increase in the levels of cytoplasmic calcium (FIGURE 3). In turn, elevated intracellular calcium leads to the activation of cPLA2 and the production of arachidonic acid (AA). The AA serves as a substrate for COX in both synoviocytes and chondrocytes, leading to the production of PGE2. PKC activation also results in activation of MAP kinase leading to activation of NF-B and, in cells and tissues which have been primed by exposure to pro-inflammatory cytokines, modulates increased gene expression of proteins involved in cartilage catabolism.

Pro-inflammatory cytokine signaling, such as mediated by both IL-1 and TNF-α through their distinct cognate receptors, also converges on regulation of cell gene expression. The signal transduction pathways utilized by these distinct receptors, employ distinct kinases that are proximal to the receptors but the signaling pathways subsequently converge at the level of MAP kinases (FIGURE 3 and 4). Signal transduction depends upon phosphorylation of residues in a cascade of kinases, including "downstream" enzymes such as p38 MAP kinase. Activation of the IL-1-receptor and TNF-receptor also leads to stimulation of MAP kinase, common steps shared by the Gq coupled GPCRs (see FIGURE 3). It is now recognized that ligand-independent "crosstalk" can transactivate kinase pathways in response to costimulation of specific GPCRs and cytokines such as IL-1, leading to synergistic cellular responses (see FIGURE 3). Thus, a combination of selective inhibitors which blocks transactivation of a common signaling pathway (as shown in FIGURES 1 and 2) leading to increased gene expression of pro-inflammatory cytokines, iNOS, COX-2, and MMPs will act synergistically to prevent inflammation and cartilage degradation after arthroscopic surgical procedures.

### IV SUMMARY

From the molecular and cellular mechanisms of action defined for these chondroprotective agents, these compounds are expected to exhibit chondroprotective action when applied perioperatively in an irrigation solution (in combination with other chondroprotective agents or in combination with other anti-pain and anti-inflammation agents described herein) or otherwise administered directly to the joint via infusion or injection. In particular, these agents are expected to be effective drugs when delivered by an irrigation solution during an arthroscopic surgical procedure. Each metabolically active chondroprotective agent may be delivered in combination with one or more other chondroprotective agents, including small molecule drugs, peptides, proteins, recombinant chimeric proteins, antibodies, oligonucleotides or gene therapy vectors (viral and nonviral), to the spaces of the joint. For example, a drug such as a MAPK inhibitor can exert its actions on any cells associated with the fluid spaces of the joint and structures comprising the joint that are involved in the normal function of the joint or are present due to a pathological condition. These cells and structures include, but are not limited to: synovial cells, including both Type A fibroblast and type B macrophage cells; the cartilaginous components of the joint such as chondroblasts and chondrocytes; cells associated with bone, including periosteal cells, osteocytes, osteoblasts, osteoclasts; inflammatory cells including lymphocytes, macrophages, mast cells, monocytes, eosinophils; and other cells including endothelial cells, smooth muscle cells, fibroblasts and neural cells; and combinations of the above.

There is described formulations of the active therapeutic agent(s) which may be delivered in a formulation useful for introduction and administration of the drug into the joint that would enhance the delivery, uptake, stability or pharmacokinetics of the chondroprotective agent(s). Such a formulation may include, but is not limited to, microparticles, microspheres or nanoparticles composed of proteins, liposomes, carbohydrates, synthetic organic compounds, or inorganic compounds. There is described the delivery of a combination of chondroprotective agents, or one or more chondroprotective agents with one or more anti-pain and/or anti-inflammation agents present either as multiple pharmaceutically active substances within a homogeneous vehicle (e.g., a single encapsulated microsphere) or as a discrete mixture of individual delivery vehicles (e.g., a group of microspheres encapsulating one or more agents). Examples of formulation molecules include, but are not limited to, hydrophilic polymers, polycations (e.g. protamine, spermidine, polylysine), peptide or synthetic ligands and antibodies capable of targeting materials to specific cell types, gels, slow release matrices, soluble and insoluble particles, as well as formulation elements not listed.

There is described the local delivery of a combination of two or more chondroprotective agents, or one or more chondroprotective agents in combination with one or more anti-pain and/or anti-inflammation agents, alone or in combination with one or more anti-pain and/or anti-inflammatory agents, via an irrigation solution, an infusion containing the drugs which are present at therapeutically effective low concentrations and which enables the drugs to be delivered directly to the affected tissue or joint. The drug-containing infusion or irrigation solution may be employed pre-operatively and/or intra-operatively and/or post-operatively in connection with a surgical procedure or may be adminstered at other times not related to surgical procedures. Other conventional methods used for drug delivery have required systemic (e.g., intramuscular, intravenous, subcutaneous) administration which necessitate higher concentrations of drugs (and higher total dose) to be administered to the patient in order to achieve significant therapeutic concentrations in the targeted joint. Systemic administration also results in high concentrations in tissues other than the targeted joint which is undesirable and, depending on the dose, may result in adverse side effects. These systemic methods subject the drug to second-pass metabolism and rapid degradation, thereby limiting the duration of the effective therapeutic concentration. Since the combination of chondroprotective agents (with or without one or more anti-pain and/or anti-inflammatory agents) are administered directly to the joint by infusion or by irrigation, vascular perfusion is not required to carry the drug to the targeted tissue. This significant advantage allows for the local delivery of a lower therapeutically effective total dose for a variety of chondroprotective drugs.

### V. Method of Application

The solutions described herein have applications for a variety of operative/interventional procedures, including surgical, diagnostic and therapeutic techniques. The combination of chondroprotective agents may be administered by injection or by irrigation. For solutions for injection, the amount of active ingredient that may be combined with the carrier materials to produce a single dosage form will vary depending upon the patient to be treated, the nature of the active agents in the solution and the particular mode of administration. It will be understood, however, that the specific dose level for any particular patient will depend upon a variety of factors including the activity of the specific compound employed, the age, body weight, general health, sex and diet of the patient, time of administration, route of administration, rate of excretion of the drug combination, and the severity of the particular disease undergoing therapy.

Injectable preparations, for example, sterile injectable aqueous or oleagenous suspensions may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1/3-propanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or di-glycerides. In addition, fatty acids such as oleic acid find use in the preparation of injectables.

The solutions for injection may be administered in connection with an arthroscopic surgical procedure or at any time determined to be desireable by a physician directing patient care.

The irrigation solutions may be perioperatively applied during arthroscopic surgery of anatomic joints. As used herein, the term "perioperative" encompasses application intraprocedurally, pre- and intraprocedurally, intra- and postprocedurally, and pre-, intra- and postprocedurally. Preferably the solution is applied preprocedurally and/or postprocedurally as well as intraprocedurally. Such procedures conventionally utilize physiologic irrigation fluids, such as normal saline or lactated Ringer's, applied to the surgical site by techniques well known to those of ordinary skill in the art. The method herein involves substituting the anti-pain/anti-inflammatory/chondroprotective irrigation solutions for conventionally applied irrigation fluids. The irrigation solution is applied to the wound or surgical site prior to the initiation of the procedure, preferably before tissue trauma, and continuously throughout the duration of the procedure, to preemptively block pain and inflammation, and cartilage degradation. As used herein throughout, the term "irrigation" is intended to mean the flushing of a wound or anatomic structure with a stream of liquid. The term "application" is intended to encompass irrigation and other methods of locally introducing the solution, such as introducing a gellable version of the solution to the operative site, with the gelled solution then remaining at the site throughout the procedure. As used herein throughout, the term "continuously" is intended to also include situations in which there is repeated and frequent irrigation of wounds at a frequency sufficient to maintain a predetermined therapeutic local concentration of the applied agents, and applications in which there may be intermittent cessation of irrigation fluid flow necessitated by operating technique.

The concentrations listed for each of the agents within the solutions described herein are the concentrations of the agents delivered locally, in the absence of metabolic transformation, to the operative site in order to achieve a predetermined level of effect at the operative site. It is understood that the drug concentrations in a given solution may need to be adjusted to account for local dilution upon delivery. Solution concentrations are not adjusted to account for metabolic transformations or dilution by total body distribution because these circumstances are avoided by local delivery, as opposed to oral, intravenous, subcutaneous or intramuscular application.

Arthroscopic techniques for which the present solution may be employed include, by way of non-limiting example, partial meniscectomies and ligament reconstructions in the knee, shoulder acromioplasties, rotator cuff debridements, elbow synovectomies, and wrist and ankle arthroscopies. The irrigation solution is continuously supplied intraoperatively to the joint at a flow rate sufficient to distend the joint capsule, to remove operative debris, and to enable unobstructed intra-articular visualization.

Suitable arthroscopic irrigation solutions for inhibition of cartilage degradation and control of pain and inflammation during such arthroscopic techniques are provided in Examples 1-4 herein below.

In each of the solutions described herein, the agents are included in low concentrations and are delivered locally in low doses relative to concentrations and doses required with conventional methods of drug administration to achieve the desired therapeutic effect. It is impossible to obtain an equivalent therapeutic effect by delivering similarly dosed agents via other (i.e., intravenous, subcutaneous, intramuscular or oral) routes of drug administration since drugs given systemically are subject to first- and second-pass metabolism and are often rapidly cleared from the system circulation.

Practice of the present invention should be distinguished from conventional intra-articular injections of opiates and/or local anesthetics at the completion of arthroscopic or "open" joint (e.g., knee, shoulder, etc.) procedures. The solutions described herein are used for continuous infusion throughout the surgical procedure to provide preemptive inhibition of pain and inflammation. In contrast, the high concentrations necessary to achieve therapeutic efficacy with a constant infusion of currently used local anesthetics can result in profound systemic toxicity.

Upon completion of the procedure, it may be desirable to inject or otherwise apply a higher concentration of the same chondroprotective agent(s) and/or pain and/or inflammation inhibitors as used in the irrigation solution at the operative site, as an alternative or supplement to opiates. In addition, the direct injection of combinations of chondroprotective agents may be desirable, as described in detail herein. A suitable chondroprotective solution for injection is provided in Example 5 herein below.

### EXAMPLES

The following are several formulations suitable for certain operative procedures followed by a summary of three clinical studies.

### Example 1

### Irrigation Solution for Arthroscopy

The following composition is suitable for use in anatomic joint irrigation during arthroscopic procedures. Each drug is solubilized in a carrier fluid containing physiologic electrolytes, such as normal saline or lactated Ringer's solution, as are the remaining solutions described in subsequent examples.

| Class of Agent | Drug | Concentration (Nanomolar) |
|---|---|---|
| MAP Kinase Inhibitor | SB203580 | 200 |
| Matrix Metalloproteinase | U-24522 | 200 |
| Inhibitor | | |
| TGF-β Agonist | TGF-β2 | 200 |

### Example 2

### Alternative Irrigation Solution for Arthroscopy

The following composition is an alternate formulation suitable for use in anatomic joint irrigation during arthroscopic procedures.

| Class of Agent | Drug | Concentration (Nanomolar) |
|---|---|---|
| MAP Kinase Inhibitor | SB203580 | 200 |
| Nitric Oxide Synthase | | |
| Inhibitor | L-NIL | 1,000 |
| Interleukin Receptor | | |
| Agonist | IL-10 | 100 |

### Example 3

### Alternate Irrigation Solution

The following drugs and concentration ranges in solution in a physiologic carrier fluid are suitable.

| Class of Agent | Drug | Concentration (Nanomolar) |
|---|---|---|
| MAP Kinase Inhibitor | SB242235 | 200 |
| Nitric Oxide Synthase | L-NIL | 10,000 |
| Inhibitor | | |
| TGF-β Agonist | TGF-β2 | 100 |

### Example 4

### Alternate Irrigation Solution

The following composition is also useful in the present invention.

| Class of Agent | Drug | Concentration (Nanomolar) |
|---|---|---|
| MAP Kinase | SB242235 | 200 |
| Inhibitor | | |
| MMP Inhibitor | U-24522 | 200 |

### Example 5

### Chondroprotective Solution for Injection

The following composition is suitable for injection into an anatomic joint. Each drug is solubilized in a carrier fluid containing physiologic electrolytes, such as normal saline or lactated Ringer's solution. A doseage of 20 ml of the solution is suitable for administration to a patient.

| Class of Agent | Drug | Concentration |
|---|---|---|
| BMP Receptor Agonist | BMP-7 | 100 ng/ml |
| Nitric Oxide Synthase | 1,3 PBTT | 4.4 µg/ml |
| Inhibitor | | |
| TGF-β Agonist | pyrrolidine-dithiocarbamate | 16.4 µg/ml |

### Example 6

### Synergistic stimulation of a rapid PGE2 burst upon exposure to IL-1 and GPCR agonists.

Fibroblast-like synoviocytes exhibit characteristics of inflammatory cells and seem to be crucial regulators of joint inflammation and cartilage degradation. A synoviocyte cell culture model system was used to characterize the synergistic interactions between IL-1 and non-cytokine inflammatory mediators which are important in modulating the destruction of joint tissue, including damage that occurs as a consequence of tissue injury during arthroscopic surgery. Experiments were conducted to investigate G-protein coupled receptor (GPCR) agonists (histamine, bradykinin and isoproteronol) on the regulation of cytokine and prostanoid production in cultured human synovial fibroblasts and to characterize the activities of ketoprofen in this system. The kinetics of induction of prostaglandin E2 (PGE₂), interleukin-6 (IL-6) and interleukin-8 (IL-8) in response to stimulation with interleukin-1 (IL-1) are described. The ability of GPCR ligands to potentiate cytokine production following IL-1 priming was investigated.

In Examples 6-8, the following experimental methods and materials were employed unless otherwise indicated.

Cell Culture. Synovial tissue was obtained from osteoarthritis patients undergoing joint replacement surgery through the Clinical Research Center, MacNeal Hospital, and transported to the laboratory in Dulbecco's Modified Eagle's Medium (DMEM) containing penicillin (100 units/ml), streptomycin (100 µg/ml), and fungizone (0.25 µg/ml). The synovium was dissected and minced with scissors, and plated as explants in culture medium composed of DMEM containing L-glutamine (2 mM), heat inactivated fetal bovine serum (10% v/v), plus antibiotics. Cultures were housed at 37° C in a humidified atmosphere of 5% CO₂. Adherent synovial cells grew out of the explants within 2-3 weeks, and were passaged by trypsinization. Seed cultures were fed twice weekly and were passaged at confluency. Experiments were performed on cells from passages 3-8. Experimental cultures were plated into 35 mm dishes at a density of 7.5 X 10³ cells/cm² in 2 ml culture medium. Cultures were grown to near confluency for experiments, and contained 2.3 ± 0.3 X 10⁵ cells (mean ± S.E.M.,n=3), and 104 ± 13 µg protein (n=10). The growth medium was replaced twice weekly.

Experimental Treatments. One day prior to initiation of experimental treatments, medium was changed to experimental growth medium composed of DMEM containing 2% heat-inactivated fetal bovine serum, plus L-glutamine and antibiotics as above, to render the cells quiescent. The next day, cultures were primed by addition of specified concentrations of 1L-1 or additional ligands to the conditioned growth medium for 12-24 hr intervals, as indicated. In some experiments, conditioned growth medium was collected for analysis following priming with IL-1. Acute experimental treatments were performed after this priming interval, as follows. Cultures were removed from the incubator, washed three times with 2 ml aliquots of Locke's physiological buffer (LB composition in mM: NaCl, 154; KCl, 2.6; KH₂PO₄, 2.15; K₂HPO₄, 0.85; MgCl₂, 5; CaCl₂, 2; D-glucose, 10; HEPES, 10; pH 7.4, BSA, 0.1% w/v), and then equilibrated with an additional aliquot of LB containing specified ligands for 10 min on a 37° bath. This solution was removed by aspiration and replaced with a fresh buffer aliquot containing indicated ligands for specified time intervals at 37°. Pharmacological inhibitors typically were added during the 10 min preincubation interval, and agonists plus the specified inhibitors were present during the 3 min challenge interval.

Measurement of prostaglandin E2. Following indicated treatment protocols, aliquots of culture supernatant (1ml) were collected and rapidly frozen in liquid nitrogen. Samples were stored at -80° until processing. Aliquots of culture supernatant were analyzed by competitive binding radioimmunoassay as specified by the manufacturer (Sigma Chemical Co.), using an antibody with equivalent reactivity toward prostaglandins E2 and E1. For quantitation, a standard curve was prepared with each assay using fixed concentrations of [³H]prostaglandin E2, and increasing concentrations of authentic competing prostaglandin E2.

Measurement of IL-6. Production of the cytokine, IL-6, was also measured in aliquots of supernatant culture media which had been stored frozen at -80 ° C. IL-6 was measured by sandwich ELISA with alkaline phosphatase detection as described by the manufacturer (Pharmingen) and quantitated using standard curves prepared with the respective pure recombinant human cytokines. Experimental determinations were performed on duplicate cultures.

### Assays for [³H]thymidine Incorporation and MTT

Synoviocyte cell lines were routinely evaluated for competence to proliferate in response to IL-1, measured as incorporation of [³H]thymidine (Kimball & Fisher, 1988). In this preparation, maximally effective concentrations of IL-1 stimulate [³H]thymidine incorporation by 10-20 fold compared to quiescent cultures maintained in 2% serum (data not shown).

Data analysis. Immunoassays were routinely performed in duplicate aliquots from each culture. Experimental determinations were performed on duplicate or triplicate cultures. Each experiment was repeated in at least two cell lines. Nonlinear regression curve fitting and statistical analyses were performed using Graph-PAD Prism software (San Diego, CA).

Materials. Cell culture: Cell culture media were obtained from Sigma or Gibco/BRL. Fetal bovine serum was from Atlanta Biologicals Inc. (Norcross, GA). Drugs: Recombinant human interleukin-1 was obtained from Genzyme (Cambridge, MA). Ketoprofen was provided by Omeros Medical Systems, Inc. (Seattle, WA). Amitriptyline, forskolin, 5-hydroxytryptamine, isoproterenol, Bradykinin, histamine, and prostaglandin E2 were from Sigma. Radiochemicals: [³H]Prostaglandin E2, was obtained from American Radiolabeled Chemicals, Inc. (St. Louis, MO). All other reagents were obtained in the highest purity available from standard commercial suppliers.

The effect of GPCR agonists, histamine and bradykinin, on PGE2 production in human synovial cells was measured with and without prior stimulation to IL-1 to assess the functional interactions between agonists mediating a common pharmacological effect through these different classes of receptors. Overnight exposure of cultured human synovial fibroblasts to IL-1 (10 U/ml) results in a delayed (4 hrs) and sustained large enhancement of PGE2 production, which can be measured by radioimmunoassay as increased PGE2 in the culture supernatant. The progressive increase in PGE2 production during prolonged IL-1 treatment (16-24 hr) has been shown to arise from the coordinated upregulated expression of cPLA2 and the COX-2 (Crofford, 1984, Hulkower et al., 1984). Cultures which have been primed by overnight exposure to IL-1 respond to subsequent challenges with maximally effective concentrations of histamine (100 µM) or bradykinin (1 µM) with additional rapid (minutes) and robust production of PGE2. Representative data for the time course of PGE2 production in response to histamine or bradykinin stimulation are shown in Figure 7. Under these conditions, histamine elicits a 5-10 fold increase in PGE2 production compared to IL-1 primed cells receiving no GPCR agonist addition. Bradykinin elicits a 10-15 fold increase. The absolute quantity of PGE2 produced during the brief 2 min agonist challenge approaches or exceed quantities that are cumulatively produced during the entire 18 hr IL-1 priming interval. This is remarkable insofar as Fig. 7 shows that the vast majority of the histamine-elicited burst in PGE2 production occurs within the initial 2 min period since minimal additional accumulation is observed over the subsequent 60 min period. The bradykinin-stimulated PGE2 response continues to increase (2-fold) over the same time period. In the absence of IL,-1 priming, naive synoviocytes show no detectable PGE2 production in response to stimulation with either GPCR agonist alone. Under conditions of IL-1 priming, histamine and bradykinin both synergistically potentiated PGE2 release.

Using cultured synovial fibroblasts from osteoarthritis patients, we found time-dependent synergistic interactions between the pro-inflammatory cytokine, IL-1, and physiologically relevant G-protein coupled receptors on PGE2 production, and evaluated the actions of target therapeutic agents. GPCR agonists acting through endogenous synoviocyte receptors which are coupled to increases in intracellular calcium, inositol phosphates and PKC signaling pathways rapidly and dramatically amplify PGE2 production in cells previously primed by IL-1. COX inhibitors effectively attenuated both the agonist-elicited rapid burst and the long-term accumulation of PGE2. Thus, different GPCR and IL-1 pathways for intracellular signal transduction synergistically interact to bring about either rapid or slower, long-term regulation of PGE2 responses.

The synergism between IL-1 and calcium-regulatory GPCRs in synoviocytes that produce the rapid PGE2 burst may in part be explained by the rapid augmentation of arachidonic acid release, a measure of cPLA2 activation in many cell types. In addition to inducing COX-2 expression, IL-1 increases expression of cPLA2 (Hulkower et al., 1994). These two proteins act together to provide free arachidonic acid substrate for COX-2. The upregulation of the key eicosonoid metabolizing enzymes induced by IL-1, combined with the ability of the GPCR ligands to activate arachidonate release, would therefore be predicted to increase overall substrate flux through prostanoid synthesis. cPLA2 is the only known PLA2 that exhibits functional properties indicative of receptor regulation and is likely to be involved in eicosonoid production and intracellular signaling. Since cPLA2 is activated by increasing calcium concentrations for full activity and bradykinin B2 and histamine H1 receptor activation is coupled to mobilization of intracellular calcium, this is likely the predominant factor regulating the rapid agonist-stimulated burst in PGE2 production. Finally, the very rapid and transient increase in cytoplasmic calcium triggered by B2 or H1 receptor activation is similar to the kinetics known for cPLA2 activation, arachidonic acid release, and the observed PGE2 burst.

### Example 7

### Inhibition of PGE2 burst formation by cyclooxygenase inhibitors.

The actions of ketoprofen, a cyclooxygenase inhibitor, to attenuate PGE2 formation were determined by co-incubation with IL-1 during prolonged exposure (16 hr); and by brief pre-incubation prior to a subsequent GPCR agonist challenge interval, as shown in Figure 8. Addition of specified concentrations of ketoprofen during overnight priming with IL-1 abolishes PGE2 formation, with IC₅₀ = 4.5 ± 0.8 nM determined by nonlinear regression analysis (mean ± SEM, n=4 synoviocyte cell lines). Similar determinations (data not shown) were performed with the cyclooxygenase inhibitors etodolac (IC₅₀ = 15.2 ± 4.6 nM, n=4), ketorolac (2.2 ± 0.4 nM, n=4), and indomethacin (3.2 ± 1.5 nM, n=2).

Figure 8 also shows the ketoprofen concentration-dependent inhibition of the agonist-elicited PGE2 burst in response to a challenge by 100 µM histamine (IC₅₀ = 3.4 ± 0.2 nM, n=3) or 1 µM bradykinin (IC₅₀ = 9.5 ± 2.0 nM, n=3) in synoviocytes primed overnight with IL-1 (10 U/ml). These values are comparable to those observed for ketoprofen inhibition during overnight IL-1 induction of PGE2. This result demonstrates that the onset of inhibition by the COX inhibitor occurs within the 10 min pretreatment interval prior to GPCR agonist addition, consistent with a direct, reversible inhibition of the COX activity and not due a mechanism linked to changes in the expression levels of the prostanoid regulatory enzymes. This immediate inhibitory effect also provides a basis for the immediate effectiveness of this drug when delivered locally to the intra-articular in an irrigation solution during arthroscopic surgery.

### Example 8

### Induction of IL-6 production by IL-1 and GPCR agonists and inhibition by ketoprofen.

The kinetics of induction of interleukin-6 in response to stimulation with IL-1 are described. Synoviocyte cultures were exposed to the indicated treatments with IL-1 plus either histamine to activate signaling through inositol trisphosphate (InsP3)/protein kinase C pathway or isproterenol to activate increases in intracellular cAMP. Production of PGE₂, IL-6, and IL-8 were measured in the culture supernatants following 1, 2, 4, 6, and 24 hr treatments. In this experiment, each treatment interval was performed in a separate culture. In the above treatment regime, production of IL-6 was robustly increased by IL-1 following 24-hr exposure, but no IL-6 was detected within the initial 6 hr interval. IL-6 production in response to IL-1 was not augmented further by addition of histamine, and histamine alone failed to stimulate IL-6 production. IL-1 also produced a significant elevation of IL-8 (2000 pg/ml), which was first measurable at 6 hr of treatment. IL-8 production was sustained and greatly increased at 24-hr exposure to IL-1.

The effect of ketoprofen on the induction of cytokine production by IL- and GPCR agonists was examined. The protocol also tested the effects of IL-1 concentration dependence on the IL-6 steady state induction. Synoviocyte cultures were exposed to indicated concentrations of IL-1 and GPCR agonists. Culture supernatants were collected and replaced with fresh media aliquots containing the same agonist additions at 8-hr intervals. PGE₂, IL-6, and IL-8 in the supernatants were assayed as described.

Data for IL-6 production are shown in Fig. 9 which shows IL-6 production at 16 hr (corresponding to treatment interval from 8-16 hr) in the presence of indicated concentrations of IL-1 plus added ligand. Addition of histamine or isoproterenol does not enhance IL-6 production compared to IL-1 alone. At 1.0 pg/ml IL-1, ketoprofen causes a partial (≤50%) inhibition of IL-1-elicited IL-6 production. Furthermore, ketoprofen inhibited IL-6 production in the histamine or isoproterenol/IL-1 co-stimulated samples.

The synoviocyte cell culture model system was used to characterize the synergistic interactions between IL-1 and non-cytokine inflammatory mediators which are important in modulating the destruction of joint tissue, including damage that occurs as a consequence of tissue injury during arthroscopic surgery. The results can be summarized as follows: (1) IL-1 induces large increases in PGE₂, IL-6, and IL-8 in cultured synoviocytes, whereas quiescent cultures do not produce detectable quantities of these mediators, (2) the induction of PGE₂ occurs most rapidly and results in release of PGE₂ to the culture supernatant at 4 hr, followed by IL-8 at 6 hr, and IL-6 at longer intervals, and (3) all three mediators remain elevated in the culture supernatant following 24 hr IL-1 exposure.

In contrast to their actions on PGE₂ production, the GPCR agonists do not enhance IL-1 induction of IL-6 or IL-8 and also do not increase IL-6 and IL-8 release following priming with IL-1. IL-1 induction of IL-6 and IL-8 appears to be reinforced by the concomitant induction of PGE₂ since ketoprofen reduces the production of these cytokines in response to IL-1. This result indicates that ketoprofen could provide a therapeutic chondroprotective effect when delivered to the joint during surgical procedures.

Taken together, these results demonstrate interactions between specific G-coupled receptor signaling pathways and the activation of synoviocytes by pro-inflammatory stimulation with IL.-1. A similar mechanism is expected to be operative in chondrocytes. These interactions provide a means of integrating and modulating pro-inflammatory responses of synoviocytes and chondrocytes depending on inputs from other autocoid or neurotransmitter receptor systems within the joint. These findings underscore the rationale and potential clinical benefit of therapeutic interventions which target inhibition of G-protein coupled receptors that mediate signalling through calcium mobilization, phosphoinositide hydrolysis and PKC activation and are coupled to increases in production of PGE₂ in arthroscopic surgery. These receptors on synoviocytes and chondrocytes include histamine H₁, bradykinin, Substance P, 5HT2, and the purinergic P2Y receptors.

## Claims

1. The use, in the manufacture of a medicament in solution form for local delivery to a joint of a patient, for the protection of joint cartilage by both promotion of cartilage anabolic processes and inhibition of cartilage catabolic processes, of;
(a) a therapeutically effective amount of at least one anabolic chondroprotective agent selected from IL-4, IL-10, IL-13, TGFβ1, TGFβ2, BMP-4, BMP-7, IGF-1 and b FGF; and
(b) a therapeutically effective amount of at least one chondroprotective agent which is an inhibitor of cartilage catabolism selected from IL-1 receptor antagonists, TNF-α receptor antagonists, cyclooxygenase-2 inhibitors, MAP kinase inhibitors, nitric oxide synthase inhibitors and nuclear factor kβ inhibitors.

2. The use of claim 1 wherein the solution is to be delivered to the joint perioperatively during a surgical procedure.

3. The use of claim 2 wherein the solution is to be delivered to the joint during the surgical procedure.

4. The use of claim 2 wherein the procedure is an arthroscopic surgical procedure.

5. The use of claim 1 wherein the solution is to be delivered to a joint by injection.

6. The use of claim 1 wherein the solution is to be delivered prophylacticaly to the joint of a patient or to the joint of a patient suffering from articular cartilage degradation.

## Patentansprüche

1. Die Verwendung in der Herstellung eines Arzneistoffes in Lösungsform für die lokale Einbringung in das Gelenk eines Patienten, zum Schutz des Gelenkknorpels durch sowohl die Förderung anabolischer Knorpelprozesse als auch die Hemmung von katabolischen Knorpelprozessen von:
a. einer therapeutisch wirksamen Menge von mindestens einem anabolischen, chondroprotektiven Wirkstoff gewählt aus IL-4, IL-10, IL-13, TGFβ1, TGFβ2, BMP-4, BMP-7, IGF-1 und b FGF; und
b. einer therapeutisch wirksamen Menge von mindestens einem chondroprotektiven Wirkstoff, der ein Knorpel-Katabolismus-Inhibitor ist, gewählt aus IL-1-Rezeptor-Antagonisten; TNF-α-Rezeptor-Antagonisten; Cyclooxygenase-2-Inhibittiren; MAP-Kinase-Inhibitoren, NO-Synthase-Inhibitoren und NFκβ-Inhibitoren.

2. Die Verwendung von Anspruch 1, wobei die Lösung perioperativ während eines chirurgischen Verfahrens in das Gelenk einzubringen ist.

3. Die Verwendung von Anspruch 2, wobei die Lösung während des chirurgischen Verfahrens in das Gelenk einzubringen ist.

4. Die Verwendung von Anspruch 2, wobei das Verfahren ein arthroskopischer Eingriff ist.

5. Die Verwendung von Anspruch 1, wobei die Lösung durch Injektion in das Gelenk einzubringen ist.

6. Die Verwendung von Anspruch 1, wobei die Lösung prophylaktisch in das Gelenk eines Patienten oder in das Gelenk eines Patienten mit artiktilater Knorpelschädigung einzubringen ist.

## Revendications

1. Utilisation, dans la fabrication d'un médicament sous forme de solution destiné à être délivré localement à une articulation d'un patient, pour la protection du cartilage articulaire, par l'action aussi bien de promouvoir les processus anaboliques du cartilage que d'inhiber les processus cataboliques du cartilage :
(a) d'une quantité efficace sur le plan thérapeutique d'au moins un agent chondroprotecteur anabolique choisi parmi IL-4, IL-10, IL-13, TGFβ1, TGFβ2, BMP-4, BMP-7, IGF-1 et b-FGF ; et
(b) d'une quantité efficace sur le plan thérapeutique d'au moins un agent chondroprotecteur qui est un inhibiteur du catabolisme du cartilage choisi parmi les antagonistes du récepteur de l'IL-1, les antagonistes du récepteur du TNF-α, les inhibiteurs de la cyclooxygénase-2, les inhibiteurs de MAP kinase, les inhibiteurs de l'oxyde nitrique synthétase et les inhibiteurs du facteur nucléaire κβ.

2. Utilisation selon la revendication 1, dans laquelle la solution est à délivrer à l'articulation en périopératoire au cours d'une intervention chirurgicale.

3. Utilisation selon la revendication 2, dans laquelle la solution est à délivrer à l'articulation pendant l'intervention chirurgicale.

4. Utilisation selon la revendication 2, dans laquelle la procédure est une procédure chirurgicale arthroscopique.

5. Utilisation selon la revendication 1, dans laquelle la solution est à délivrer à une articulation par injection.

6. Utilisation selon la revendication 1, dans laquelle la solution est à délivrer en prophylaxie à l'articulation d'un patient ou à l'articulation d'un patient souffrant de dégradation du cartilage articulaire.
